(19) **Europäisches Patentamt**
**European Patent Office**
**Office européen des brevets**

(11) **EP 2 717 049 B1**

(12) **EUROPEAN PATENT SPECIFICATION**

(45) Date of publication and mention
of the grant of the patent:
**17.04.2024  Bulletin 2024/16**

(21) Application number: **12792564.2**

(22) Date of filing: **31.05.2012**

(51) International Patent Classification (IPC):
**G01N 30/86** *(2006.01)*    **G01N 30/88** *(2006.01)*
**G01N 30/74** *(2006.01)*    **G16C 20/20** *(2019.01)*

(52) Cooperative Patent Classification (CPC):
**G01N 30/8675; G16C 20/20; G01N 30/8686;**
G01N 30/74; G01N 30/8624; G01N 30/8634;
G01N 30/8665; G01N 30/8693; G01N 2030/862;
G06F 2218/10; G06F 2218/14

(86) International application number:
**PCT/JP2012/003602**

(87) International publication number:
**WO 2012/164946 (06.12.2012 Gazette 2012/49)**

(54) **METHOD FOR EVALUATING PATTERN, METHOD FOR EVALUATING MULTI-COMPONENT SUBSTANCE, EVALUATION PROGRAM, AND EVALUATION DEVICE**

VERFAHREN ZUR BEWERTUNG VON MUSTERN, VERFAHREN ZUR BEWERTUNG VON MEHRKOMPONENTENMASSEN, BEWERTUNGSPROGRAMM UND BEWERTUNGSVORRICHTUNG

PROCÉDÉ D'ÉVALUATION D'UN MOTIF, PROCÉDÉ D'ÉVALUATION D'UNE SUBSTANCE À PLUSIEURS COMPOSANTS, PROGRAMME ET DISPOSITIF D'ÉVALUATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.06.2011   JP 2011123844**

(43) Date of publication of application:
**09.04.2014   Bulletin 2014/15**

(73) Proprietor: **TSUMURA & CO.**
**Minato-ku**
**Tokyo 107-8521 (JP)**

(72) Inventors:
 • **MORI, Yoshikazu**
  **Inashiki-gun, Ibaraki 300-1192 (JP)**
 • **NODA, Keiichi**
  **Inashiki-gun, Ibaraki 300-1192 (JP)**

(74) Representative: **Oppermann, Frank**
**OANDO Oppermann & Oppermann**
**Wilhelminenstrasse 1a**
**65193 Wiesbaden (DE)**

(56) References cited:
**EP-A1- 1 760 464**      **WO-A1-97/39347**
**WO-A2-2005/079261**     **CN-A- 1 670 529**
**JP-A- 2 196 959**       **JP-A- 10 073 582**
**JP-A- 2007 315 941**    **US-A1- 2003 110 000**

 • **CARDEAL ET AL: "Comprehensive two-dimensional gas chromatography for fingerprint pattern recognition in cachaca production", TALANTA, ELSEVIER, AMSTERDAM, NL, vol. 74, no. 4, 2 January 2008 (2008-01-02), pages 793-799, XP022405965, ISSN: 0039-9140**

**Description**

FIELD OF THE INVENTION

The present invention relates to an evaluating method for a pattern, an evaluating program for performing an evaluation method for a pattern and an evaluating apparatus for a pattern.

DESCRIPTION OF THE PRIOR ART

As multicomponent materials, for example, there are natural-product-originated drugs such as kampo medicines that are drugs (hereinafter, referred to as multicomponent drugs) composed of multiple components. Quantitative and qualitative profiles in such drugs change due to a geological factor, an ecological factor, a collecting season, a collecting area, a collecting aetas, weather during the growing period and the like of raw material crude drugs.

Thus, for such multicomponent drugs and the like, predetermined criteria are regulated as qualities for securing the safety and the effectiveness thereof, and national supervising agencies, chemical organizations, manufacturers, and the like perform quality evaluations based on the criteria.

In general, however, the determination criteria on the quality and the like for a multicomponent drug are set based on the content and the like of one or several distinctive components selected from components in the multicomponent drug.

For example, in Non-Patent Literature 1, in a case that effective components of a multicomponent drug are not identified, it selects a plurality of components that have physical properties such as a quantitative analyzability, high water-solubility, undegradability in hot water, and non-chemical reactivity with other components and uses the contents of these components acquired through chemical analysis as evaluation criteria.

In addition, it is known to apply chromatography to a multicomponent drug, obtain an ultraviolet-visible absorption spectrum for each retention time, and set evaluation criteria based on some pieces of component information included therein.

For example, according to Patent Literature 1, some peaks included in HPLC chromatogram data (hereinafter, referred to as a chromatogram) are selected and encoded as barcodes, thereby evaluating a multicomponent drug.

In such methods, however, evaluation targets are limited to "contents of specific components" or "peaks of specific components in chromatogram," and thus only some components contained in a multicomponent drug are set as the evaluation targets. Accordingly, since a multicomponent drug includes many components other than the components that are the evaluation targets, such methods are insufficient as a method of evaluating the multicomponent drug in terms of accuracy.

In order to accurately evaluate the quality of a multicomponent drug, it is necessary to evaluate a pattern that covers information of all peaks or almost all peaks without small peaks corresponding to several %. Accordingly, it is necessary to associate all the peaks or almost all peaks with each other between multicomponent drugs.

However, it is difficult to efficiently associate a plurality of peaks with high accuracy. This interferes with an efficient evaluation of multicomponent drugs with high accuracy.

Described more, crude drugs are natural products, and therefore, multicomponent drugs even which have the same product name may have slightly different components. Hence, even if drugs have the same quality, content ratios of components thereof may be different from each other or a component present in one drug may not be present in the other drug (hereinafter, referred to as an inter-drug error). In addition, there is also a factor that peak intensity or peak elution time in a chromatogram has no precise repeatability (hereinafter, referred to as an analysis error). Accordingly, all peaks or almost all peaks may not be associated with peaks that are originated from the same components between the multicomponent drugs (hereinafter, referred to as peak assignment), thereby interfering with an efficient evaluation with high accuracy.

PRIOR ART LITERATURE

PATENT LITERATURE

PATENT LITERATURE1: JP 2002-214215 A

NON- PATENT LITERATURE

NON- PATENT LITERATURE1: Pharmaceuticals monthly vol. 28, No. 3, pp 67 to 71 (1986)
CARDEAL ET AL, "Comprehensive two-dimensional gas chromatography for fingerprint pattern recognition in cachaca production", TALANTA, ELSEVIER, AMSTERDAM, NL, (20080102), vol. 74, no. 4, ISSN 0039-9140, pages 793 - 799, XP022405965 describes a gas chromatography for fingerprint pattern recognition in cachaça production comprising an evaluating method for a pattern comprising a pattern acquiring step acquiring a target pattern of chromatograms measured for different samples. The problem is the identification of co-incident peaks and different peaks between samples in the absence of a suitable metric or criteria for expressing the overall similarity. Chromatograms of two samples are overlayed (evaluation target and evaluation criteria) to specify the co-incident peaks, and to simply compare the retention indices between the target fingerprint (calculated RI) and the reference fingerprint (literature RI) in which peaks of chromatograms are indicated by retention indices to specify components of the target fingerprint. Moreover, the comparison of the target fingerprint and the reference fingerprint is not intended for peak assignment with high accuracy, but for specifying the different peaks between two samples. Furthermore, each retention index of the target fingerprint and the reference fingerprint is the indexed peak using the retention time, and therefore the peaks are merely compared directly with each other.

WO 97/39347 A1 discloses to compare a standard chromatogram (reference pattern) with each one of sample chromatograms (target pattern), thereby allowing similarities between the standard chromatogram and the sample chromatograms to be determined. For example, WO 97/39347 A1 merely sets the comparison range common to a solid line of the standard chromatogram and a dotted line of the sample chromatogram, simply compares the corresponding segments of the solid line and dotted line defined by the comparison range by means of regressive approach, and then calculates a residual value for each sample chromatogram. Accordingly, the similarities between the standard chromatogram and the sample chromatograms according to the residual values can be determined without requiring identification of particular peaks of the chromatograms. WO 97/39347 A1 does not need to specify the corresponding peaks between the standard chromatogram and the sample chromatograms.

CN 1 670 529 A describes a method comprising the steps of preparation of a test solution and a reference sample and the determination of a HPLC fingerprint. CN 1 670 529 A uses a chromatogram at a measurement wavelength of 203nm and evaluates a pattern of a fingerprint using a "Similarity Evaluation System for Chromatographic Fingerprint of Traditional Chinese Medicine (Version 2004A) [HPLC]. CN 1 670 529 A merely extracts a plurality of peaks as the common peaks to evaluate a pattern of the fingerprint and evaluates the similarities among different batches of samples. CN 1 670 529 A does not prepare peak patterns for each peak using peripheral peaks to specify the common peaks between the reference and target fingerprints.

EP 1 760 464 A1 discloses a method for evaluation of a multicomponent-medicine wherein acquired evaluation (MD) values are determined using a quality engineering method (MT method).

SUMMARY OF THE INVENTION

PROBLEM TO BE SOLVED BY THE INVENTION

A problem to be solved is that there is a limit on an efficient evaluation of a pattern covering information of peaks with high accuracy with use of an existing evaluation technique.

MEANS FOR SOLVING THE PROBLEM

In order to improve the accuracy and the efficiency of an evaluation, the present invention provides an evaluating method for a pattern with the features of claim 1, an evaluation program with the features of claim 16, an evaluation apparatus with the features of claim 20.

EFFECT OF THE INVENTION

The evaluating method for a pattern according to the present invention has the above-described configuration, and therefore, peak assignment can be performed based on pattern comparison between the peak patterns. Accordingly, the peaks of the target pattern can be efficiently assigned to the respective peaks of the reference pattern with high accuracy, thereby further improving the accuracy and the efficiency of the evaluation.

In the same way, the evaluating method for a multicomponent material according to the present invention can further improve the accuracy and the efficiency of the evaluation of a multicomponent material.

The evaluation program for performing an evaluating method according to the present invention has the above-described configurations, and therefore, the accuracy and the efficiency of the evaluation can be further improved by realizing each function in a computer.

The evaluating apparatus according to the present invention has the above-described configurations, and therefore, the accuracy and the efficiency of the evaluation can be further improved by operating each part.

BRIEF DESCRIPTION OF THE DRAWINGS

[Fig. 1] It is a block diagram of an evaluating apparatus for a multicomponent drug (Embodiment 1).

[Fig. 2] It is a block diagram illustrating procedures of evaluating a multicomponent drug (Embodiment 1).

[Fig. 3] It is an explanatory diagram of a FP that is prepared from a three-dimensional chromatogram data (hereinafter, referred to as a 3D chromatogram) (Embodiment 1).

[Fig. 4] It is a graph illustrating FPs of respective drugs in which (A) is a drug A, (B) is a drug B, and (C) is a drug C (Embodiment 1).

[Fig. 5] It is a diagram illustrating retention time points of a target FP and a reference FP (Embodiment 1).

[Fig. 6] It is a diagram illustrating a retention time appearance pattern of the target FP (Embodiment 1).

[Fig. 7] It is a diagram illustrating a retention time appearance pattern of the reference FP (Embodiment 1).

[Fig. 8] It is a table illustrating the numbers of matches in a retention time appearance distance between the target FP and the reference FP (Embodiment 1).

[Fig. 9] It is a table illustrating the degrees of matching between the retention time appearance patterns of the target FP and the reference FP (Embodiment 1).

[Fig. 10] It is diagram illustrating an assignment target peak of the target FP (Embodiment 1).

[Fig. 11] It is a peak pattern diagram according to three peaks including the assignment target peak (Embodiment 1).

[Fig. 12] It is a peak pattern diagram according to five peaks including the assignment target peak (Embodiment 1).

[Fig. 13] It is a diagram illustrating an allowable range for the assignment target peak (Embodiment 1).

[Fig. 14] It is a diagram illustrating assignment candidate peaks of the reference FP for the assignment target peak (Embodiment 1).

[Fig. 15] It is a peak pattern diagram according to three peaks of assignment candidate peaks for the assignment target peak (Embodiment 1).

[Fig. 16] It is a peak pattern diagram according to three peaks of another assignment candidate peaks for the assignment target peak (Embodiment 1).

[Fig. 17] It is a peak pattern diagram according to three peaks of another assignment candidate peaks for the assignment target peak (Embodiment 1).

[Fig. 18] It is a peak pattern diagram according to three peaks of another assignment candidate peaks for the assignment target peak (Embodiment 1).

[Fig. 19] It is a peak pattern diagram according to five peaks of assignment candidate peaks for the assignment target peak (Embodiment 1).

[Fig. 20] It is a peak pattern diagram according to five peaks of another assignment candidate peaks for the assignment target peak (Embodiment 1).

[Fig. 21] It is a peak pattern diagram according to five peaks of another assignment candidate peaks for the assignment target peak (Embodiment 1).

[Fig. 22] It is a peak pattern diagram according to five peaks of another assignment candidate peaks for the assignment target peak (Embodiment 1).

[Fig. 23] It is a diagram illustrating peak pattern configuring candidate peaks for the assignment target peak and an assignment candidate peak (Embodiment 1).

[Fig. 24] It is a diagram illustrating the number of all the peak patterns for the assignment target peak in a case that four peak pattern configuring candidate peaks are set (Embodiment 1).

[Fig. 25] It is a diagram illustrating the number of all the peak patterns for an assignment candidate peak in a case that four peak pattern configuring candidate peaks are set (Embodiment 1).

[Fig. 26] It is an explanatory diagram illustrating comprehensive comparison of peak patterns for the assignment target peak with respect to peak patterns for an assignment candidate peak (Embodiment 1).

[Fig. 27] It is an explanatory diagram illustrating comprehensive comparison of peak patterns for the assignment target peak with respect to peak patterns for the assignment candidate peak (Embodiment 1).

[Fig. 28] It is an explanatory diagram illustrating comprehensive comparison of peak patterns for the assignment target peak with respect to peak patterns for the assignment candidate peak (Embodiment 1).

[Fig. 29] It is an explanatory diagram illustrating comprehensive comparison of peak patterns for the assignment target peak with respect to peak patterns for the assignment candidate peak (Embodiment 1).

[Fig. 30] It is an explanatory diagram illustrating comprehensive comparison of peak patterns for the assignment target peak with respect to peak patterns for the assignment candidate peak (Embodiment 1).

[Fig. 31] It is an explanatory diagram illustrating comprehensive comparison of peak patterns for the assignment target peak with respect to peak patterns for the assignment candidate peak (Embodiment 1).

[Fig. 32] It is an explanatory diagram illustrating comprehensive comparison of peak patterns for the assignment target peak with respect to peak patterns for the assignment candidate peak (Embodiment 1).

[Fig. 33] It is an explanatory diagram illustrating comprehensive comparison of peak patterns for the assignment target peak with respect to peak patterns for the assignment candidate peak (Embodiment 1).

[Fig. 34] It is an explanatory diagram illustrating comprehensive comparison of peak patterns for the assignment target peak with respect to peak patterns for the assignment candidate peak (Embodiment 1).

[Fig. 35] It is an explanatory diagram illustrating comprehensive comparison of peak patterns for the assignment target peak with respect to peak patterns for the assignment candidate peak (Embodiment 1).

[Fig. 36] It is an explanatory diagram illustrating comprehensive comparison of peak patterns for the assignment target peak with respect to peak patterns for the assignment candidate peak (Embodiment 1).

[Fig. 37] It is an explanatory diagram illustrating comprehensive comparison of peak patterns for the assignment target peak with respect to peak patterns for the assignment candidate peak (Embodiment 1).

[Fig. 38] It is an explanatory diagram illustrating comprehensive comparison of peak patterns for the assignment target peak with respect to peak patterns for the assignment candidate peak (Embodiment 1).

[Fig. 39] It is an explanatory diagram illustrating comprehensive comparison of peak patterns for the assignment target peak with respect to peak patterns for the assignment candidate peak (Embodiment 1).

[Fig. 40] It is an explanatory diagram illustrating comprehensive comparison of peak patterns for the assignment target peak with respect to peak patterns for the assignment candidate peak (Embodiment 1).

[Fig. 41] It is an explanatory diagram illustrating comprehensive comparison of peak patterns for the assignment target peak with respect to peak patterns for the assignment candidate peak (Embodiment 1).

[Fig. 42] It is an explanatory diagram illustrating comprehensive comparison of peak patterns for the assignment target peak with respect to peak patterns for the assignment candidate peak (Embodiment 1).

[Fig. 43] It is an explanatory diagram illustrating comprehensive comparison of peak patterns for the assignment target peak with respect to peak patterns for the assignment candidate peak (Embodiment 1).

[Fig. 44] It is an explanatory diagram illustrating comprehensive comparison of peak patterns for the assignment target peak with respect to peak patterns for the assignment candidate peak (Embodiment 1).

[Fig. 45] It is an explanatory diagram illustrating comprehensive comparison of peak patterns for the assignment target peak with respect to peak patterns for the assignment candidate peak (Embodiment 1).

[Fig. 46] It is an explanatory diagram illustrating comprehensive comparison of peak patterns for the assignment target peak with respect to peak patterns for the assignment candidate peak (Embodiment 1).

[Fig. 47] It is an explanatory diagram illustrating comprehensive comparison of peak patterns for the assignment target peak with respect to peak patterns for the assignment candidate peak (Embodiment 1).

[Fig. 48] It is an explanatory diagram illustrating comprehensive comparison of peak patterns for the assignment target peak with respect to peak patterns for the assignment candidate peak (Embodiment 1).

[Fig. 49] It is an explanatory diagram illustrating comprehensive comparison of peak patterns for the assignment target peak with respect to peak patterns for the assignment candidate peak (Embodiment 1).

[Fig. 50] It is an explanatory diagram illustrating comprehensive comparison of peak patterns for the assignment target peak with respect to peak patterns for the assignment candidate peak (Embodiment 1).

[Fig. 51] It is an explanatory diagram illustrating comprehensive comparison of peak patterns for the assignment target peak with respect to peak patterns for the assignment candidate peak (Embodiment 1).

[Fig. 52] It is an explanatory diagram illustrating comprehensive comparison of peak patterns for the assignment target peak with respect to peak patterns for the assignment candidate peak (Embodiment 1).

[Fig. 53] It is an explanatory diagram illustrating comprehensive comparison of peak patterns for the assignment target peak with respect to peak patterns for the assignment candidate peak (Embodiment 1).

[Fig. 54] It is an explanatory diagram illustrating comprehensive comparison of peak patterns for the assignment target peak with respect to peak patterns for the assignment candidate peak (Embodiment 1).

[Fig. 55] It is an explanatory diagram illustrating comprehensive comparison of peak patterns for the assignment target peak with respect to peak patterns for the assignment candidate peak (Embodiment 1).

[Fig. 56] It is an explanatory diagram illustrating comprehensive comparison of peak patterns for the assignment target peak with respect to peak patterns for the assignment candidate peak (Embodiment 1).

[Fig. 57] It is an explanatory diagram illustrating comprehensive comparison of peak patterns for the assignment target peak with respect to peak patterns for the assignment candidate peak (Embodiment 1).

[Fig. 58] It is an explanatory diagram illustrating comprehensive comparison of peak patterns for the assignment target peak with respect to peak patterns for the assignment candidate peak (Embodiment 1).

[Fig. 59] It is an explanatory diagram illustrating comprehensive comparison of peak patterns for the assignment target peak with respect to peak patterns for the assignment candidate peak (Embodiment 1).

[Fig. 60] It is an explanatory diagram illustrating comprehensive comparison of peak patterns for the assignment target peak with respect to peak patterns for the assignment candidate peak (Embodiment 1).

[Fig. 61] It is an explanatory diagram illustrating comprehensive comparison of peak patterns for the assignment target peak with respect to peak patterns for the assignment candidate peak (Embodiment 1).

[Fig. 62] It is a diagram illustrating a calculating method of the degree of matching between peak patterns of the assignment target peak and an assignment candidate peak according to three peaks (Embodiment 1).

[Fig. 63] It is a diagram illustrating a calculating method of the degree of matching between peak patterns of the assignment target peak and the assignment candidate peak according to three peaks (Embodiment 1).

[Fig. 64] It is a diagram illustrating a calculating method of the degree of matching between peak patterns of the assignment target peak and the assignment candidate peak according to five peaks (Embodiment 1).

[Fig. 65] It is a diagram illustrating UV spectra of an assignment target peak and an assignment candidate peak (Embodiment 1).

[Fig. 66] It is an explanatory diagram illustrating the degree of matching between the UV spectra of the assignment target peak and the assignment candidate peak (Embodiment 1).

[Fig. 67] It is an explanatory diagram illustrating the degree of matching of the assignment candidate peak by comparison of both the peak patterns and the UV spectra together (Embodiment 1).

[Fig. 68] It is an explanatory diagram illustrating assignment of the target FP to a reference group FP (Embodiment 1).

[Fig. 69] It is a diagram illustrating a state in which the target FP is assigned to the reference group FP (Embodiment 1).

[Fig. 70] It is a diagram illustrating various target FPs and evaluation values (MD values) thereof (Embodiment 1).

[Fig. 71] It is a diagram illustrating various target FPs and evaluation values (MD values) thereof (Embodiment 1).

[Fig. 72] It is a diagram illustrating various target FPs and evaluation values (MD values) thereof (Embodiment 1).

[Fig. 73] It is a diagram illustrating various target FPs and evaluation values (MD values) thereof (Embodiment 1).

[Fig. 74] It is a diagram illustrating various target FPs and evaluation values (MD values) thereof (Embodiment 1).

[Fig. 75] It is a process chart illustrating an evaluating method of a multicomponent drug (Embodiment 1).

[Fig. 76] It is an evaluating flowchart for a multicomponent drug (Embodiment 1).

[Fig. 77] It is a data processing flowchart of a FP preparing function according to a single wavelength (Embodiment 1).

[Fig. 78] It is a data processing flowchart of a FP preparing function according to a plurality of wavelengths (Embodiment 1).

[Fig. 79] It is a data processing flowchart of the FP preparing function according to the plurality of wavelengths (Embodiment 1).

[Fig. 80] It is a data processing flowchart of a peak assigning process 1 (selection of a reference FP) (Embodiment 1).

[Fig. 81] It is a data processing flowchart of a peak assigning process 2 (calculation of an assignment score) (Embodiment 1).

[Fig. 82] It is a data processing flowchart of a peak assigning process 3 (specifying a corresponding peak) (Embodiment 1).

[Fig. 83] It is a data processing flowchart of a peak assigning process 4 (assignment to a reference group FP) (Embodiment 1).

[Fig. 84] It is a data processing flowchart of the peak assigning process 4 (assignment to the reference group FP) (Embodiment 1).

[Fig. 85] It is a flowchart of a process of calculating the degree of matching between retention time appearance patterns in the peak assigning process 1 (selection of the reference FP) (Embodiment 1).

[Fig. 86] It is a flowchart of a process of calculating the degree of matching between UV spectra in the peak assigning process 2 (calculation of an assignment score) (Embodiment 1).

[Fig. 87] It is a flowchart of a process of calculating the degree of matching between peak patterns in the peak assigning process 2 (calculation of an assignment score) (Embodiment 1).

[Fig. 88] It is a flowchart for preparing a reference FP feature value file (Embodiment 1).

[Fig. 89] It is a flowchart illustrating details of a "process of integrating reference FP assigning results (preparation of a FP correspondence table)" (Embodiment 1).

[Fig. 90] It is a flowchart illustrating details of the "process of integrating reference FP assigning results (preparation of a FP correspondence table)" (Embodiment 1).

[Fig. 91] It is a flowchart illustrating details of a "peak-feature value converting process (preparation of a reference group FP)" in detail (Embodiment 1).

[Fig. 92] It is a table illustrating a data example of a 3D chromatogram (Embodiment 1).

[Fig. 93] It is a table illustrating a data example of peak information (Embodiment 1).

[Fig. 94] It is a table illustrating a FP data example (Embodiment 1).

[Fig. 95] It is a table illustrating an assignment score calculation result (determination result) file example of a target FP with respect to a reference FP (Embodiment 1).

[Fig. 96] It is a table illustrating a process of collating corresponding peaks between a target FP and a reference FP (Embodiment 1).

[Fig. 97] It is a table illustrating a collation result file example (Embodiment 1).

[Fig. 98] It is a table illustrating a data example of a reference group FP (Embodiment 1).

[Fig. 99] It is a table illustrating a target FP peak feature value file example (Embodiment 1).

[Fig. 100] It is a flowchart illustrating details of a modified example of Subroutine 2 that is applied instead of the process illustrated in Fig. 86 (Embodiment 1).

[Fig. 101] It is a table illustrating a calculating example of moving averages and moving inclinations (Embodiment 1).

EMBODIMENT FOR CARRYING OUT THE INVENTION

The object capable of improving the accuracy and the efficiency of an evaluation is realized by comparing and evaluating results as a feature value that is acquired by assigning peaks of a target pattern to respective peaks of a reference group pattern all together with high accuracy and high efficiency.

The assigning of peaks all together with high accuracy and high efficiency is realized by comparing peak patterns that are acquired by patterning peaks of the target pattern and a reference pattern with use of peaks that are present at least on one of sides located in front and in the rear thereof in the direction of the time axis.

EMBODIMENT 1

Embodiment 1 of the present invention applies an evaluating method, an evaluating program and an evaluating apparatus for a pattern as an evaluating method, an evaluating program and an evaluating apparatus for a multicomponent material, e.g., a multicomponent drug.

A multicomponent drug is defined as a drug that contains a plurality of effective chemical components. Examples of the multicomponent drug include a crude drug, a combination of crude drugs, an extract thereof, and a kampo medicine, but are not limited thereto. In addition, the dosage form is not particularly limited, and, examples include a liquid medicine, an extract, a capsule, a granule, a pill, suspension-emulsion, a powder, a spiritus, a tablet, an infusion-decoction, a tincture, a troche, aromatic water, a fluid extract, which are specified in "general rule for preparations" of "The Japanese Pharmacopoeia", Fifteenth Edition. As the multicomponent material, materials other than a drug are also included.

Specific examples of the kampo medicine are written in Industry Standard and Voluntarily Revision of "Precautions" in 148 Prescriptions for Medical Kampo Drug Formulation and in Guide to General Kampo Prescription (1978).

In an evaluation of a multicomponent drug, it evaluates whether or not an evaluation target drug is equivalent to a plurality of drugs that are defined as normal products. For this, first, a target FP is prepared by extracting information unique to the drug from a three-dimensional chromatogram data (hereinafter, referred to as a 3D chromatogram) of the evaluation target drug.

Next, each peak of the target FP is assigned to peak correspondence data (hereinafter, referred to as a reference group FP) of all reference FPs, which is prepared by performing a peak assigning process to all the reference FPs, whereby a peak feature value is acquired.

Next, equivalency between peaks of the reference group FP and the assigned peaks of the target FP (hereinafter, referred to as target FP assignment peaks) is evaluated by MT method. Finally, it is determined whether or not the evaluation target drug is equivalent to a normal product by comparing an acquired evaluation value (hereinafter, referred to as a MD value) with a preset determination value (an upper limit value of the MD value).

The 3D chromatogram is a HPLC chromatogram data (hereinafter, referred to as chromatogram) of a multicomponent drug that is a multicomponent material as an evaluation target and includes UV spectra.

The FP is fingerprint data that is configured by maximum values or area values (hereinafter, referred to as peaks) in signal strength (height) of peaks detected at a specific wavelength and by appearance time points (hereinafter, referred to as retention time points) of the peaks.

The target FP is acquired by extracting a plurality of peaks, retention time points and UV spectra thereof at a specific detection wavelength from a 3D chromatogram that is three-dimensional chromatogram data of a kampo medicine being an evaluation target.

The reference FP corresponds to the target FP and is a FP of a kampo medicine as a multicomponent drug that is a multicomponent material determined as a normal product.

[Evaluating Device for Multicomponent Drug]

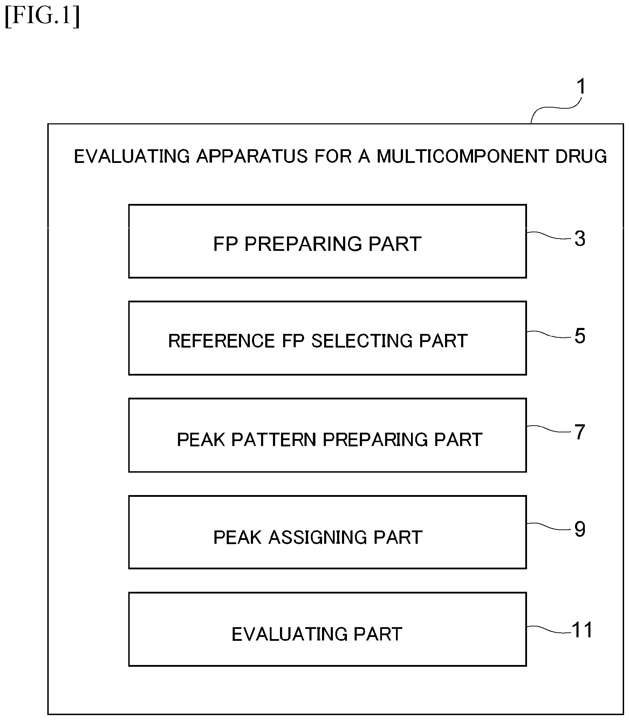

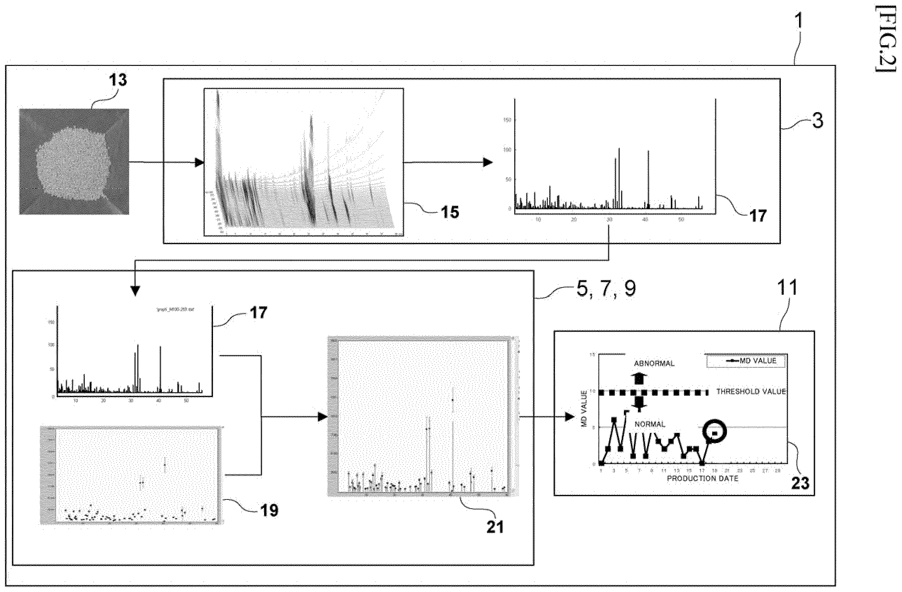

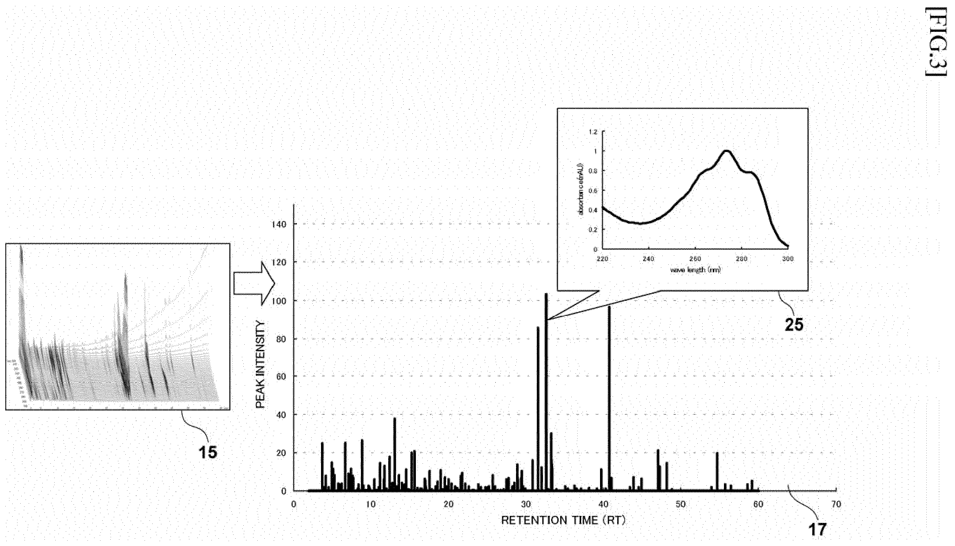

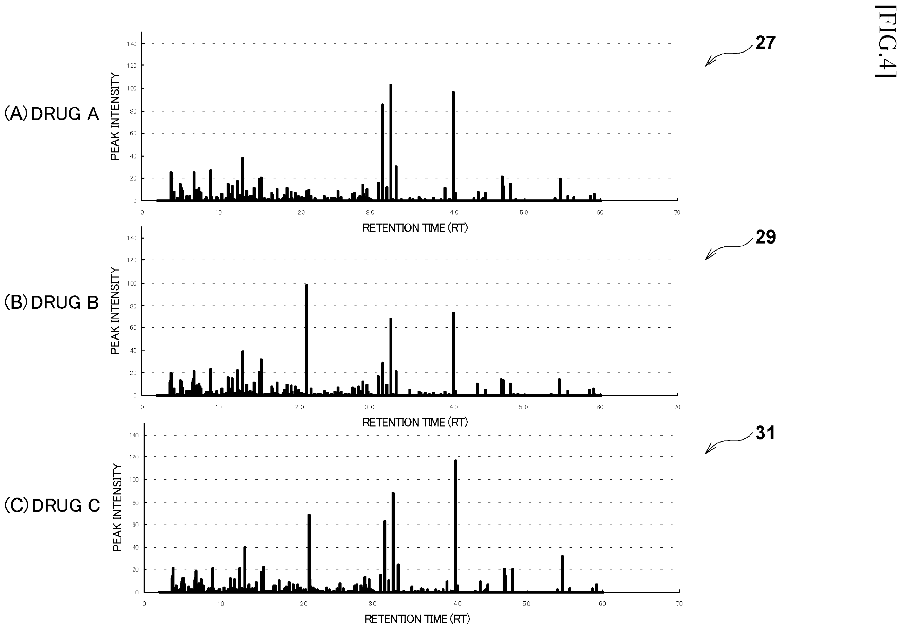

Fig. 1 is a block diagram of an evaluating apparatus for a multicomponent drug, Fig. 2 is a block diagram illustrating procedures of evaluating a multicomponent drug, Fig. 3 is an explanatory diagram of a FP that is prepared from a 3D chromatogram, Fig. 4(A) is a FP of a drug A, (B) is a FP of a drug B, and (C) is a FP of a drug C.

As illustrated in Figs. 1 and 2, the evaluating apparatus 1 for a multicomponent drug as an evaluating apparatus for a pattern includes a FP preparing part 3 as a pattern acquiring part, a reference FP selecting part 5, a peak pattern preparing part 7, a peak assigning part 9, and an evaluating part 11. The evaluating apparatus 1 for a multicomponent drug is configured by a computer and, although not illustrated in the drawings, includes a CPU, a ROM, a RAM, and the like. The evaluating apparatus 1 for a multicomponent drug can evaluate a multicomponent drug by implementing an evaluating program for a multicomponent drug as an evaluating program for a multicomponent material that is installed in the computer. However, the evaluation of the multicomponent drug may be realized by using an evaluating program recording medium for a multicomponent drug that stores the evaluating program and by reading out it with the evaluating apparatus 1 configured by the computer for a multicomponent drug.

In this embodiment, the FP preparing part 3, the reference FP selecting part 5, the peak pattern preparing part 7, the peak assigning part 9, and the evaluating part 11 are configured by a single computer. Alternatively, the FP preparing part 3, the reference FP selecting part 5, the peak pattern preparing part 7, the peak assigning part 9, and the evaluating part 11 may be configured by respective discrete computers, or the FP preparing part 3 and the reference FP selecting part 5, the peak pattern preparing part 7and the peak assigning part 9, and the evaluating part 11 may be configured by discrete computers.

The FP preparing part 3 acquires a target pattern of an evaluation target whose peaks change in a time series. More precisely, the FP preparing part 3, for example, is a functional part that prepares and acquires a target FP 17 (hereinafter, it may be simply referred to as an "FP 17") as a target pattern. The FP 17, similarly to the 3D chromatogram 15, is configured by three-dimensional information (peaks, retention time points, and UV spectra).

The FP 17, therefore, is data that directly succeed to the information unique to the drug. In spite of that, the data volume of the FP 17 is compressed at the ratio of about 1/70, and therefore, information amount to be processed is much smaller than that of the 3D chromatogram 15, thereby increasing processing speed.

The 3D chromatogram 15 is a result of applying high performance liquid chromatography (HPLC) to a kampo medicine 13 (Fig. 2). In the 3D chromatogram 15, a movement speed of each component appears to represent as a movement distance during specific time, or an appearance in a time series from a column end is represented in a chart. In the HPLC, detector responses are plotted with respect to the time axis, and appearance time points of peaks are called retention time points.

Although the detector is not particularly limited, an absorbance detector employing an optical characteristic is used as the detector. A peak is three-dimensionally acquired as a signal strength according to a detection wavelength of ultraviolet (UV). As a detector employing an optical characteristic, a transmittance detector may be used.

The detection wavelengths are not particularly limited, and are a plurality of wavelengths selected preferably from a range of 150 nm to 900 nm, selected more preferably from a range of 200 nm to 400 nm corresponding to a UV-visible absorption range, and selected further more preferably from a range of 200 nm to 300 nm.

The 3D chromatogram 15 at least includes a number (lot number), retention time points, detection wavelengths, and peaks of a kampo medicine as data.

In addition, the 3D chromatogram 15 can be also acquired by using commercially-available devices. As such a commercially-available device, there is "Agilent 1100 system" or the like. Furthermore, the chromatograph is not limited to the HPLC, and any other type of chromatography may be employed.

In the 3D chromatogram 15, as illustrated in Figs. 2 and 3, the x-axis represents the retention time point, the y-axis represents the detection wavelength, and the z-axis represents signal strength.

The FP 17 at least includes a number (lot number), retention time points, peaks at a specific wavelength, and UV spectra of a kampo medicine as data.

The FP 17 is two-dimensionally represented with the x-axis representing the retention time points and the y-axis representing the peaks for the specific detection wavelength as illustrated in Figs. 2 and 3. However, the FP 17 is data that includes UV spectrum information for each peak that is similar to the UV spectrum 25 represented with respect to one peak as illustrated in Fig. 3. The specific detection wavelength for which the FP 17 is prepared is not particularly limited and may be selected in various manners. However, it is important for the FP 17 to include all the peaks of the 3D chromatogram in order to succeed to the information. Accordingly, in Embodiment 1, the detection wavelength is set to 203 nm that includes all the peaks of the 3D chromatogram.

Meanwhile, there are cases where all the peaks are not included at a single wavelength. In such a case, a plurality of detection wavelengths are set to prepare a FP that includes all the peaks by combining the plurality of wavelengths as described later.

In Embodiment 1, although the peak is set as the maximum value of the signal strength (peak height), the area value may be used as the peak. In addition, a FP may not include UV spectra, so that the FP is set as two-dimensional display information in which the x-axis represents the retention time points, and the y-axis represents the peaks for a specific wavelength. In such a case, the FP can be prepared from a 2D chromatogram as a chromatogram that includes a number (lot number) and retention time points of a kampo medicine as data.

Fig. 4(A) is a FP of a drug A, Fig. 4(B) is a FP of a drug B, and Fig. 4(C) is a FP of a drug C.

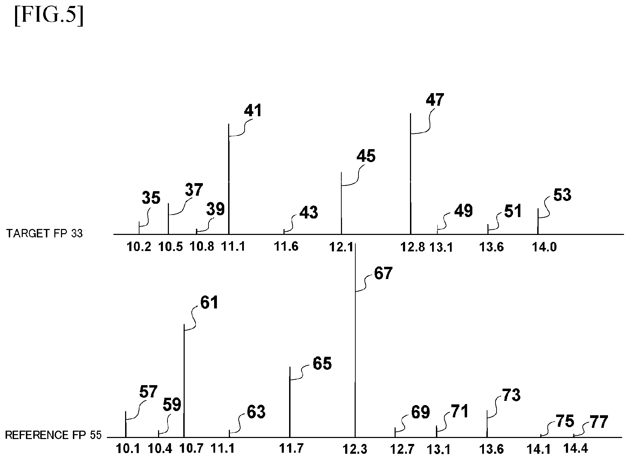

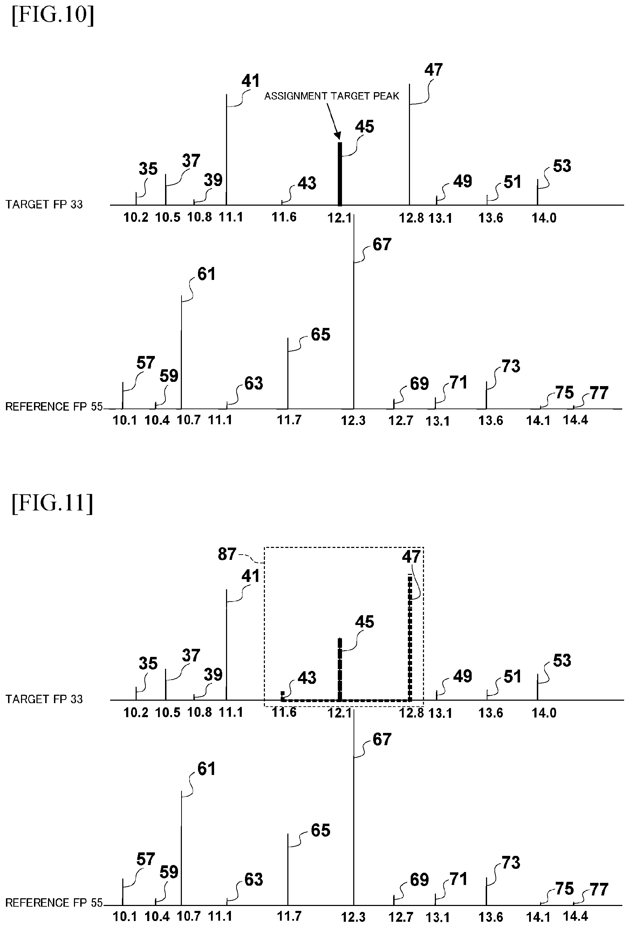

The reference FP selecting part 5 is a functional part that selects a reference FP that is used by the peak pattern preparing part 7 from among a plurality of reference FPs. The reference FP selecting part 5 selects a FP of a multicomponent material that is appropriate to the assignment of the peaks to the target FP from among the plurality of reference FPs. In other words, in order to perform peak assignment of each peak of the target FP with high accuracy, as illustrated in Figs. 5 to 9, the degree of matching between retention time point appearance patterns of the peaks of the target FP and each reference FP are calculated to select a reference FP with the minimum degree of matching from among all the reference FPs. This will be described in detail later. The peak pattern preparing part 7 is a functional part that, as illustrated in Figs. 10 to 12, prepares a peak pattern configured by a total of n+1 peaks including n peaks that are present at least on one of sides located in front and in the rear of a peak (hereinafter, referred to as an assignment target peak) of the target FP 33 that is a target to be assigned in the direction of the time axis, as a peak pattern of an assignment target peak. Here, "n" is a natural number. This will be described in detail later.

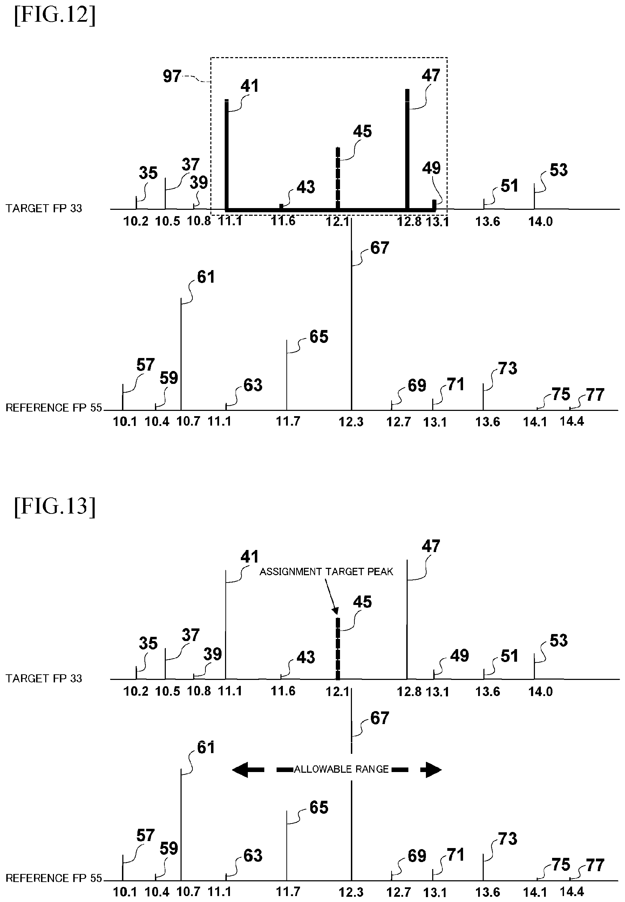

Fig. 11 illustrates a peak pattern configured by a total of three peaks that include two peaks being present at least on one of sides located in front and in the rear in the time axis direction, and Fig. 12 illustrates a peak pattern configured by a total of five peaks that include four peaks being present at least on one of sides located in front and in the rear in the time axis direction.

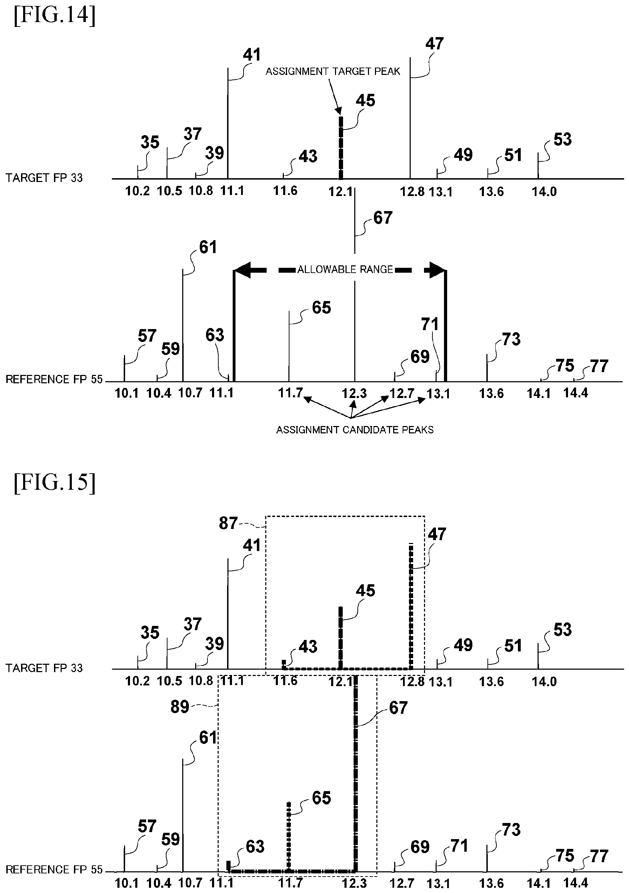

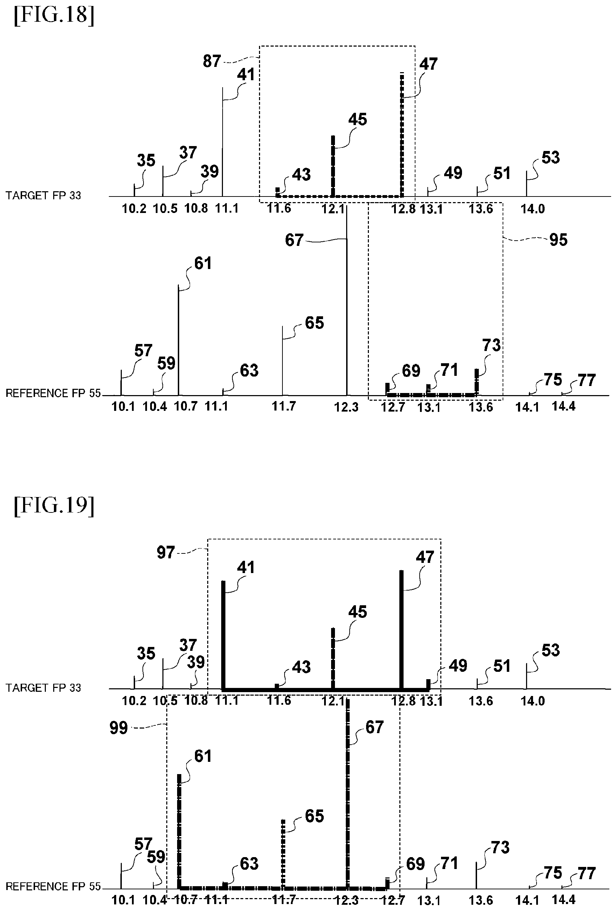

In addition, the peak pattern preparing part 7 is a functional part that, as illustrated in Figs. 13 to 22 (to be described later), prepares peak patterns each configured by a total of n+1 peaks including n peaks that are present at least on one of sides located in front and in the rear in the time axis direction for all the peaks (hereinafter, referred to as assignment candidate peaks) each having a difference from the retention time point of the assignment target peak within a set range (allowable range) in the reference FP 55, as the peak patterns of the assignment candidate peaks. Figs. 15 to 18 (to be described later) show peak patterns each configured by a total of three peaks including two peaks that are located at least on one of sides located in front and in the rear in the time axis direction. Figs. 19 to 22 (to be described later) show peak patterns each configured by a total of five peaks including four peaks that are located at least on one of sides located in front and in the rear in the time axis direction.

The allowable range is not particularly limited, but is preferably in the range of 0.5 to 2 minutes with the object of the accuracy and efficiency. In Embodiment 1, the allowable range is set to one minute.

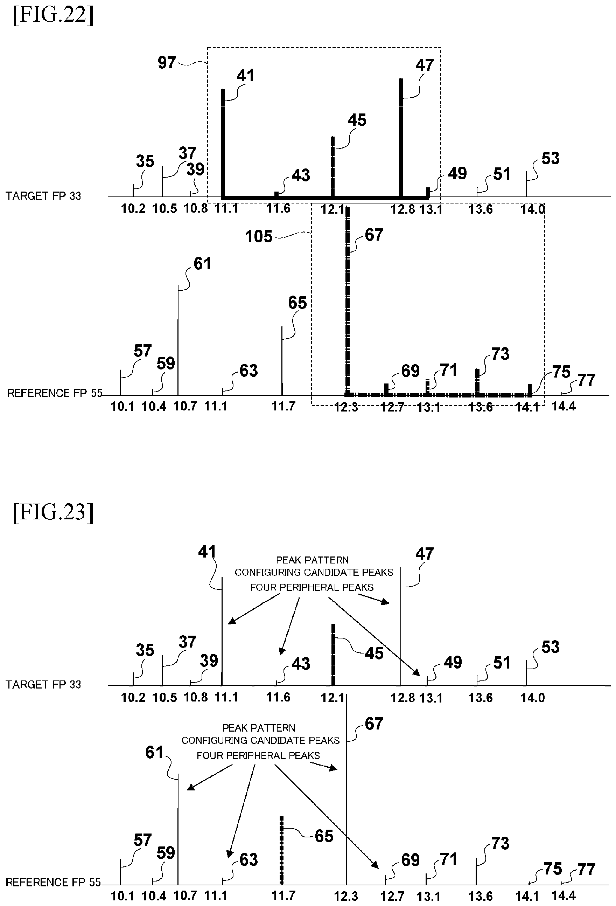

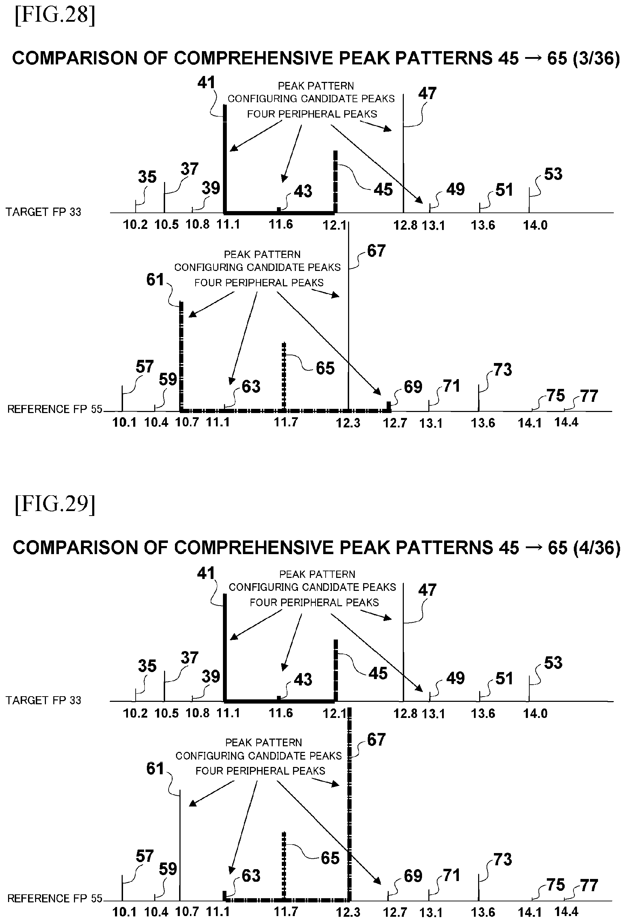

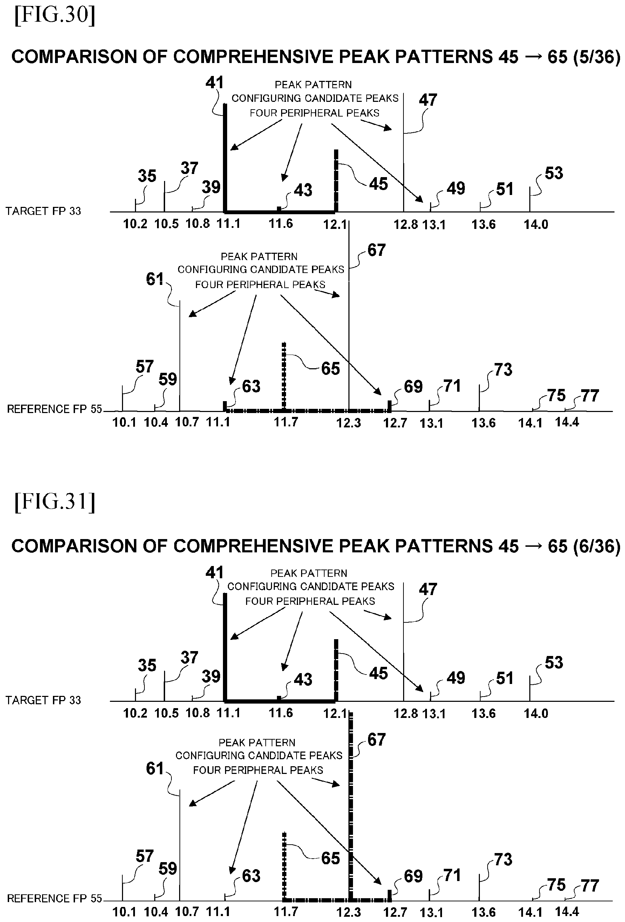

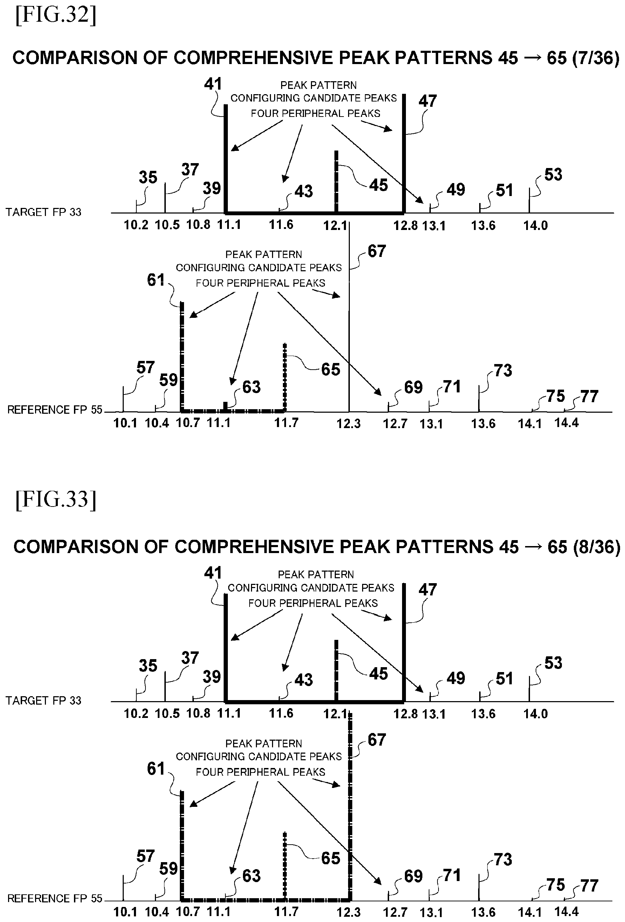

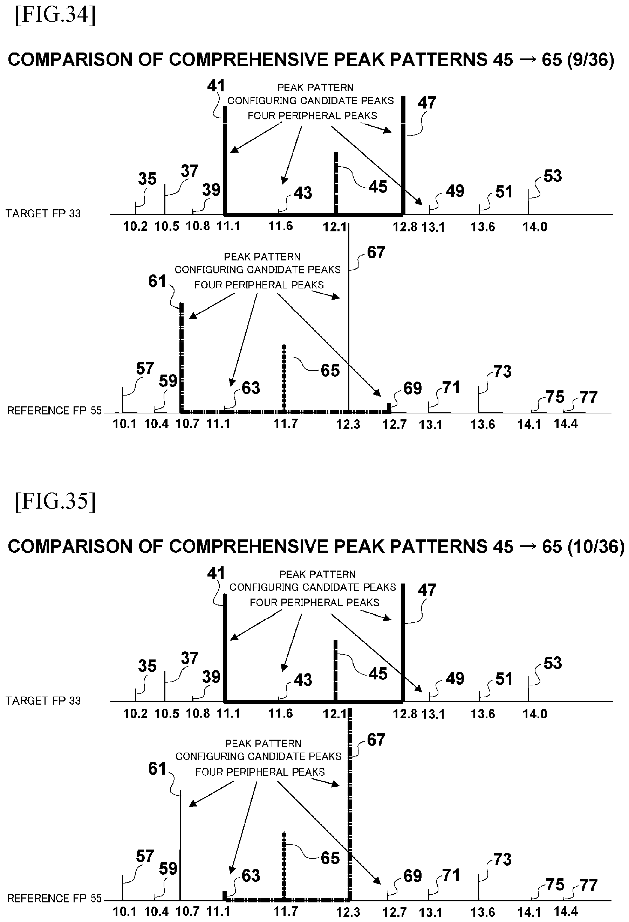

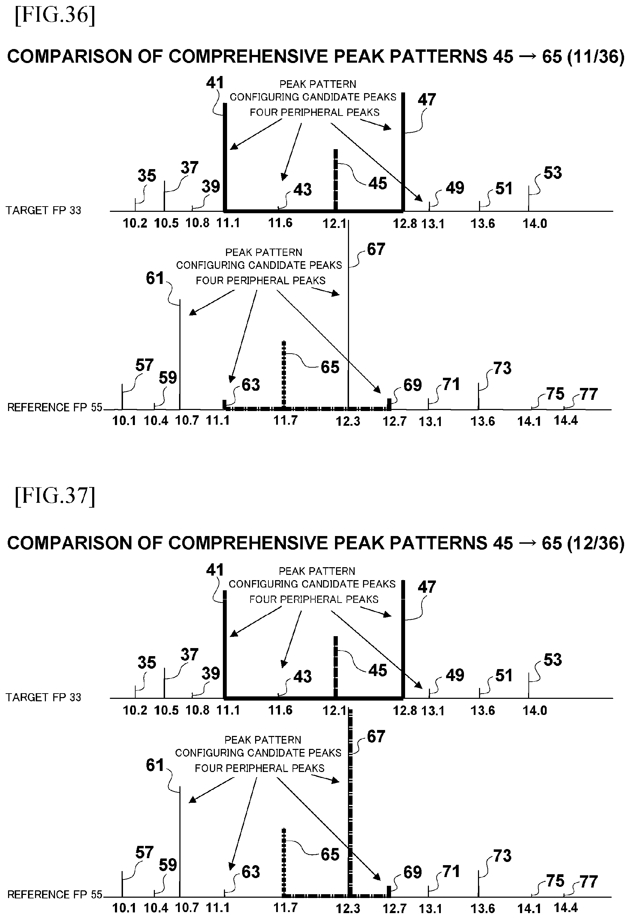

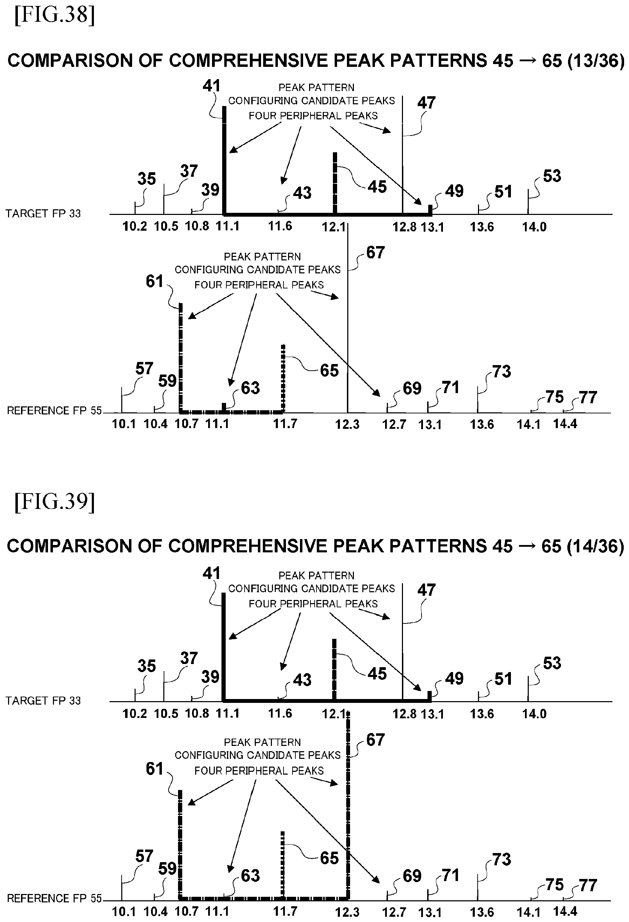

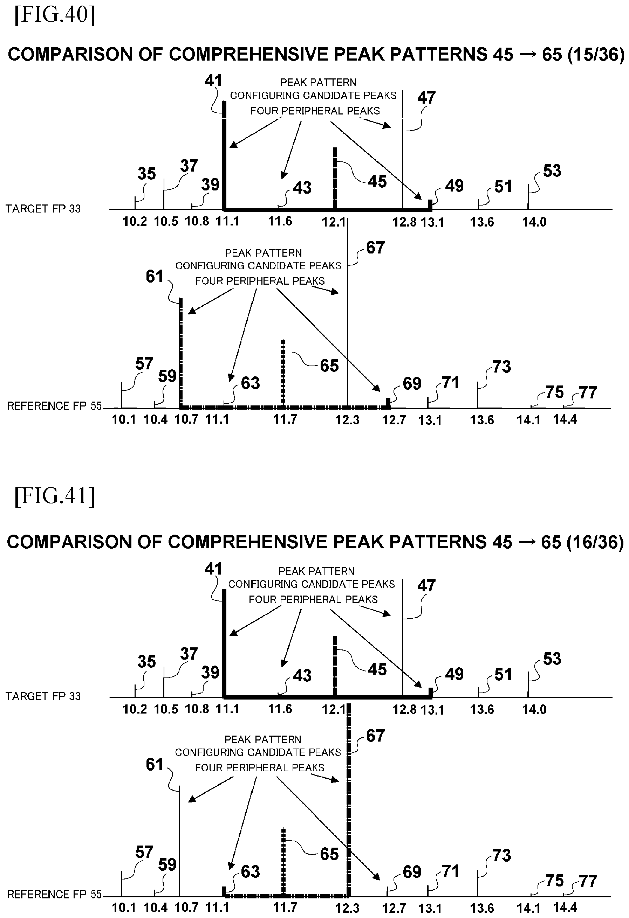

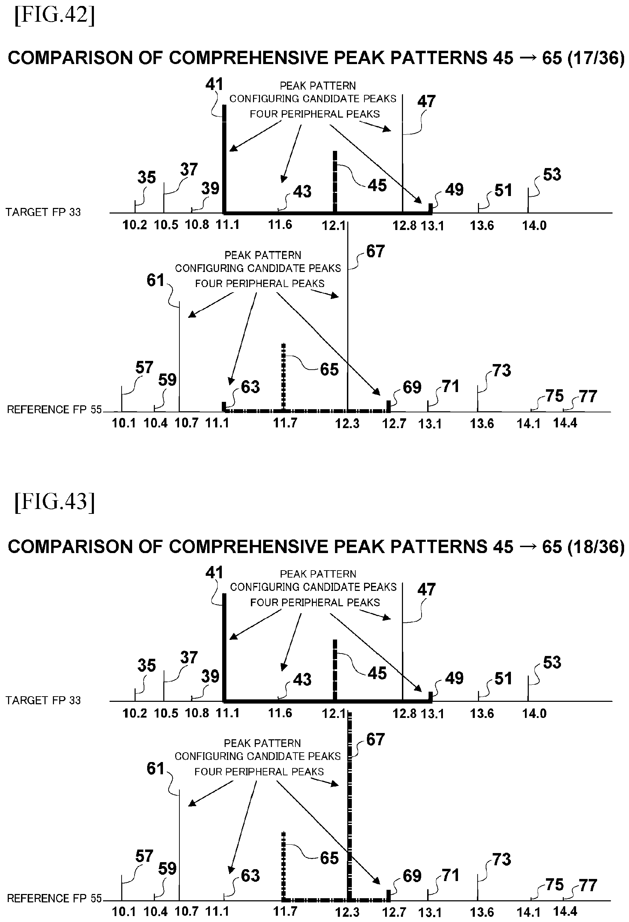

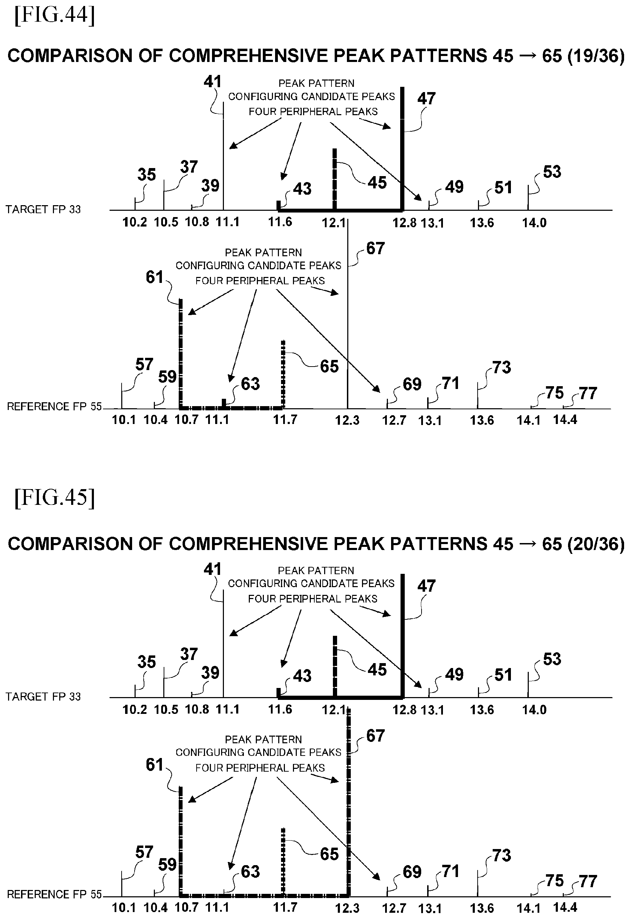

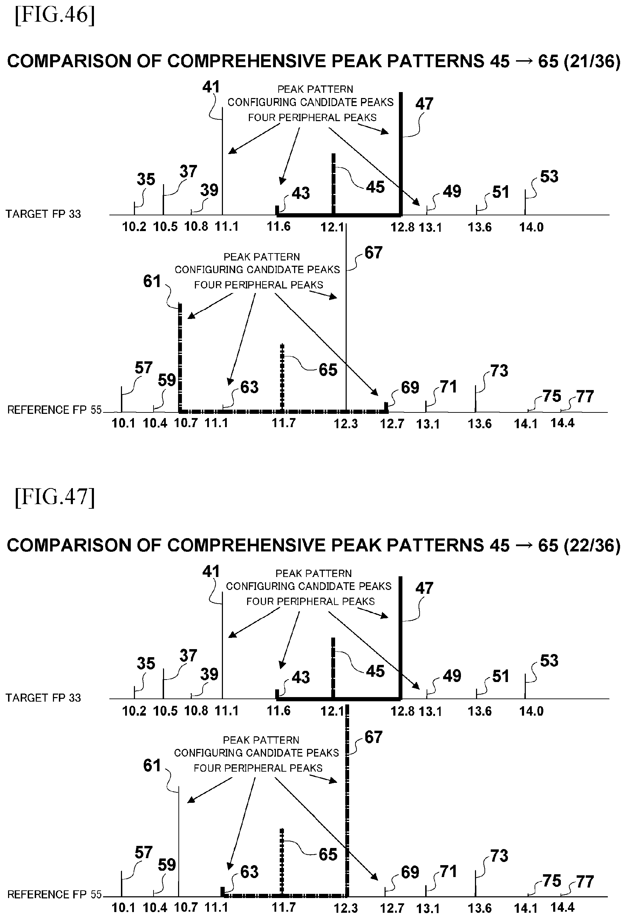

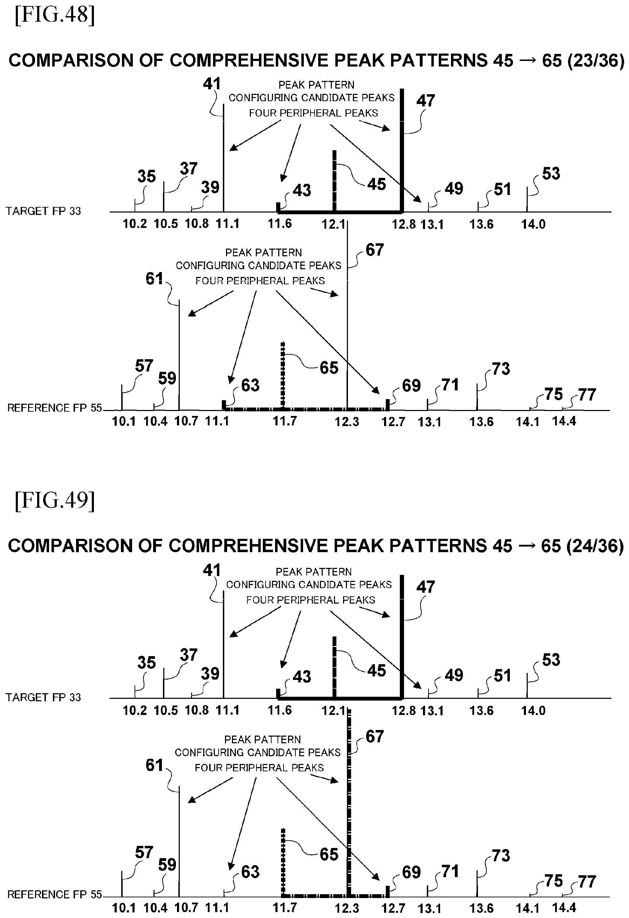

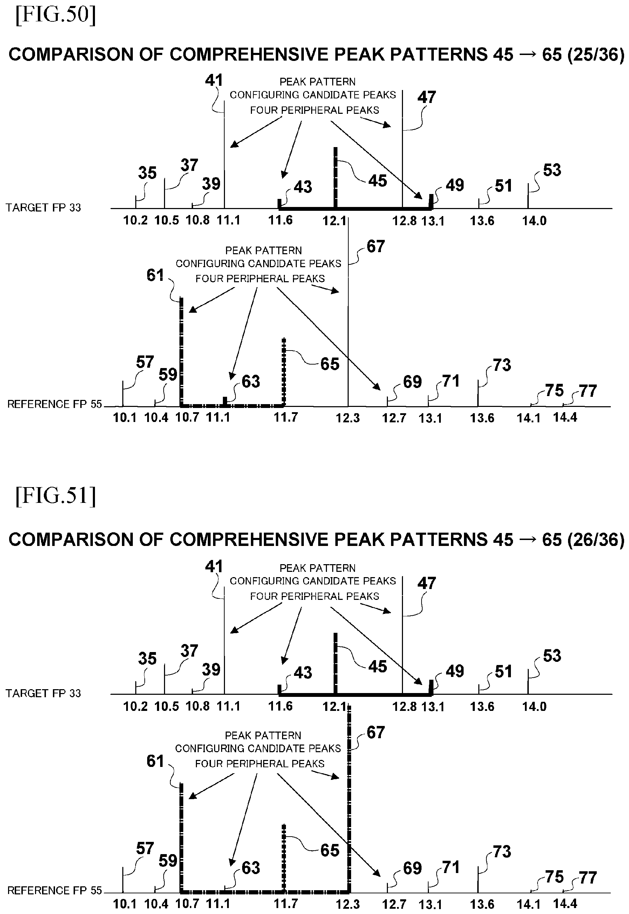

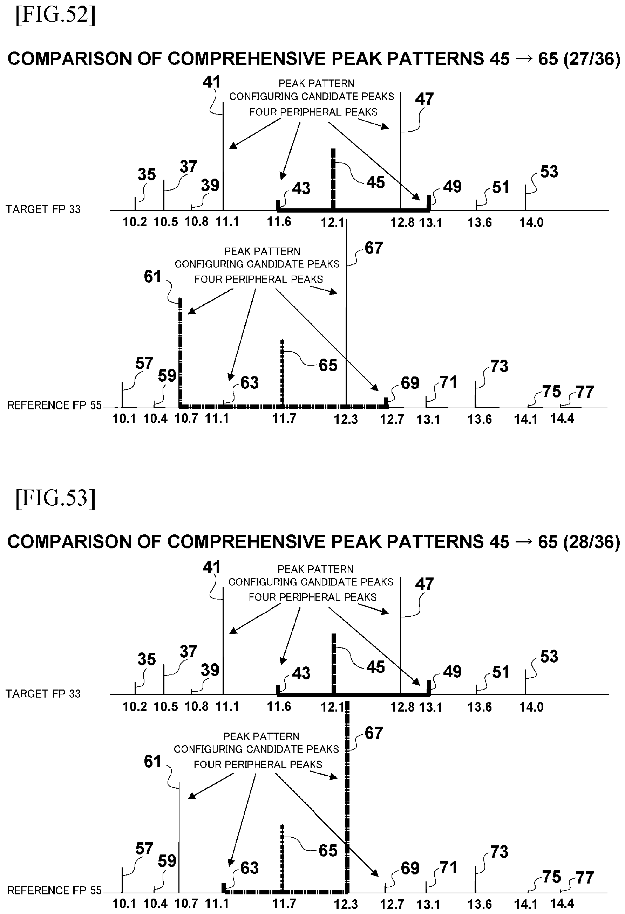

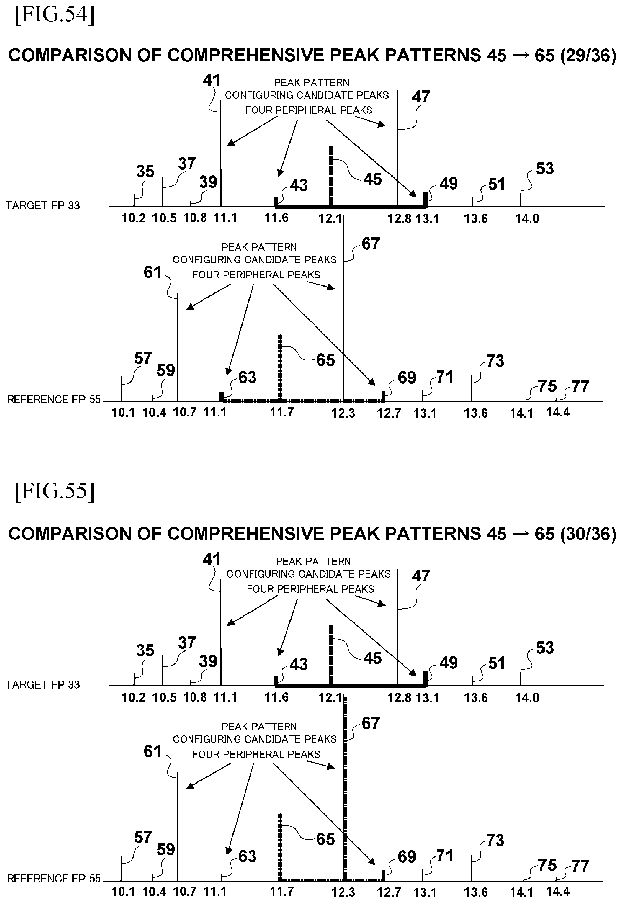

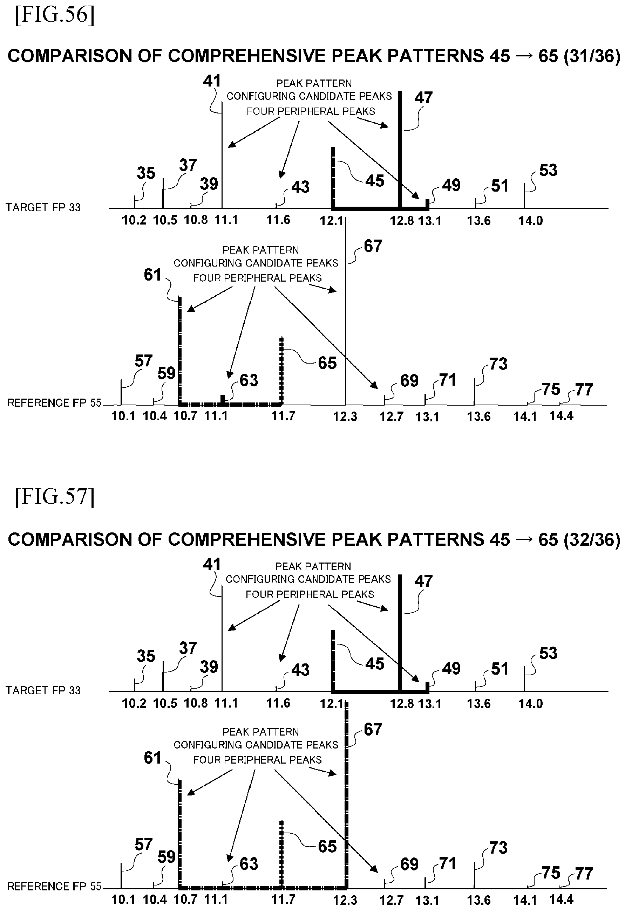

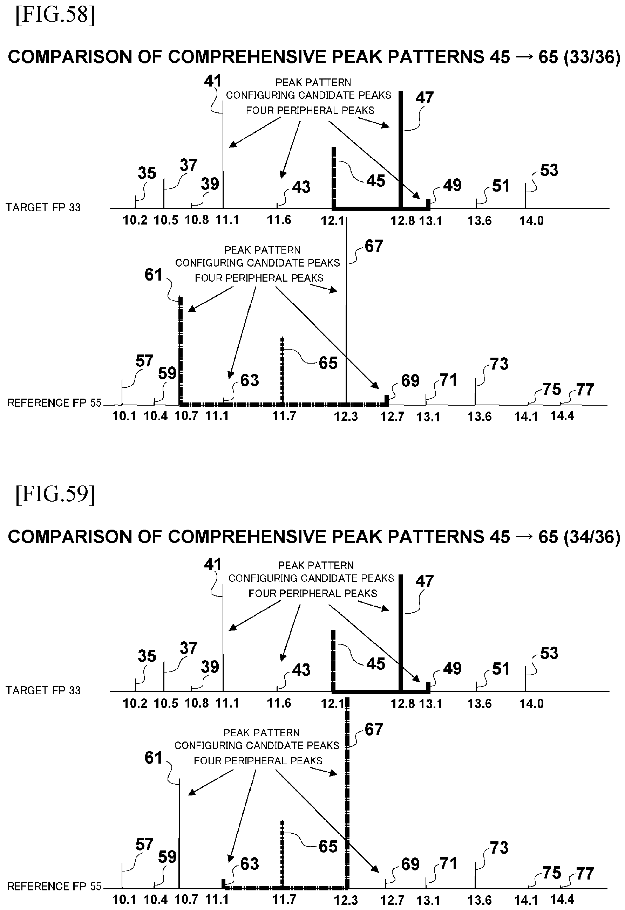

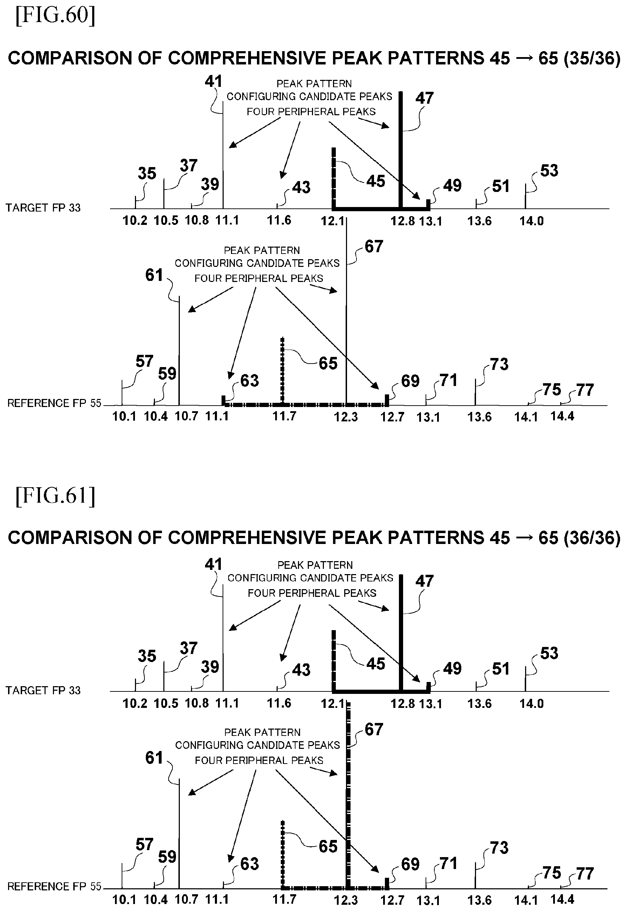

In addition, the peak pattern preparing part 7 is configured to be able to flexibly respond to even a case where there is a difference between the number of the peaks of the target FP 33 and that of the reference FP 55 (in other words, there are one or more peaks that are not present on one side). For this, as illustrated in Figs. 23 to 61 (to be described later), peak patterns are comprehensively prepared by changing peaks configuring the peak patterns (hereinafter, referred to as peak pattern configuring peaks) for both assignment target peaks and assignment candidate peaks. Figs. 23 to 61 illustrate cases where the peak pattern is configured by a total of three peaks including two peaks that are located at least on one of sides located in front and in the rear in the time axis direction.

The peak assigning part 9 is a functional part that compares the individual peak patterns of the target FP and the reference FP to specify corresponding peaks. In the embodiment, the corresponding peaks are specified by calculating the degree of matching between peak patterns for assignment target peaks and assignment candidate peaks and the degree of matching between the UV spectra. It will be described specifically later.

In addition, the peak assigning part 9 is a functional part that calculates the degrees of matching for the assignment candidate peaks by integrating aforementioned two kinds of the degrees of matching to assign each peak of the target FP 33 to each peak of the reference FP 55 based on the calculated degrees of matching.

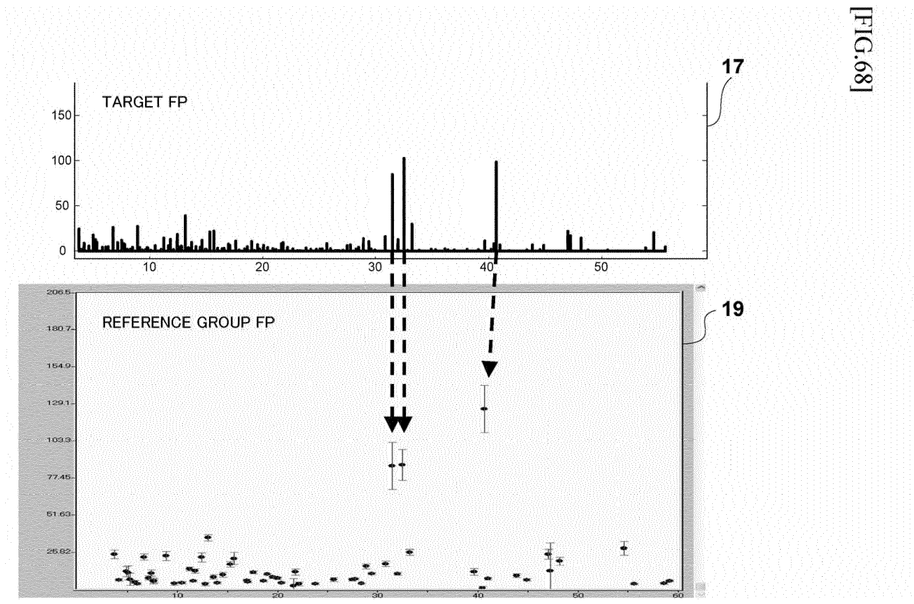

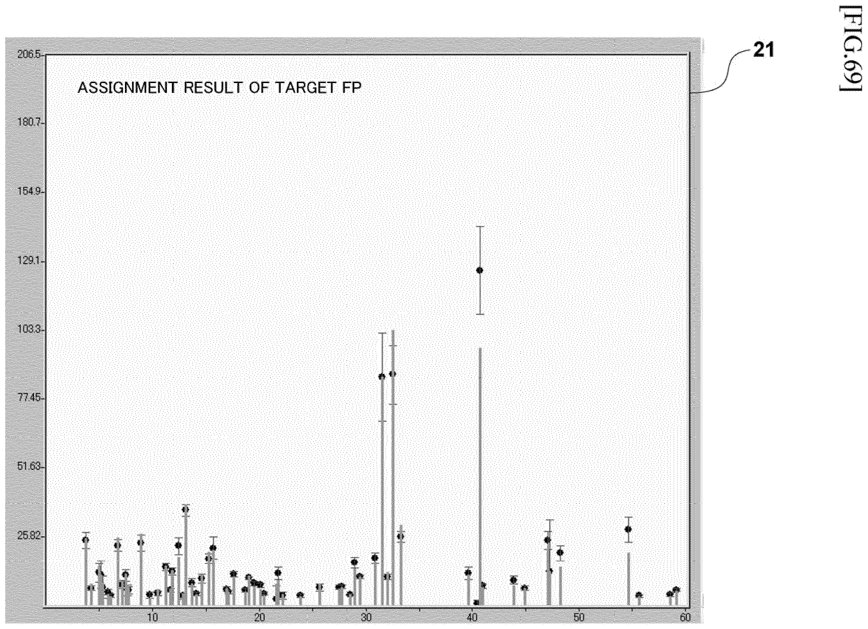

Furthermore, the peak assigning part 9 is a functional part that finally assigns the peaks of the target FP to respective peaks of the reference group FP as illustrated in Figs. 68 and 69 (to be described later), based on a result of the assignment between the target FP 33 and the reference FP 55.

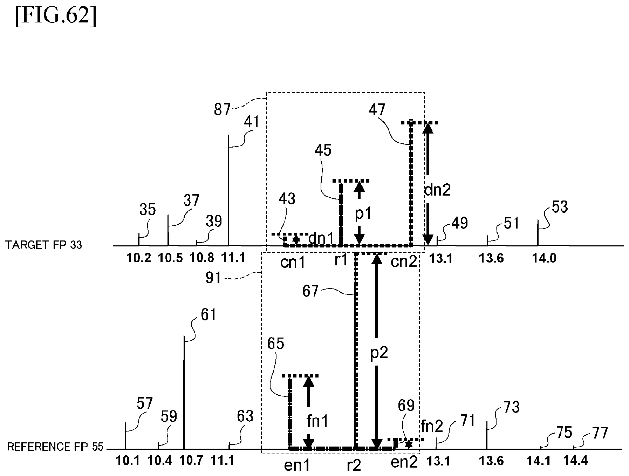

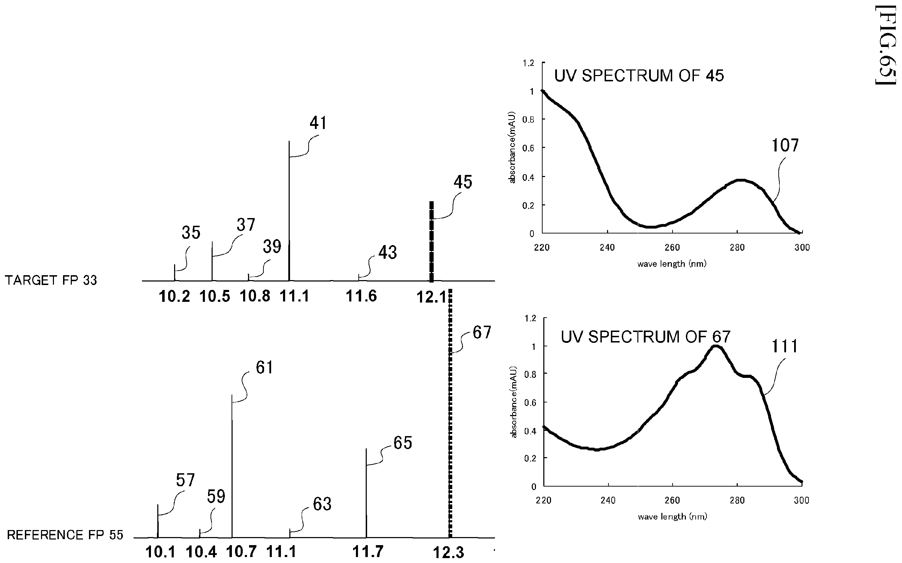

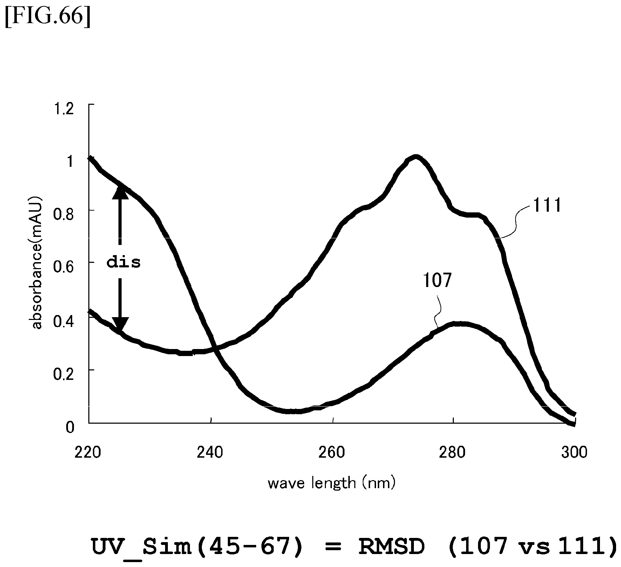

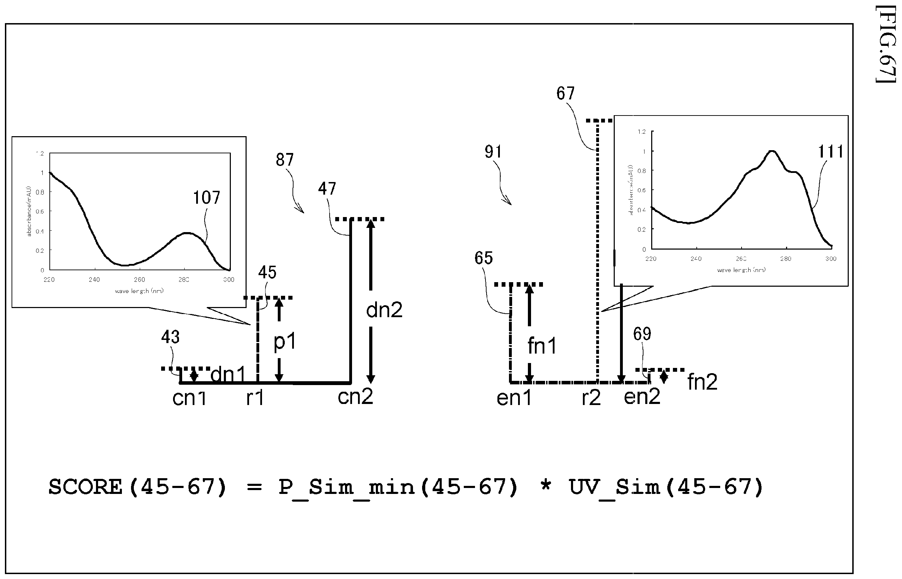

The peak assigning part 9 calculates the degree of matching between peak patterns based on differences between corresponding peaks and retention time points of the peak patterns of the assignment target peak and the assignment candidate peak as illustrated in Figs. 62 to 64 (to be described later). The degree of matching between the UV spectra is calculated based on a difference between the absorbance of the UV spectrum 107 of an assignment target peak 45 and the absorbance of the UV spectrum 111 of a assignment candidate peak 67 for each wavelength as illustrated in Figs. 65 and 66 (to be described later). Further, as illustrated in Fig. 67 (to be described later), the degree of matching of the assignment candidate peak 67 is calculated by multiplying these two kinds of the degrees of matching together.

The evaluating part 11 is a functional part that evaluates the peaks that are specified and assigned by the peak assigning part 9 by comparison with the peaks of the plurality of reference FPs. In the embodiment, the evaluating part 11 is a functional part that evaluates the equivalency between the target FP assignment peaks 21 and the reference group FP 19 with MT method.

MT method represents a calculation technique that is generally known in quality engineering. For example, MT method is described in pp 136 to 138, "Mathematics for Quality Engineering" published by Japanese Standards Association (2000); in pp 454 to 456 of Quality Engineering of Application Course "Technical Developments in Chemistry, Pharmacy and Biology" published by Japanese Standards Association (1999); in pp 78 to 84 of Quality Engineering 11(5) (2003); and in "Introduction to MT System" (2008).

In addition, MT method program software that is commercially available in the market can be used. As such commercially-available MT method program software, there are "ATMTS" provided by Angle Try Associates, "TM-ANOVA" provided by Japanese Standards Association, an "MT method for Windows" provided by OHKEN Co., Ltd, and the like.

The evaluating part 11 assigns a variable axis according to MT method to one of the lot number and the retention time point of a kampo medicine or the UV detection wavelength of the target FP 17 and sets the peaks as feature values according to MT method.

Although the assignment of the variable axis is not particularly limited, it is preferable that the retention time point is assigned to a so-called category axis according to MT method, the number of a multicomponent-based drug is assigned to a so-called number row axis, and the peak is assigned to a so-called feature value according to MT method.

Here, the category axis and the number row axis are defined as below. According to MT method, an average value $m_j$ and a standard deviation $\sigma_j$ are acquired for a data set $X_{ij}$, a correlation coefficient "r" between "i" and "j" is acquired from a value $x_{ij} = (X_{ij} - m_j)/\sigma_j$ that is the standardized $X_{ij}$, and accordingly, a unit space or a Mahalanobis distance is acquired. At this time, the category axis and the number row axis are defined such that "the average value $m_j$ and the standard deviation $\sigma_j$ are acquired for each value of the category axis by changing the value of the number row axis."

Based on the data and the feature values to which the axes are assigned, a reference point and an unit quantity (it may be abbreviated as a "unit space") are acquired using MT method. Here, the reference point, the unit quantity, and the unit space are defined in accordance with the description of MT method presented in the above-described literatures.

According to MT method, an MD value is acquired as a value that represents the degree of a difference between a drug to be evaluated and the unit space. Here, the MD value is defined in the same way as the description of MT method presented in the literatures, and the MD value is acquired with the method described in the literatures.

By using the MD value acquired in this manner, the drug to be evaluated can be evaluated by determining the degree of a difference from a plurality of drugs defined as normal products.

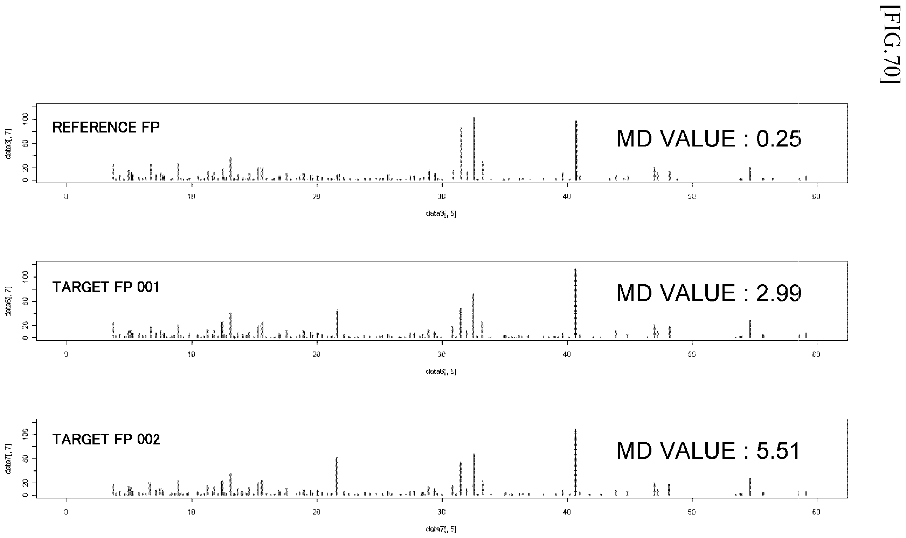

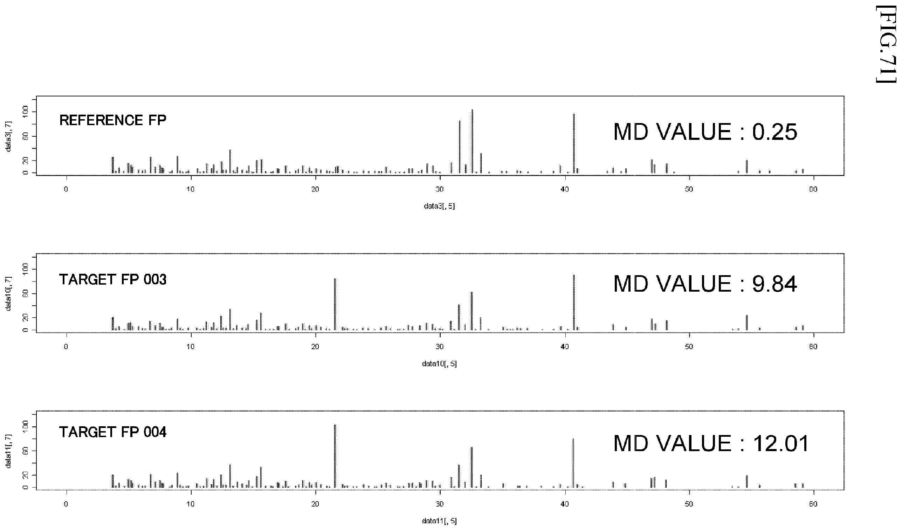

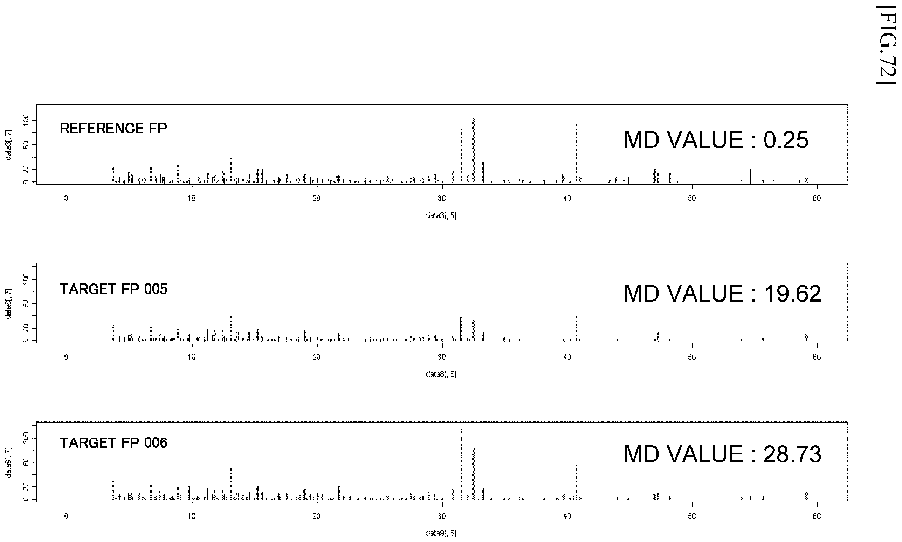

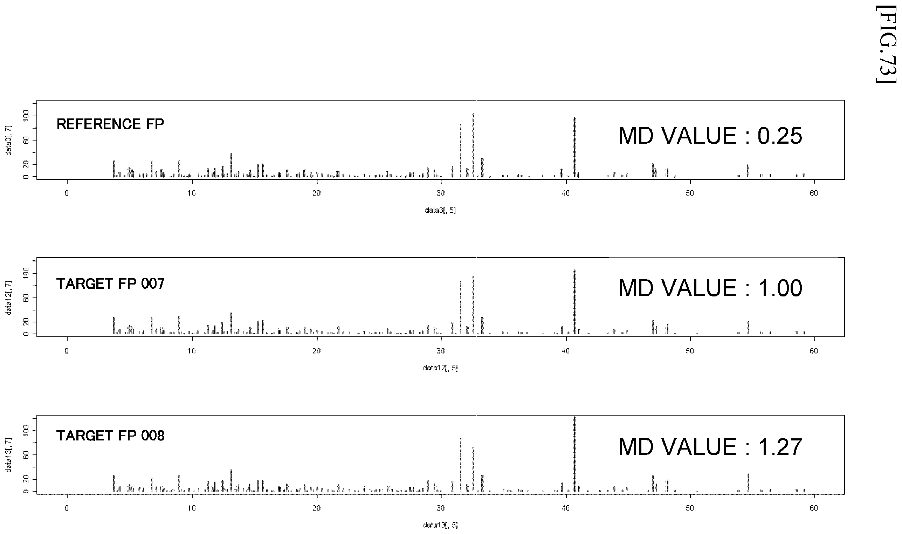

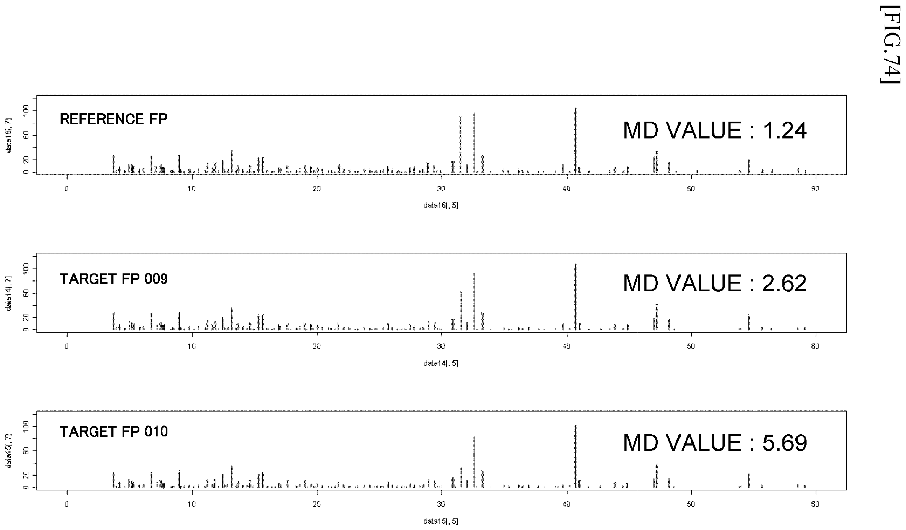

For example, by performing the assignment process for each target FP illustrated in Figs. 70 to 74 as above, a MD value (MD value: 0.25, 2.99, or the like) can be acquired in accordance with MT method.

When this MD value is evaluated with respect to an MD value of a normal product, MD values are similarly acquired for a plurality of drugs defined as normal products. A threshold value is set from the MD values of these normal products, the MD value of the evaluation target drug is plotted as an evaluation result 23 of the evaluating part 11 illustrated in Fig. 2 to determine whether a normal product or an abnormal product. In the evaluation result 23 of the evaluating part 11 illustrated in Fig. 2, for example, an MD value of 10 or less is determined as a normal product.

In addition, it is sufficient for the evaluating part 11 to be able to compare and evaluate the equivalency between the target FP assignment peaks 21 and the reference group FP 19, and therefore, a pattern recognition technique other than MT method or the like can be used.

[Operating Principle of Peak Pattern Process]

Figs. 5 to 67 illustrate an operating principle of the reference FP selecting part 5, the peak pattern preparing part 7, the peak assigning part 9, and the evaluating part 11.

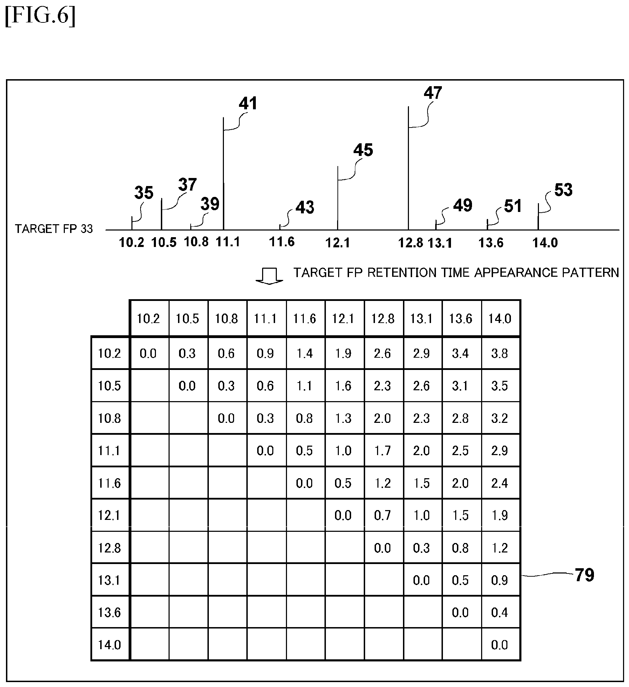

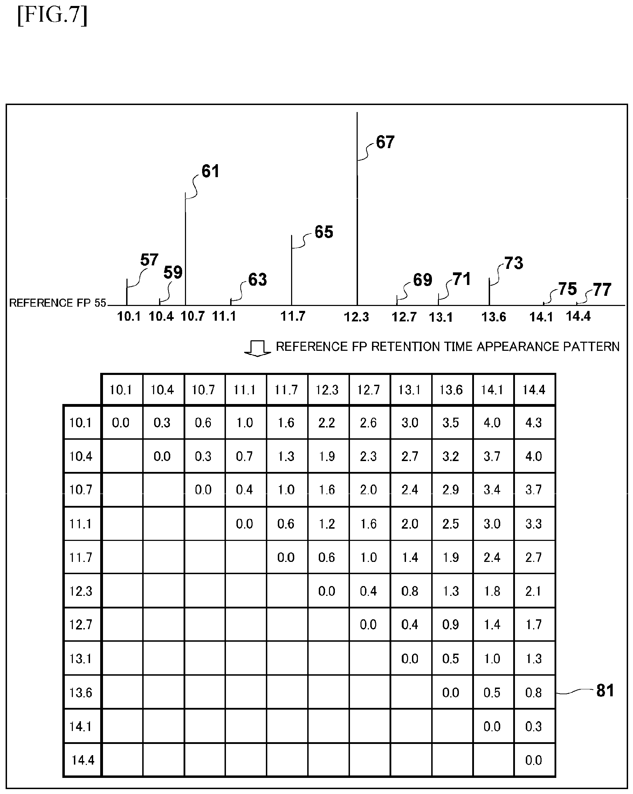

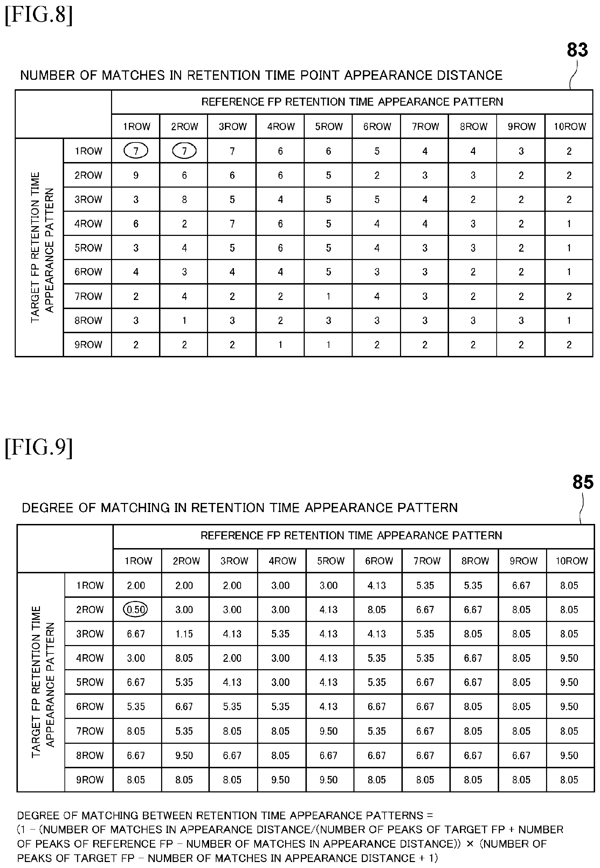

Figs. 5 to 9 are diagrams each illustrating the degree of matching between the retention time appearance patterns of the target FP and the reference FP according to the reference FP selecting part 5. Fig. 5 is a diagram illustrating the retention time points of the target FP and the reference FP, Fig. 6 is a diagram illustrating the retention time appearance pattern of the target FP, and Fig. 7 is a diagram illustrating the retention time appearance pattern of the reference FP. Fig. 8 is a diagram illustrating the number of matches in the retention time appearance distance between the target FP and the reference FP, and Fig. 9 is a diagram illustrating the degrees of matching between the retention time appearance patterns of the target FP and the reference FP.

Fig. 5 shows the retention time points of the target FP 33 and the reference FP 55. Figs. 6 and 7 show the retention time appearance patterns in which all of inter-retention time point distances calculated based on the retention time points of the target FP 33 and the reference FP 55 are arranged in a table form. Fig. 8 shows the numbers of matches between the retention time appearance distances calculated based on the appearance patterns and arranged in a table form. Fig. 9 shows the degrees of matching between the retention time appearance patterns calculated based on the number of matches and arranged in a table form. Figs. 10 to 12 are diagrams explaining a peak pattern that is prepared with use of an assignment target peak and peripheral peaks thereof by the peak pattern preparing part 7. Fig. 10 is a diagram that shows the assignment target peak of the target FP, Fig. 11 is diagram that shows a peak pattern prepared with use of three peaks including two peripheral peaks, and Fig. 12 is a diagram that shows a peak pattern prepared with use of five peaks including four peripheral peaks.

Figs. 13 and 14 explain a relation between the assignment target peak and assignment candidate peaks according to the peak pattern preparing part 7, Fig. 13 is a diagram illustrating an allowable range of the assignment target peak, and Fig. 14 is a diagram illustrating assignment candidate peaks of the reference FP for the assignment target peak.

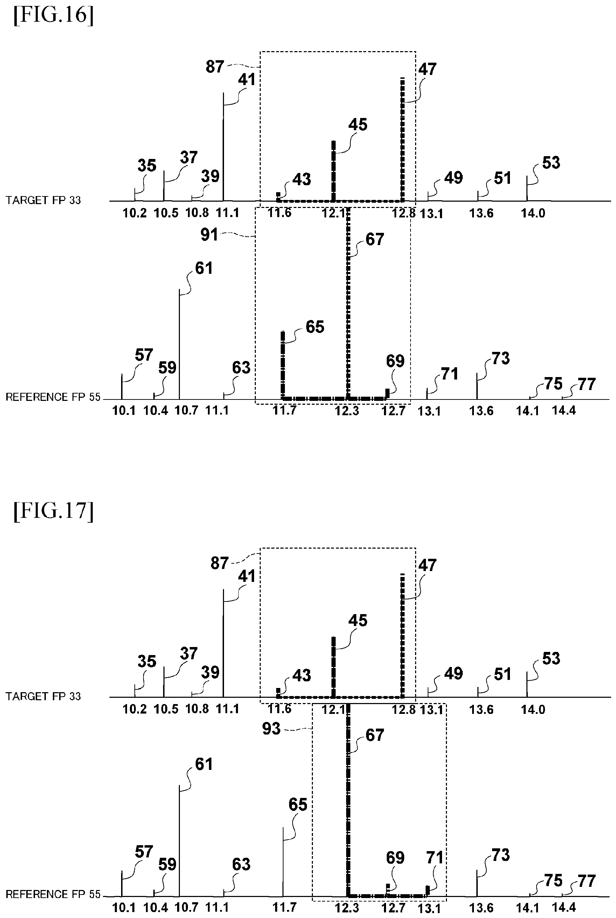

Figs. 15 to 18 are peak pattern examples of the assignment target peak and assignment candidate peak that are prepared by three peaks according to the peak pattern preparing part 7. Fig. 15 is a peak pattern diagram according to three peaks of the assignment target peak and assignment candidate peaks, Fig. 16 is a peak pattern diagram according to three peaks of another assignment candidate peaks for the assignment target peak, Fig. 17 is a peak pattern diagram according to three peaks of another assignment candidate peaks for the assignment target peak, and Fig. 18 is a peak pattern diagram according to three peaks of another assignment candidate peaks for the assignment target peak.

Figs. 19 to 22 are peak pattern diagrams of an assignment target peak and assignment candidate peak that are prepared with use of five peaks according to the peak pattern preparing part 7.

Figs. 23 to 61 are diagrams explaining the principle of comprehensive comparison in which peak patterns of the assignment target peak and assignment candidate peak according to the peak pattern preparing part 7 are comprehensively prepared and compared with each other.

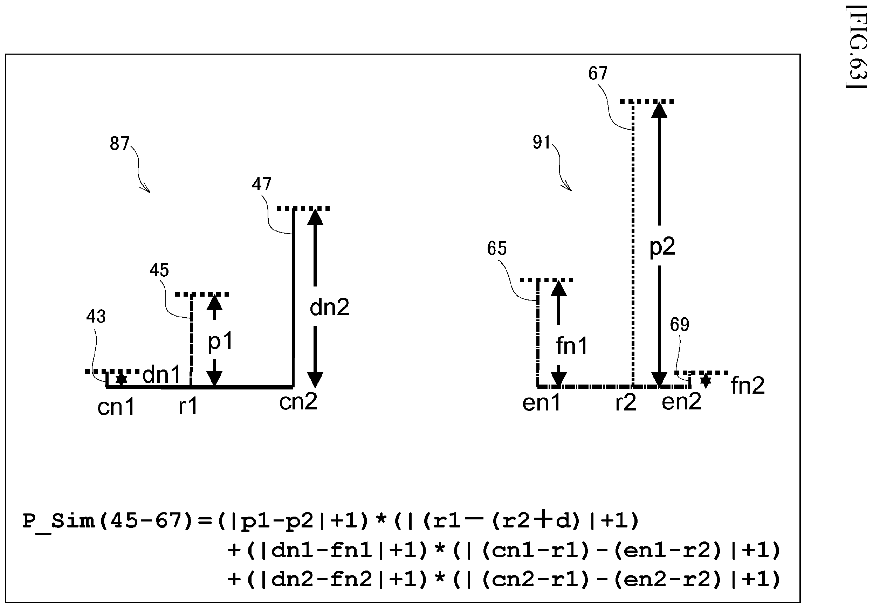

Figs. 62 and 63 are diagrams explaining a calculating method of the degree of matching between peak patterns prepared with use of three peaks according to the peak assigning part 9.

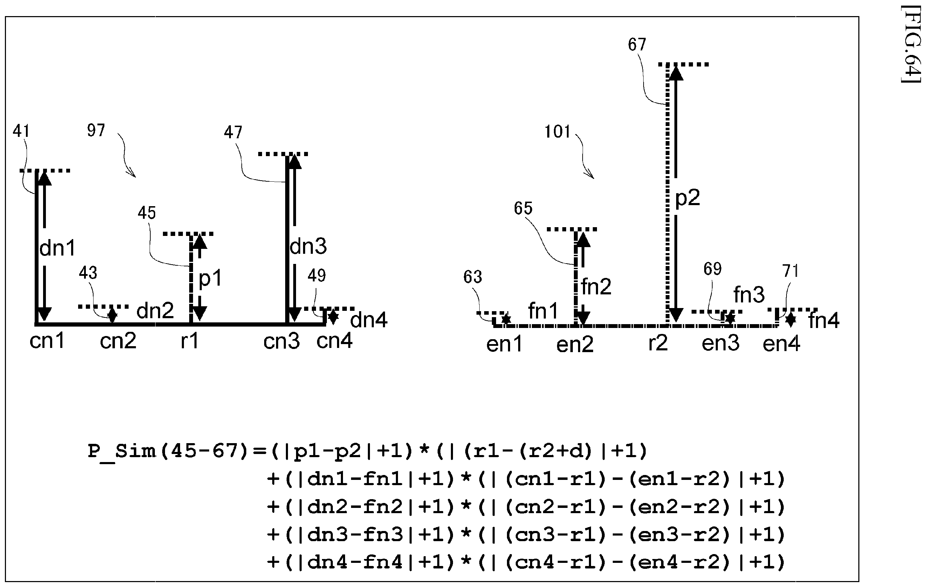

Fig. 64 is a diagram explaining a calculating method of the degree of matching between peak patterns prepared with use of five peaks according to the peak assigning part 9.

Fig. 65 is a diagram illustrating UV spectra 107 and 111 of the assignment target peak 45 and the assignment candidate peak 67 according to the peak assigning part 9.

Fig. 66 is a diagram explaining the degree of matching between the UV spectrum 107 of the assignment target peak 45 and the UV spectrum 111 of the assignment candidate peak 67 according to the peak assigning part 9.

Fig. 67 is a diagram explaining the degree of matching of the assignment candidate peak that is calculated based on the degree of matching between peak patterns of the assignment target peak 45 and the assignment candidate peak 67 and the degree of matching between UV spectra according to the peak assigning part 9.

Fig. 68 is a diagram explaining the assignment of each peak of the target FP 17 to the reference group FP 19 according to the peak assigning part 9.

Fig. 69 is a diagram explaining a target FP peak feature value 21 that represents a state in which each peak of the target FP 17 is assigned to the reference group FP 19 according to the peak assigning part 9.

Figs. 70 to 74 are diagrams illustrating various target FPs and evaluation values (MD values) thereof according to the evaluating part 11.

[Selection of Reference FP]

The function of the above-described reference FP selecting part 5 will be further described with reference to Figs. 5 to 9.

Fig. 5 is the diagram illustrating the retention time points of the target FP and the reference FP, Fig. 6 is the diagram illustrating the retention time appearance pattern of the target FP, and Fig. 7 is the diagram illustrating the retention time appearance pattern of the reference FP. Fig. 8 is the diagram illustrating the number of matches in the retention time appearance distance between the target FP and the reference FP, and Fig. 9 is the diagram illustrating the degrees of matching between the retention time appearance patterns of the target FP and the reference FP.

Fig. 5 shows the retention time points of the target FP 33 and the reference FP 55. Figs. 6 and 7 show the retention time appearance patterns in which all of inter-retention time point distances calculated based on the retention time points of the target FP 33 and the reference FP 55 are arranged in a table form. Fig. 8 shows the numbers of matches between the retention time appearance distances calculated based on the appearance patterns and arranged in a table form. Fig. 9 shows the degrees of matching between the retention time appearance patterns calculated based on the number of matches and arranged in a table form.

In the peak assigning process for the target FP 33, the peaks of the target FP 33 are assigned to a reference FP whose FP pattern is closest to the target FP 33 as much as possible. Selecting this reference FP that is closest to the target FP 33 from among a plurality of reference FPs is an important point for performing assignment with high accuracy.

Thus, as a method of evaluating similarity to a FP pattern of the target FP 33 in an objective and simplified manner, the similarity of the FP pattern is evaluated based on the degree of matching between the retention time appearance patterns.

For example, in a case where the retention time points of the target FP 33 and the reference FP 55 are as illustrated in Fig. 5, retention time appearance patterns of the target FP 33 and the reference FP 55 are formed as illustrated in Figs. 6 and 7. In Figs. 6 and 7, for the target FP 33 and the reference FP 55 illustrated on the upper side, as tables illustrated on the lower side, patterns are prepared in the form of tables in which the value of each cell is configured by an inter-retention time point distance.

In Fig. 6, the retention time points of peaks (35, 37, 39, 41, 43, 45, 47, 49, 51, and 53) of the target FP 33 are (10.2), (10.5), (10.8), (11.1), (11.6), (12.1), (12.8), (13.1), (13.6), and (14.0).

Accordingly, an inter-retention time point distance between the peaks 35 and 37 is (10.5) - (10.2) = (0.3). Similarly, a distance between the peaks 35 and 39 is (0.6), a distance between the peaks 37 and 39 is (0.3), etc. The followings are similarly acquired and a target FP appearance pattern 79 is formed into a table on the lower side of Fig. 6.

In Fig. 7, the retention time points of the peaks (57, 59, 61, 63, 65, 67, 69, 71, 73, 75, and 77) of the reference FP 55 are (10.1), (10.4), (10.7), (11.1), (11.7), (12.3), (12.7), (13.1), (13.6), (14.1), and (14.4).

Accordingly, in the same way, inter-retention time point distances form a reference FP appearance pattern 81 into a table on the lower side of Fig. 7.

The individual peaks patterned as illustrated in Figs. 6 and 7 are compared in a round-robin system so as to acquire the number of matches. For example, the value of each cell of the target FP appearance pattern represented in the table illustrated on the lower side of Fig. 6 is compared with the value of each cell of the reference FP appearance pattern represented in the table on the lower side of Fig. 7, thereby acquiring the number 83 of matches as illustrated in Fig. 8.

Namely, all the inter-retention time point distances of the retention time appearance patterns of the target FP 33 and the reference FP 55 are sequentially compared with each other in units of rows in a round-robin system, thereby calculating the number of the distances that match within a set range.

For example, comparing the first rows of the target and reference FP retention time appearance patterns 79 and 81 in Figs. 6 and 7, the number of matches is seven. This number of matches of seven is written into the first row of the target and reference FP retention time appearance pattern illustrated in Fig. 8. For the other rows in Figs. 6 and 7, similarly, the first to ninth rows of the target FP retention time appearance pattern is compared with the first to tenth rows of the reference FP retention time appearance pattern in a round-robin system, thereby acquiring the numbers of matches, respectively.

The results are represented in Fig. 8. In Fig. 8, a leftmost circled number of 7 is a result of the comparison between the first rows of the target and reference FP retention time appearance patterns, and a number of 7 represented next thereto is a result of the comparison between the first row of the target FP retention time appearance pattern and the second row of the reference FP retention time appearance pattern. The set range is preferably in the rage of 0.05 to 0.2 minutes, but is not limited thereto. In Embodiment 1, the set range is 0.1 minutes.

When the degree of matching between retention time appearance patterns is RP, a degree ($RP_{fg}$) of matching between a retention time appearance pattern of the f-th row of the target FP 33 and a retention time appearance pattern of the g-th row of the reference FP 55 is calculated using Tanimoto coefficient as:

$$RP_{fg} = \{1 - (m/(a + b - m))\} \times (a - m + 1).$$

In the equation, "a" is the number of peaks of the target FP 33 (the number of target FP peaks), "b" is the number of peaks of the reference FP 55 (the number of reference FP peaks), and "m" is the number of matches in an appearance distance (see Fig. 8). The degree (RP) of matching between retention time appearance patterns is calculated by the above-described equation based on the number 83 of matches in Fig. 8 (see the degree 85 of matching in Fig. 9).

A minimum value (RP_min) of these RPs is set as the degree of matching between the retention time appearance patterns of the target FP 33 and the reference FP 55. In the case of Fig. 9, (0.50) is the degree of matching of the target FP 33 with respect to the reference FP.

The degrees of matching are calculated for all the reference FPs, and a reference FP having the smallest degree of matching is selected, and the peaks of the target FP are assigned to the reference FP.

The reference FP selecting part 5 may pattern the target FP 33 and the reference FP 55 at peak heights ratios.

The peaks patterned with use of the peak height ratios are compared in a round-robin system, to calculate the number of matches in the height ratio within a set range. By performing the calculation, similarly to the case of Fig. 8, the number of matches can be acquired.

In addition, in the case where the peaks are patterned at the peak height ratios, there is a case where a plurality of similar values are present in one row, and thus these values are required not to be counted a plurality of times.

The degree of matching can be acquired by setting the Tanimoto coefficient as "the number of matches in height ratio/(the number of target FP peaks + the number of reference FP peaks - the number of matches in the height ratio)" and approaching (1 - Tanimoto coefficient) to zero.

In addition, (1 - Tanimoto coefficient) is weighted by (the number of target FP peaks - the number of matches in height ratio + 1) to be "(1 - Tanimoto coefficient) $\times$ (the number of target FP peaks - the number of matches the an appearance distance or the height ratio + 1", whereby a reference FP that matches more peaks (35, 37, ···) of the target FP 33 in accordance with the weighting can be selected.

[Feature value Prepared In Accordance With Peak Pattern]

The functions of the peak pattern preparing part 7 and the peak assigning part 9 will be described further with reference to Figs. 10 to 69.

When the assignment target peak 45 is assigned to one of peaks of the reference FP 55, it works out to that the peak should be assigned to which one of the peaks as illustrated in Fig. 10. If this peak assignment is carried out based on only information of the peak retention time points or UV spectra, sufficient accuracy cannot be acquired by the peak assignment based on the single kind of information. This is because all the three kinds of information include errors due to the inter-drug error and the analysis error.

In addition, as illustrated in Figs. 13 and 14, in a case of setting an allowable range of a deviation between the retention time points of each peak of the assignment target peak 45 and the reference FP 55 and performing peak assignment based on two kinds of information including presence of peaks of the reference FP 55 within the allowable range and UV spectrum information, an assignment destination is determined by synthesizing all the information to improve accuracy compared to the peak assignment according to the single kind of information.

However, even in a case where the peak assignment is performed based on the three kinds of information, UV spectra with similar components are the almost same as the characteristics. Accordingly, if a plurality similar components are included in the assignment candidate peaks, the assignment is consequently performed based on only peak information, whereby sufficient accuracy cannot be acquired. Hence, in order to perform peak assignment with high accuracy, more information is necessary to be added to the three kinds of information.

Then, peak patterns including information of peripheral peaks as illustrated in Figs. 11 and 12 are prepared, and the peak assignment is performed based on the comparison of the peak patterns.

If the peak pattern includes the peripheral peaks, the peripheral information is added to the prior three kinds of information. Accordingly, the peak assignment can be performed based on four kinds of information, whereby higher assignment accuracy can be acquired.

As a result, massive peaks can be efficiently assigned all together through one assignment process with high accuracy.

In addition, by configuring data used for the peak assignment as the four kinds of information including the peripheral information, there is no need of restriction conditions (definition of peaks and the like) to be set in a conventional peak assignment process.

In the case illustrated in Fig. 11, a peak pattern 87 that includes peaks 43 and 47 being present on both sides in the time axis direction is prepared for the assignment target peak 45.

In the case illustrated in Fig. 12, a peak pattern 97 including peaks 41, 43, 47, and 49 that are present on both sides in the time axis direction is prepared for the assignment target peak 45.

In the cases of Figs. 13 and 14, an allowable range of the deviation between the retention time points of each peak of the assignment target peak 45 and the reference FP 55 is set, and peaks of the reference FP 55 that are present within the allowable range are set as candidate peaks (hereinafter, referred to as assignment candidate peaks) that correspond to the assignment target peak 45.

In the case of Fig. 15, as a peak pattern to be compared with the peak pattern 87 for the assignment target peak 45, a peak pattern 89 that includes peaks 63 and 67 being present on both sides located in front and in the rear in the time axis direction is prepared for an assignment candidate peak 65.

In the cases of Figs. 16 to 18, as a peak pattern to be compared with the peak pattern 87 for the assignment target peak 45, peak patterns 91, 93, and 95 that include peaks that are present on both sides located in front and in the rear in the time axis direction are prepared for another assignment candidate peaks 67, 69, and 71, respectively.

In order to compare peak patterns with higher accuracy, it is important to prepare a peak pattern in which the numbers of peripheral peaks are increased for both the target FP and the reference FP as illustrated in Figs. 19 to 22.

For example, by comparing peak patterns having a total of five peaks that includes four peripheral peaks, higher assignment accuracy is acquired.

In the case of Fig. 19, as a peak pattern to be compared with the peak pattern 97 for the assignment target peak 45, a peak pattern 99 that includes peaks 61, 63, 67, and 69 being present on both sides located in front and in the rear in the time axis direction are prepared for the assignment candidate peak 65.

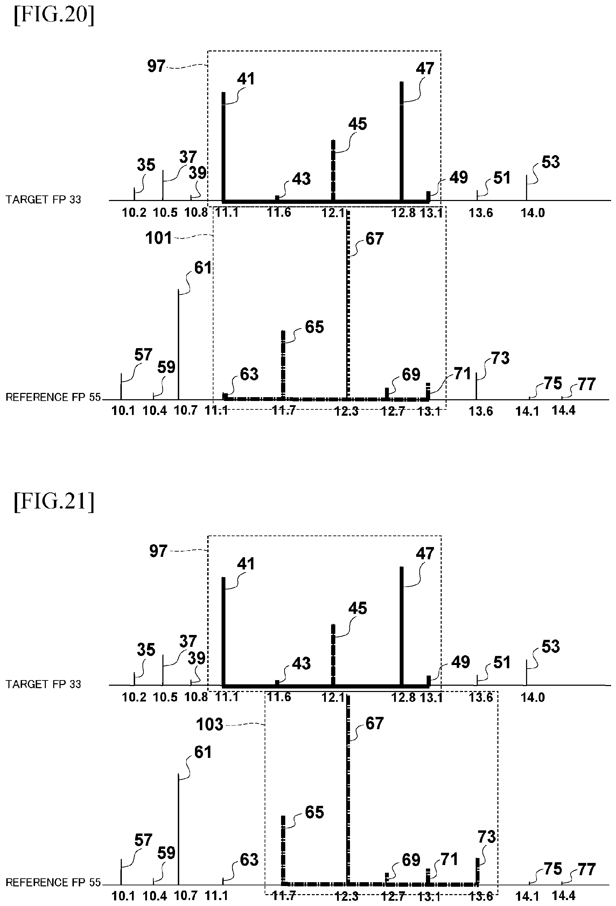

In the cases of Figs. 20 to 22, as a peak pattern to be compared with a peak pattern 97 for the assignment target peak 45, peak patterns 101, 103, and 105 that include peaks being present on both sides located in front and in the rear in the time axis direction are prepared as peak patterns for another assignment candidate peaks 67, 69, and 71, respectively.

In addition, in order to perform the assignment according to the peak patterns with higher accuracy, it is necessary to respond to a case in which there is a difference between the number of peaks of the target FP and the number of peaks of the reference FP (in other words, there is a peak that is not present on one side). For this, it is important to prepare peak patterns in which peak pattern configuring peaks are comprehensively changed for both the assignment target peak and the assignment candidate peak, as illustrated in Figs. 23 to 25.

More specifically, peaks being candidates for the peak pattern configuring peak (hereinafter, peak pattern configuring candidate peaks) are set from among peripheral peaks of the assignment target peak of the target FP in advance. Peak patterns are prepared by setting the peak pattern configuring candidate peaks as the peak pattern configuring peak in turns. Also for the assignment candidate peaks of the reference FP, similarly, peak pattern configuring candidate peaks are set to prepare peak patterns are by setting the peak pattern configuring candidate peaks as the peak pattern configuring peak in turn.

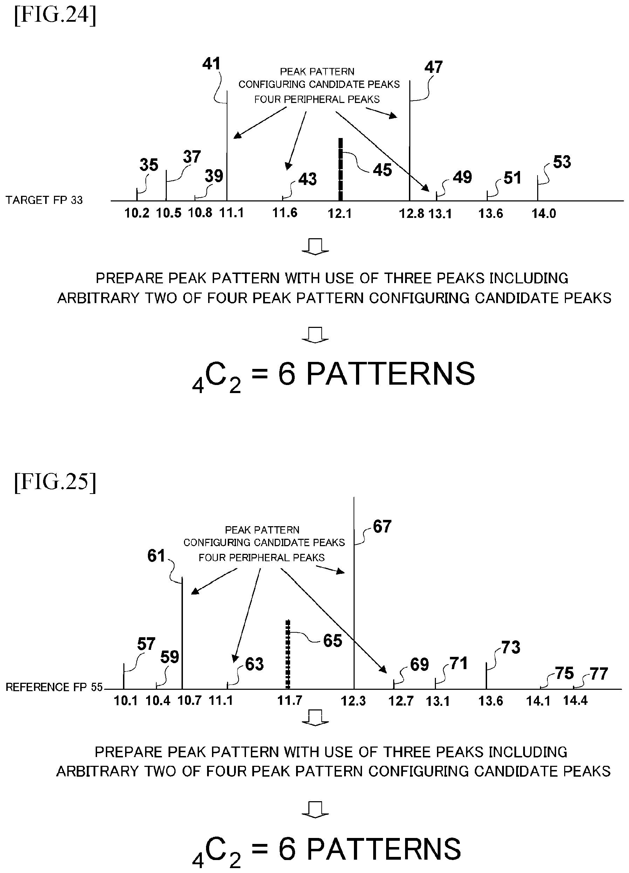

For example, as illustrated in Fig. 23, four peaks (41, 43, 47, and 49) located on the periphery in the time axis direction are set as the peak pattern configuring candidate peaks for the assignment target peak 45, and four peaks (61, 63, 67, and 69) located on the periphery in the time axis direction are set as the peak pattern configuring candidate peaks for the assignment candidate peak 65, and the peak pattern configuring peaks are set to arbitrary two peaks. In this case, peak patterns of 4C2 (= 6) patterns are prepared for each of the assignment target peak 45 and the assignment candidate peak 65 as illustrated in Figs. 24 and 25.

In addition, in a case where ten peak pattern configuring candidate peaks are set, arbitrary two peak pattern configuring peaks are set, and peak patterns of 10C2 (= 45) patterns are prepared for each one of the assignment target peak and the assignment candidate peak. In a case where arbitrary four peaks are set as the peak pattern configuring peaks, peak patterns of 10C4 (= 210) patterns are prepared for each one of the assignment target peak and the assignment candidate peak.

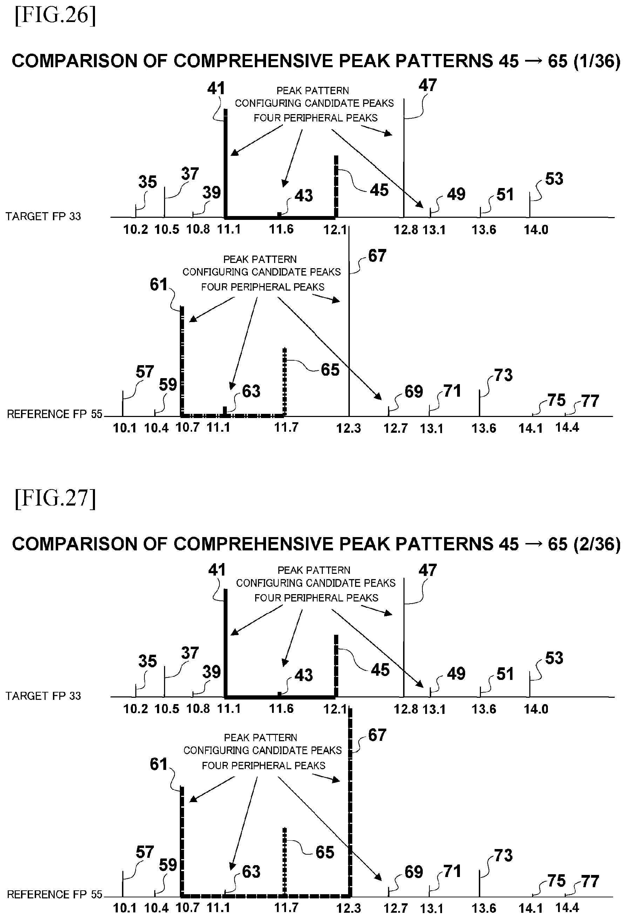

The function of the peak assigning part 9 will be described further with reference to Figs. 26 to 67.

The peak assigning part 9 calculates the degree of matching between peak patterns (hereinafter, referred to as P_Sim) based on differences in corresponding peaks and retention time points over all the peak patterns for the assignment target peak and the assignment candidate peaks prepared by the peak pattern preparing part 7. The peak assigning part 9 sets the minimum value of the P_Sim (hereinafter, referred to as P_Sim_min) as the degree of matching between peak patterns for the assignment target peak and the assignment candidate peak.

For example, as illustrated in Figs. 26 to 61, for each one of the assignment target peak 45 and the assignment candidate peak 65, four peripheral peaks located in front and in the rear in the time axis direction are set as the peak pattern configuring candidate peaks, and two arbitrary peaks are set as the peak pattern configuring peaks. According to this setting, peak patterns of 4C2 (= 6) patterns are prepared for each one of the assignment target peak and the assignment candidate peak. Accordingly, the P_Sims of the assignment target peak 45 and the assignment candidate peak 65 are calculated as 6 patterns × 6 patterns (= 36), and the P_Sim_min that is the minimum value of the P_Sims is set as the degree of matching between the assignment target peak 45 and the assignment candidate peak 65.

Incidentally, in a case where ten peak pattern configuring candidate peaks located in front and in the rear in the time axis direction are set and the peak pattern configuring peaks are set as two arbitrary peaks for each one of the assignment target peak 45 and the assignment candidate peak 65, peak patterns of 4C2 (= 45) patterns are prepared for each one of the assignment target peak and the assignment candidate peak. Accordingly, the P_Sims of the assignment target peak 45 and the assignment candidate peak 65 are calculated as 45 patterns × 45 patterns (= 2025), and the P_Sim_min that is the minimum value of the P_Sims is set as the degree of matching between the assignment target peak 45 and the assignment candidate peak 65. In addition, in a case where the peak pattern configuring peaks are set as four arbitrary peaks, peak patterns of 10C4 (= 210) patterns are prepared for each one of the assignment target peak and the assignment candidate peak. Accordingly, the P_Sims of the assignment target peak 45 and the assignment candidate peak 65 are calculated as 210 patterns × 210 patterns (= 44100), and the P_Sim_min that is the minimum value of the P_Sims is set as the degree of matching between the assignment target peak 45 and the assignment candidate peak 65.

The P_Sim is similarly calculated for all the assignment candidate peaks for the assignment target peak 45.

A calculating method of the degree of matching between peak patterns for comparing peak patterns each configured by three peaks will be described with reference to Figs. 62 and 63. In this case, the peak pattern 87 of the assignment target peak 45 and the peak pattern 91 of the assignment candidate peak 67 will be described as an example.

In the peak pattern 87 of the assignment target peak 45, peak data and a retention time point of the assignment target peak 45 are assumed to be p1 and r1, peak data and a retention time point of a peak pattern configuring peak 43 are assumed to be dn1 and cn1, and peak data and a retention time point of a peak pattern configuring peak 47 are assumed to be dn2 and cn2.

In the peak pattern 91 of the assignment candidate peak 67, peak data and a retention time point of the assignment candidate peak 67 are assumed to be p2 and r2, peak data and a retention time point of a peak pattern configuring peak 65 are assumed to be fn1 and en1, and peak data and a retention time point of a peak pattern configuring peak 69 are assumed to be fn2 and en2.

When the degree of matching between peak patterns is P_Sim, the degree of matching between peak patterns (P_Sim(45-67)), each configured by three peaks, of the assignment target peak 45 and the assignment candidate peak 67 is calculated as:

$$P\_Sim(45\text{-}67) = (|p1 - p2| + 1) \times (|(r1 - (r2 + d)| + 1)$$

$$+ (|dn1 - fn1| + 1) \times (|(cn1 - r1) - (en1 - r2)| + 1)$$

$$+ (|dn2 - fn2| + 1) \times (|(cn2 - r1) - (en2 - r2)| + 1).$$

Here, d represented in the equation is a value used for correcting the deviation of the retention time point.

The calculating method of the degree of matching between peak patterns used for comparing the peak patterns each configured by five peaks will be described with reference to Fig. 64. In this case, the peak pattern 97 of the assignment target peak 45 and the peak pattern 101 of the assignment candidate peak 67 will be described as an example.

In the peak pattern 97 of the assignment target peak 45, peak data and a retention time point of the assignment target peak 45 are assumed to be p1 and r1, and peak data and retention time points of peak pattern configuring peaks 41, 43, 47, and 49 are assumed to be dn1 and cn1, dn2 and cn2, dn3 and cn3, and dn4 and cn4.

In the peak pattern 101 of the assignment candidate peak 67, peak data and a retention time point of the assignment candidate peak 67 are assumed to be p2 and r2, and peak data and retention time points of peak pattern configuring peaks 63, 65, 69, and 71 are assumed to be fn1 and en1, fn2 and en2, fn3 and en3, and fn4 and en4.

The degree of matching between peak patterns ($P\_Sim(45\text{-}67)$) each configured by five peaks, of the assignment target peak 45 and the assignment candidate peak 67 is calculated as:

$$P\_Sim(45\text{-}67) = (|p1 - p2| + 1) \times (|(r1 - (r2 + d)| + 1)$$

$$+ (|dn1 - fn1| + 1) \times (|(cn1 - r1) - (en1 - r2)| + 1)$$

$$+ (|dn2 - fn2| + 1) \times (|(cn2 - r1) - (en2 - r2)| + 1)$$

$$+ (|dn3 - fn3| + 1) \times (|(cn3 - r1) - (en3 - r2)| + 1)$$

$$+ (|dn4 - fn4| + 1) \times (|(cn4 - r1) - (en4 - r2)| + 1).$$

Here, d represented in the equation is a value used for correcting the deviation of the retention time point.

The peak assigning part 9 calculates the degree of matching between the UV spectra of the assignment target peak and the assignment candidate peak as illustrated in Figs. 65 and 66.

Fig. 65 is the diagram illustrating UV spectra (107 and 111) of the assignment target peak 45 and the assignment candidate peak 67, and, as illustrated in Fig. 66, the degree of matching between these two UV spectra ($UV\_Sim(45\text{-}67)$) is calculated as:

$$UV\_Sim(45\text{-}67) = RMSD(107 \text{ vs } 111).$$

The RMSD is defined as a mean square deviation and is defined as the square root of arithmetic average of a value that is a square of a distance between two corresponding points (dis). In other words, RMSD is calculated as $\sqrt{\Sigma dis^2/n}$.

"n" is the number of "dis."

Here, the waveform of the UV spectrum has a maximum wavelength and a minimum wavelength, and the degree of matching also can be calculated by comparing either the maximum wavelengths or the minimum wavelengths. However, compounds having no absorbance property or compounds having similar absorbance properties, they may quite differs from each other in the waveforms as a whole while having the same maximum and minimum wavelengths. Accordingly, there is a risk that the degree of matching between the waveforms may not be calculated by comparing either the maximum wavelengths or the minimum wavelengths.

In contrast to this, in a case where the RMSD is used in accordance with the waveforms of the UV spectra, the whole waveforms are compared with each other. Therefore, the degree of matching between the waveforms of the UV spectra can be calculated with accuracy, whereby even compounds having no absorbance property or compounds having similar absorbance properties can be identified with accuracy.

The degree of matching between the UV spectra is calculated similarly for all the assignment candidate peaks of the assignment target peak 45.

In addition, the peak assigning part 9 calculates the degree of matching of the assignment candidate peak that is acquired by integrating the above-described two degrees of matching as illustrated in Fig. 67.

As illustrated in Fig. 67, the degree (SCORE(45-67)) of matching of the assignment candidate peak is calculated by multiplying the degree of matching between the peak patterns by the degree of matching between the UV spectra. It is assumed that a score representing the degree of matching between peak patterns 45 and 67 is P_Sim_min(45-67), and a score representing the degree of matching between the corresponding UV spectra 107 and 111 is UV_Sim(45-67). At this time, the degree SCORE(45-67) of matching of the assignment candidate peaks is calculated as:

$$SCORE(45\text{-}67) = P\_Sim\_min(45\text{-}67) \times UV\_Sim(45\text{-}67).$$

The degree of matching of assignment candidate peak is similarly calculated for all the assignment candidate peaks for the assignment target peak 45.

Then, the SCOREs of all the assignment candidate peaks are compared to determine an assignment candidate peak having a lowest SCORE as an assignment peak of the assignment target peak 45.

Since the peak assigning part 9 determines the peaks to which the assignment target peaks should be assigned by integrating two viewpoints, it can realize peak assignment with accuracy.

In addition, the peak assigning part 9 assigns each peak of the target FP 17 to the reference group FP 19 based on the result of the assignment of the target FP to the reference FP as illustrated in Fig. 68.

Each peak of the target FP 17 is assigned to the reference FP configuring the reference group FP through the above-described assignment process. Base on the result of the assignment, finally, the peaks are assigned to the reference group FP 19.

In addition, the reference group FP 19 is prepared by performing an assignment process like the above for the plurality of reference FPs determined as normal products, and each peak is represented by an average value (black point) of assigned peaks ± standard deviation (vertical line).

Fig. 69 shows the result of assigning the target FP 17 to the reference group FP 19, and this result is the final result of the process of assigning the target FP 17.

[MD Value]

From this result, the MD value (MD values: 0.25, 2.99, and the like) can be acquired by MT method (see Figs. 70 to 74) as described above.

[Evaluating Method for Multicomponent Drugs]

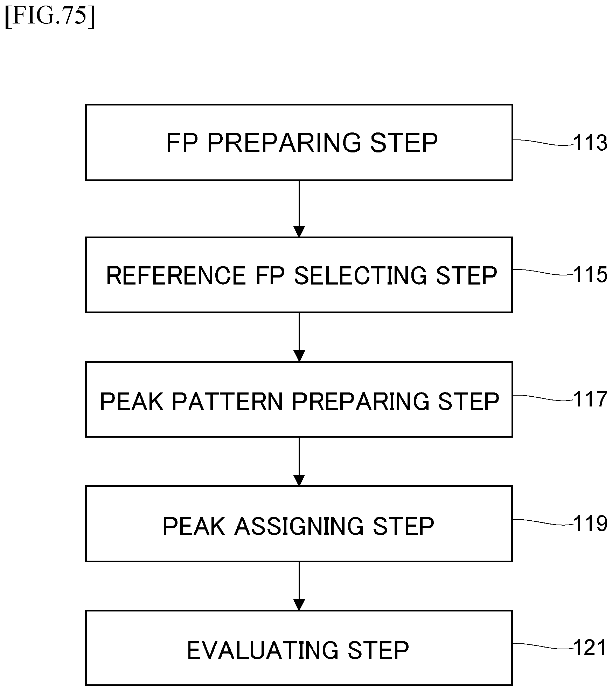

Fig. 75 is a process chart illustrating an evaluating method of a multicomponent drug according to Embodiment 1 of the present invention.

As illustrated in Fig. 75, the evaluating method of a multicomponent drug as an evaluating method for a pattern includes: a FP preparing process 113 as a pattern acquiring process; a reference FP selecting process 115 as a reference pattern selecting process; a peak pattern preparing step 117; a peak assigning step 119; and an evaluating step 121. The FP preparing process 113, the reference FP selecting process 115, the peak pattern preparing step 117, the peak assigning step 119, and the evaluating step 121 are performed by using the above-described evaluating apparatus 1 for a multicomponent drug in this embodiment, the FP preparing process 113 can be performed by using the function of the FP preparing part 3, and, similarly, the reference FP selecting process 115, the peak pattern preparing step 117, the peak assigning step 119, the evaluating step 121 can be performed by using the functions of the reference FP selecting part 5, the peak pattern specifying unit 7, the peak assigning part 9, and the evaluating part 11.

[Evaluating program for a multicomponent drug]

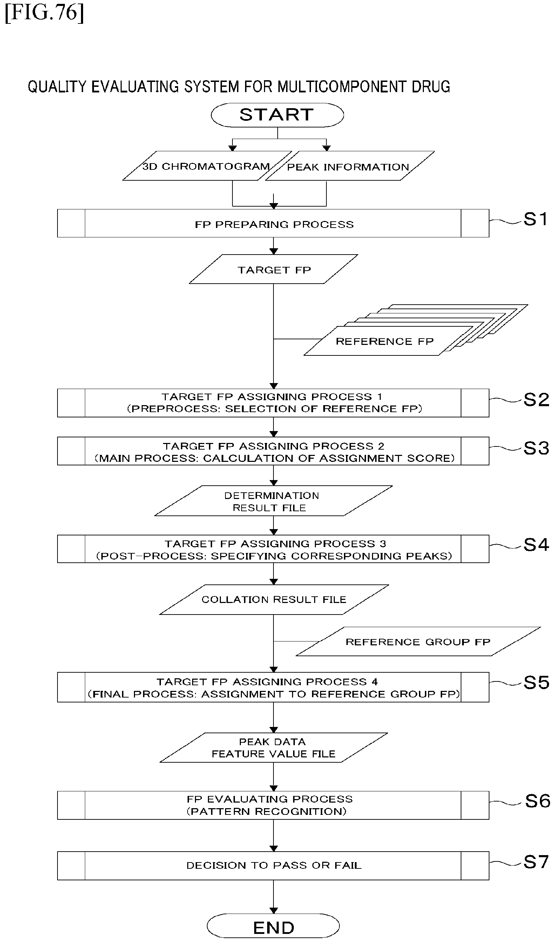

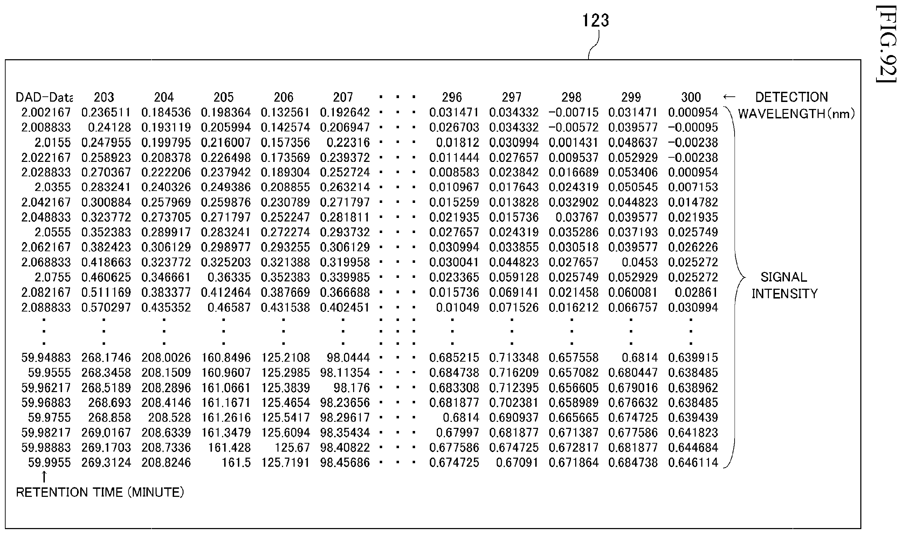

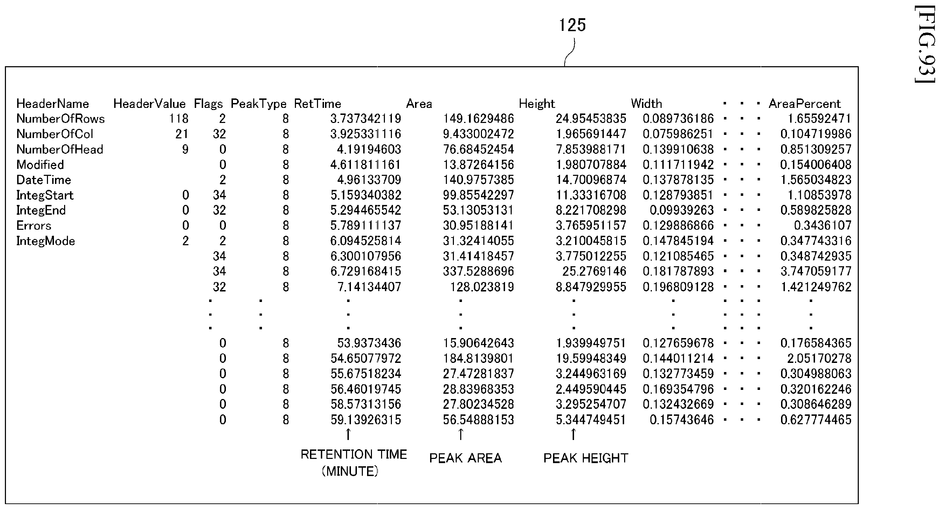

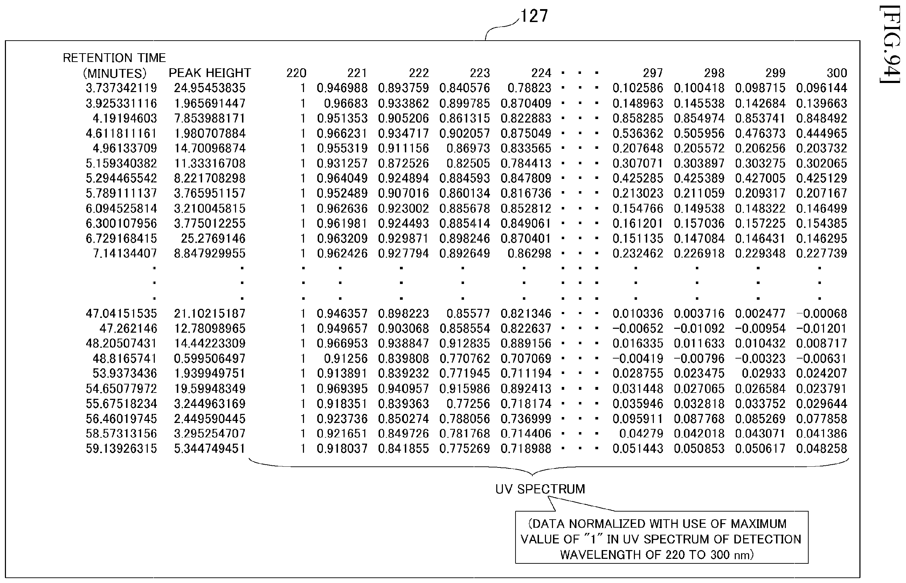

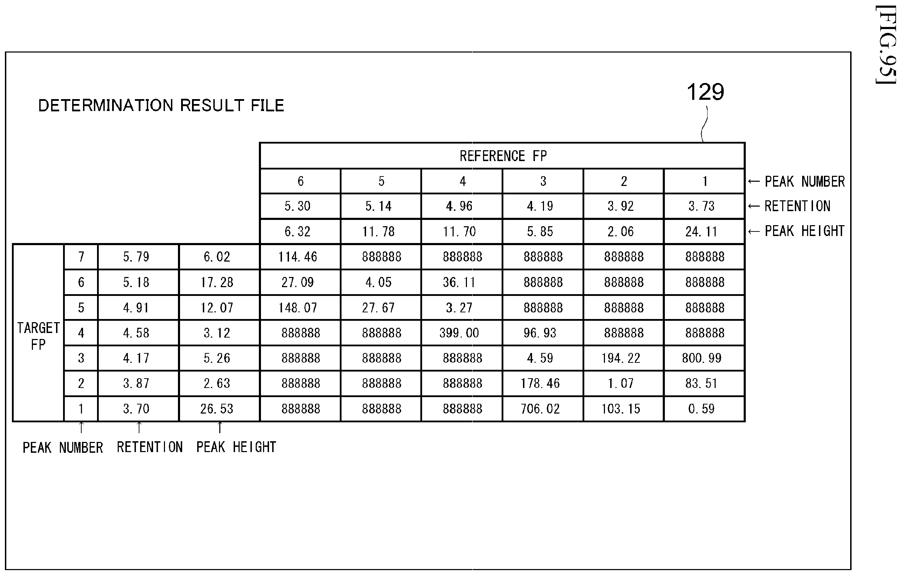

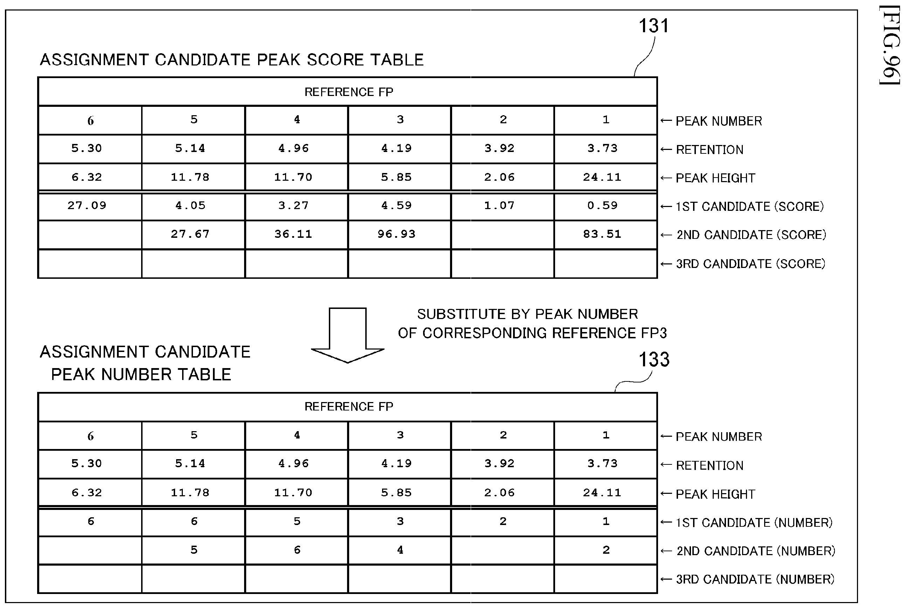

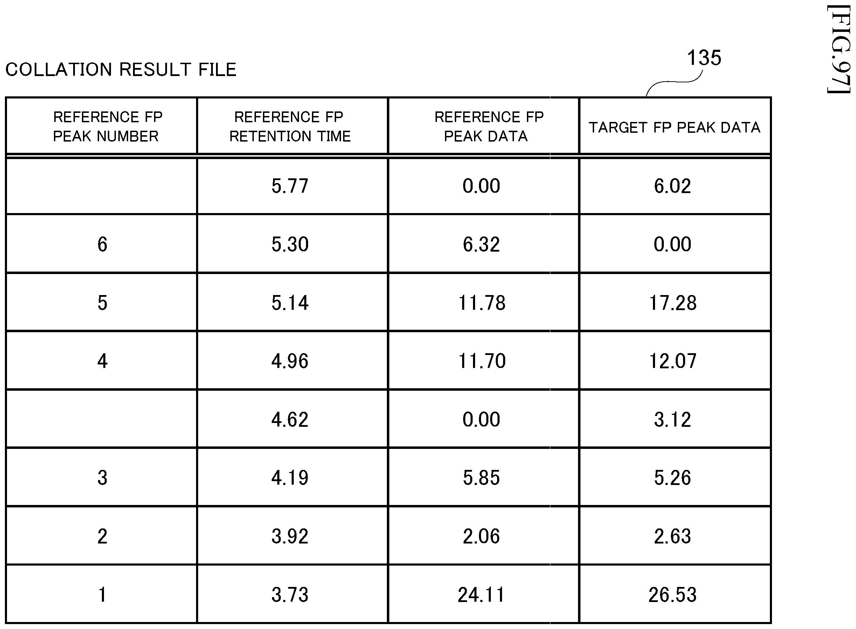

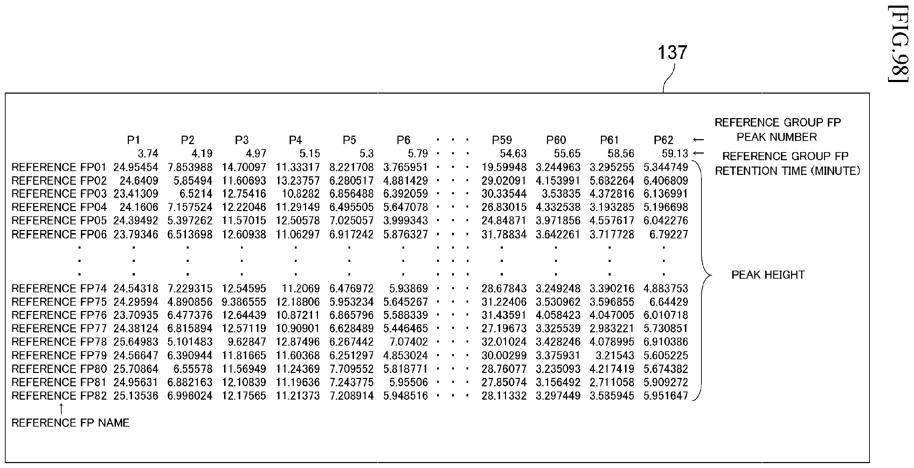

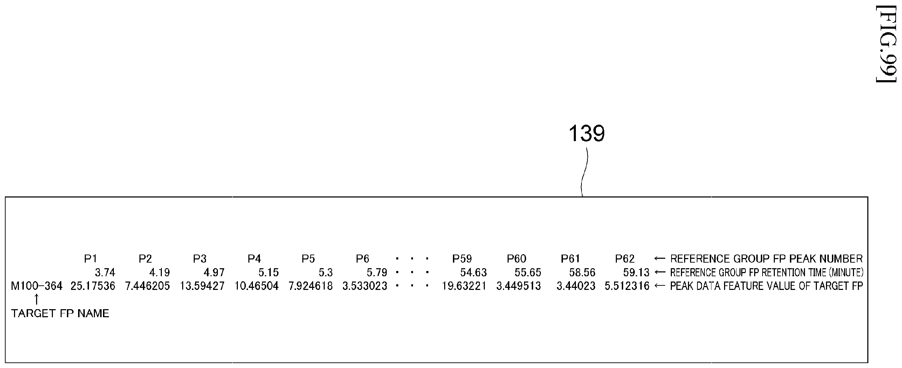

Figs. 76 to 91 are flowcharts according to the evaluating program for a multicomponent drug, Fig. 92 is a table representing a data example of 3D chromatogram, Fig. 93 is a table illustrating a peak information data example, Fig. 94 is a table illustrating a FP data example, Fig. 95 is a table illustrating a determination result file example prepared in Step S3, Fig. 96 is a table illustrating a two intermediate file example (an assignment candidate peak score table and an assignment candidate peak number table) that are prepared in the process of specifying corresponding peaks between the target FP and the reference FP, Fig. 97 is a table illustrating a collation result file example that is a result of specifying corresponding peaks between the target FP and the reference FP, Fig. 98 is a table illustrating a reference group FP data example, and Fig. 99 is a table illustrating a peak feature value file example of the target FP that is data of the target FP assigning peak.

Fig. 76 is a flowchart illustrating steps of the whole process performed for evaluating an evaluation target drug. It is started in accordance with system activation to realize the FP preparing function of the FP preparing part 3, the reference FP selecting function of the reference FP selecting part 5, the peak pattern preparing function of the peak pattern preparing part 7, the peak assigning function of the peak assigning part 9, and the evaluating function of the evaluating part 11 in the computer.

The FP preparing function is realized in Step S1, the reference FP selecting function is realized in Step S2, the peak pattern preparing function is realized in Step S3, the peak assigning function is realized in Steps S3 to S5, and the evaluating function is realized in Steps S6 and S7.

In Step S1, the "FP preparing process" is performed with a 3D chromatogram and peak information at a specific detection wavelength as input data.

The 3D chromatogram is data that is acquired by analyzing an evaluation target drug through HPLC and it is configured as three-dimensional information including a retention time points, detection wavelengths, and peaks (signal strength) as represented as a data example 123 of the 3D chromatogram in Fig. 92. The peak information is data that is acquired by processing chromatogram data at a specific wavelength, which is acquired through the same HPLC analysis, with a HPLC data analyzing tool (for example, "ChemStation" or the like). As represented as the peak information data example 125 in Fig. 93, the peak information is data configured by the maximum values and area values of all peaks detected as peaks and retention time points at those time point.

In Step S1, the FP preparing part 3 (Fig. 1) of the computer functions to prepare the target FP 17 (Fig. 2) based on the 3D chromatogram and the peak information and output the data as a file. The target FP 17, like the FP data example 127 in Fig. 94, is data configured by retention time points, peak heights, and UV spectra for respective peak heights.

In Step S2, the "target FP assigning process 1" is performed with input of the target FP and all the reference FPs output in Step S1.

In Step S2, the reference FP selecting part 5 of the computer functions to calculate the degree of matching between retention time appearance patterns of all the reference FPs with respect to the target FP 17, to select a reference FP that is appropriate to the assignment of the target FP 17.

The reference FPs are FPs prepared by the same process as that of Step S1 based on the 3D chromatogram and peak information of drugs determined as normal products. In addition, the normal product is defined as a drug of which the safety and the effectiveness are checked and a plurality of drugs with different product lots correspond thereto. The reference FP is data configured similarly to the FP data example 127 in Fig. 94.

In Step S3, the "target FP assigning process 2" is performed according to the target FP 17 and the reference FP selected in Step S2 as input.

In Step S3, the peak pattern preparing part 7 (Fig. 1) and the peak assigning part 9 (Fig. 1) of the computer functions. Through the functions thereof, peak patterns are comprehensively prepared for all the peaks of the target FP 17 and the reference FP selected in Step S2 as illustrated in Figs. 23 to 61, to calculate the degree of matching between the peak patterns (P_Sim illustrated in Fig. 63 or 64). In addition, the degree of matching between the UV spectra (UV_Sim illustrated in Fig. 66) of the target FP and the reference FP is calculated. Furthermore, the degree of matching of the assignment candidate peak (SCORE illustrated in Fig. 67) is calculated based on these two kinds of the degrees of matching, and the calculation result is output in the form of a file (determination result file).

In Step S4, the "target FP assigning process 3" is performed according to the determination result file output in Step S3 as an input.

In Step S4, the peak assigning part 7 of the computer functions to, between the target FP 17 and the reference FP, specify peaks of the reference FP that correspond to the respective peaks of the target FP based on the degree (SCORE) of matching of the assignment candidate peaks and outputs the result in the form of a file (collation result file).

In Step S5, the "target FP assigning process 4" is performed according to the collation result file output in Step S4 and the reference group FP as inputs.

The reference group FP is peak correspondence data over all the reference FPs prepared from the all reference FPs in the same process as that of Steps S2 to S4.

In Step S5, the peak assigning part 7 of the computer functions to assign the peaks of the target FP 17 to the respective peaks of the reference group FP based on the collation result file of the target FP 17 as illustrated in Figs. 68 and 69, and outputs the result to in the form of a file (peak data feature value file).

In Step S6, the "FP evaluating process" is performed according to the peak data feature value file output in Step S5 and the reference group FP as inputs.

In Step S6, the evaluating part 11 of the computer functions to evaluate the equivalency between the peak data feature value data output in Step S5 and the reference group FP by MT method, and outputs the evaluation result as an MD value (Figs. 70 to 74).

In Step S7, the "determination of a success or not" is performed according to the MD value output in Step S6 as input.

In Step S7, the evaluating part 11 of the computer functions to compare the MD value output in Step S6 with a threshold value (the upper limit of the MD value) set in advance so as to make a decision to pass or fail (Graph 23 illustrated in Fig. 2).

[S1: FP Preparing Process (Using Only Single Wavelength)]

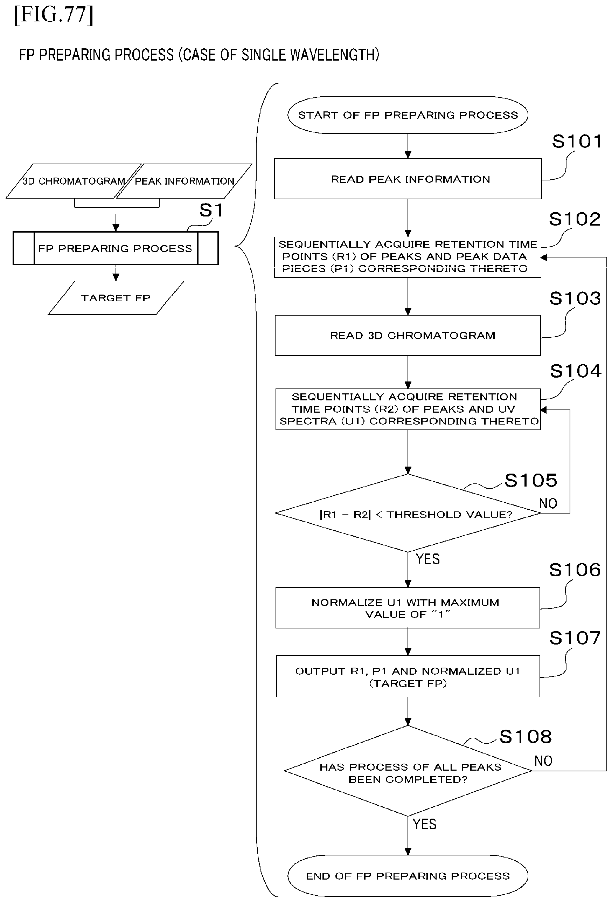

Fig. 77 is a flowchart in a case where single-wavelength peak information of the "FP preparing process" in Step S1 illustrated in Fig. 76 is used.

Fig. 77 shows details of the step of preparing the evaluation target FP for a single wavelength, for example, 203 nm. In this process, based on the 3D chromatogram and the peak information at the detection wavelength being 203 nm, a FP is prepared to comprise a retention time point, a peak and a UV spectrum of each peak detected at the detection wavelength of 203 nm.

In Step S101, a process of "reading peak information" is performed. In this process, peak information is read out as the first one of two kinds of data that are necessary for preparing a FP, and it proceeds to Step S102.

In Step S102, a process of "sequentially acquiring a retention time point (R1) of a peak and peak data (P1) corresponding thereto" is performed. In this process, retention time points (R1) and peak data (P1) of the peaks are sequentially acquired from the peak information one by one, and it proceeds to Step S103.

In Step S103, a process of "reading a 3D chromatogram" is performed. In this process, a 3D chromatogram is read as the second one of the two kinds of data necessary for preparing the FP, and it proceeds to Step S104.

In Step S104, a process of "sequentially acquiring a retention time point (R2) of a peak and a UV spectrum (U1) corresponding thereto" is performed. In this process, retention time points (R2) and UV spectra (U1) are acquired from the 3D chromatogram at each period that is a half of a sampling rate at the time of analyzing the HPLC, and it proceeds to Step S105.

In Step S105, a determining process "$|R1 - R2| \leq$ Threshold Value?" is performed. In this process, it is determined whether or not the retention time points R1 and R2 read in Steps S102 and S104 correspond to each other within a threshold value range. If corresponding (YES), it is determined that two retention time points are the same and the UV spectrum of the peak at the retention time point R1 is U1. Then, it proceeds to Step S106. If not corresponding (NO), it is determined that the two retention time points are not the same and the UV spectrum of the peak at the retention time point of R1 is not the UV spectrum U1. Then, it proceeds to Step S104 so as to perform comparison with the next data of the 3D chromatogram. The threshold value used in this determination process is the "sampling rate" of the 3D chromatogram.

In Step S106, a process of "normalizing the UV spectrum U1 with the maximum value of "1"" is performed. In this process, the UV spectrum U1 determined as the UV spectrum of the retention time point R1 in Step S105 is normalized with the maximum value of "1," and it proceeds to Step S107.

In Step S107, a process of "outputting R1 and P1 as well as the normalized U1 (target FP)" is performed. In this process, the R1 and P1 acquired from the peak information and the U1 normalized in S106 are output to the target FP, and it proceeds to Step S108.

In Step S108, a determining process "Has the process for all the peaks been completed?" is performed. In this process, it is determined whether or not all the peaks included in the peak information have been processed. If the process has not been completed for all the peaks (NO), it proceeds to Step S102 in order to process one or more peaks that have not been processed. The process of Steps S102 to S108 is repeated until the process of all the peaks is completed. If the process of all the peaks has been completed (YES), the FP preparing process is finished.

[S1: FP Preparing Process (Using A Plurality of Wavelengths)]

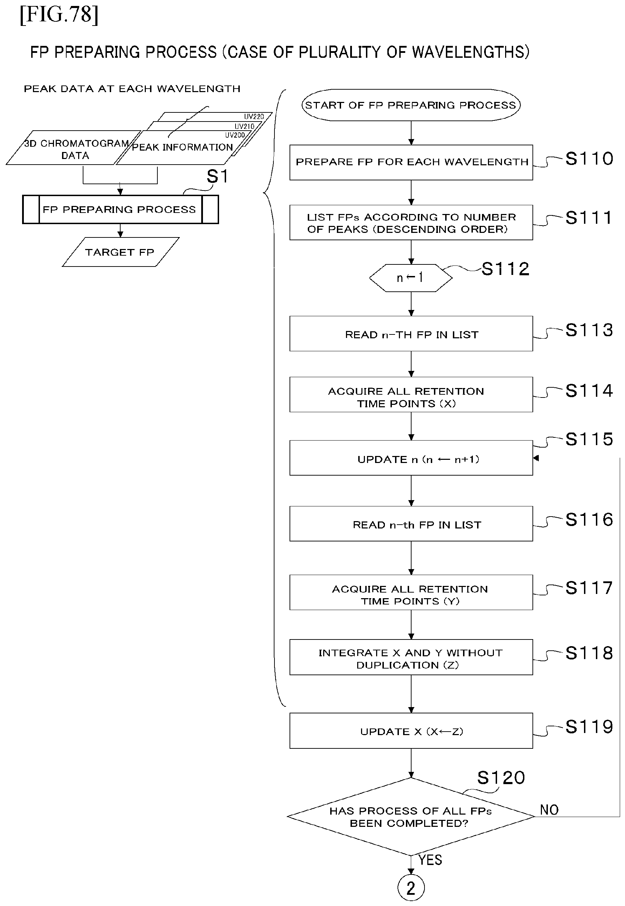

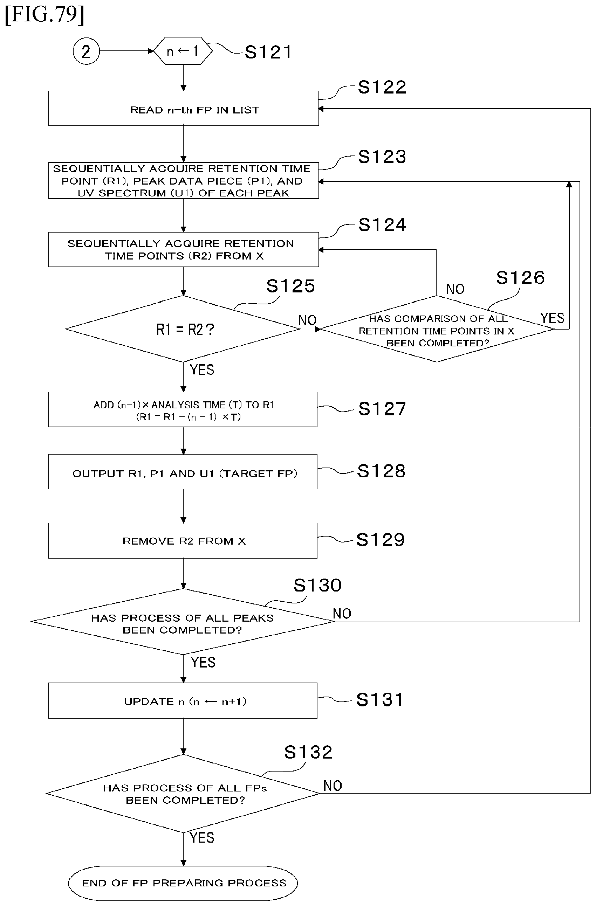

Figs. 78 and 79 are flowcharts of a case where peak information at a plurality of wavelengths are used instead of the peak information at the single wavelength in the "FP preparing process" of Step S1 illustrated in Fig. 76. For example, this is a case where a plurality of (n) wavelengths are selected in the direction of the detection wavelength axis including 203 nm to prepare a FP.

This FP preparing process is for preparing a FP that covers all the peaks of the 3D chromatogram with use of peak information of a plurality of wavelengths in a case where all the peaks detected in the 3D chromatogram cannot be covered at the single wavelength as illustrated in Fig. 77.

In addition, Figs. 78 and 79 illustrate details of the step in which n FPs are prepared at respective wavelengths by performing the above-described FP preparing process by means of only a single wavelength, and, based on the FPs, a FP according to the plurality of wavelengths is prepared.

In Step S110, a process of "preparing a FP for each wavelength" is performed. In this process, the above-described FP preparing process using only the single wavelength is performed for each wavelength so as to prepare n FPs, and it proceeds to Step 5111.

In Step S111, a process of "listing the FPs according to the number of peaks (descending order)" is performed. In this process, the n FPs are listed in the descending order of the number of peaks, and it proceeds to Step S112.

In Step S112, as initialization of a counter for sequentially processing n FPs, one is substituted into n (n <- 1), and it proceeds to Step S113.

In Step S113, a process of "reading the n-th FP in the list" is performed. In this process, the n-th FP in the list is read, and it proceeds to Step S114.

In Step S114, a process of "acquiring all the retention time points (X)" is performed. In this process, all the retention time point information of the FPs read in S113 is acquired, and it proceeds to Step S115.

In Step S115, a process of "updating n (n <- n+1)" is performed. In this process, "n+1" is substituted into "n" as the update of "n" in order to transfer the process to the next FP, and it proceeds to Step S116.

In Step S116, a process of "reading the n-th FP in the list" is performed. In this process, the n-th FP in the list is read, and it proceeds to Step S117.

In Step S117, a process of "acquiring all the retention time points (Y)" is performed. In this process, the retention time point information of all the FPs read in S116 is acquired, and it proceeds to Step S118.

In Step S118, a process of "integrating X and Y without duplication (Z)" is performed. In this process, the retention time point information X acquired in S114 and retention time point information Y acquired in Step S117 are integrated without duplication, thereafter, the integrated information is stored in Z, and it proceeds to Step S119.

In Step S119, a process of "updating X (X <- Z)" is performed. In this process, as the update of X, Z stored in Step S118 is substituted for X, and it proceeds to Step S120.

In Step S120, a determining process "Have all the FPs been processed?" is performed. In this process, it is determined whether or not all the n FPs prepared in Step S110 have been processed. If processed (YES), it proceeds to Step S121. If there are one or more FPs that have not been processed (NO), it proceeds to Step S115 in order to perform the process of Steps S115 to S120 for the FPs that have not been processed. Until the process of all the FPs are completed, the process of Steps S115 to S120 is repeated.

In Step S121, as the initialization of the counter for sequentially processing n FPs, "1" is substituted into "n" (n <- 1), and it proceeds to Step S122.

In Step S122, a process of "reading the n-th FP in the list" is performed. In this process, the n-th FP in the list is read, and it proceeds to Step S123.

In Step S123, a process of "sequentially acquiring a retention time point (R1), peak data (P1), and a UV spectrum (U1) of each peak" is performed. In this process, retention time points (R1), peak data pieces (P1), and UV spectra (U1) of peaks are sequentially acquired from the FP read in Step S122 one by one, and it proceeds to Step S124.

In Step S124, a process of "sequentially acquiring retention time points (R2) from X" is performed. In this process, retention time points (R2) are sequentially acquired from X in which the retention time points of all the FPs are stored without duplication one by one, and it proceeds to Step S125.

In Step S125, a determining process "R1 = R2?" is performed. In this process, it is determined whether or not R1 acquired in Step S123 and R2 acquired in Step S124 are the same. If being the same (YES), it proceeds to Step S127. If not being the same (NO), it proceeds to Step S126.

In Step S126, a determining process "Has the comparison of all the retention time points of X been completed?" is performed. In this process, it is determined whether or not the comparison of R1 acquired in S123 with all the retention time points of X has been completed. If completed (YES), it is determined that the peak at the retention time point of R1 has been processed and it proceeds to Step S123 in order to transfer the process to the next peak. If not completed (NO), it proceeds to Step S124 in order to transfer the process to the next retention time point of X.

In Step S127, a process of "adding (n - 1) $\times$ analysis time (T) to R1 (R1 <- R1 + (n - 1) $\times$ T)" is performed. In this process, for each retention time point that is present in the first FP, which has the highest number of peaks, in the list, the retention time point is unchanged. For the retention time of a peak that is not present in the 1st FP in the list but is present in the 2nd FP, an analysis time (T) is added to R1. For the retention time of a peak that is not present in the 1st to (n - 1)-th FP in the list but is present in the n-th FP, (n - 1) $\times$ T is added to R1. Then, it proceeds to Step S128.

In Step S128, a process of "outputting R1, P1, and U1 (target FP)" is performed. In this process, R1 processed in Step S127, P1 and U1 acquired in Step S123 are output to the target FP, and it proceeds to Step S129.

In Step S129, a process of "removing R2 from X" is performed. In this process, since the process at the retention time points R1 (= R2) have been completed in Steps S127 and S128, the retention time points (R2) that have been processed are removed from X, and it proceeds to S130.

In Step S130, a determining process "Have all peak processes been completed?" is performed. In this process, it is determined whether or not the process has been completed for all the peaks of the n-th FP in the list. If completed (YES), the FP preparing process for the n-th FP in the list is finished to proceed to Step S131. If not completed (NO), it proceeds to Step S123 in order to process any peak that has not been completed. Until the process of all the peaks is finished, the process of Steps S123 to S130 is repeated.

In Step S131, a process of "updating n (n <- n+1)" is performed. In this process, in order to transfer the process to the next FP, "n+1" is substituted into "n" as the update of "n" to proceed to Step S132.

In Step S132, a determining process "Have all FP processes been completed?" is performed. In this process, it is determined whether or not all the n FPs prepared in Step S110 have been processed. If processed (YES), the FP preparing process is finished. If there are one or more FPs that have not been processed (NO), it proceeds to Step S122 in order to perform the process of Steps S122 to S132 for the FPs that have not been processed. Until the process of all the FPs is completed, the process of Steps S122 to S132 is repeated.

[S2: Target FP Assigning Process 1]

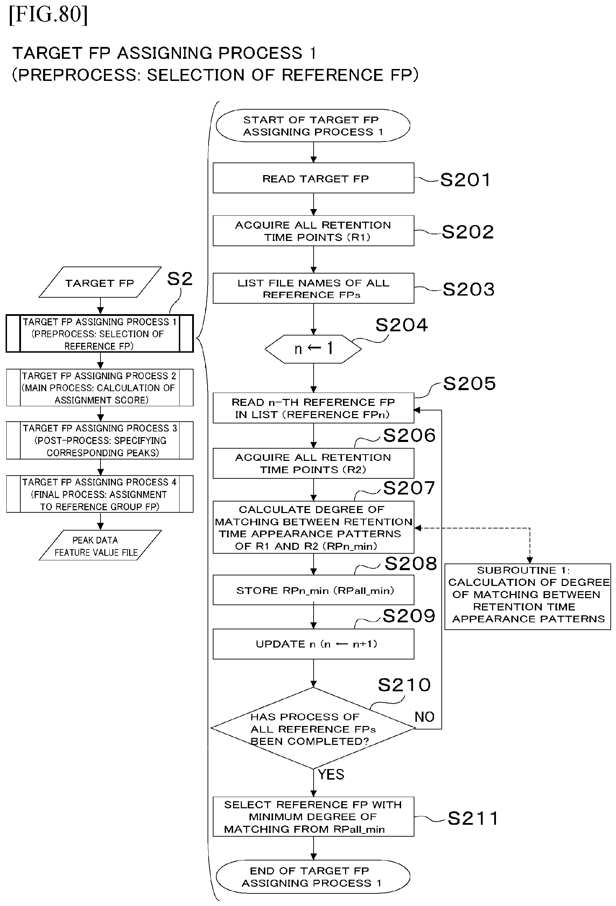

Fig. 80 is a flowchart illustrating details of the "target FP assigning process 1" of Step S2 in Fig. 76. This process is a preprocess of the assigning process and selects a reference FP that is appropriate to the assignment of the target FP 17 from among a plurality of reference FPs regarded as normal products.

In Step S201, a process of "reading a target FP" is performed. In this process, the FP that is an assignment target is read, and it proceeds to Step S202.

In Step S202, a process of "acquiring all the retention time points (R1)" is performed. In this process, all the retention time point information of the target FP that is read in S201 is acquired, and it proceeds to Step S203.

In Step S203, a process of "listing file names of all the reference FPs" is performed. In this process, file names of all the reference FPs are listed in advance in order to sequentially process all the reference FPs later, and it proceeds to Step S204.

In Step S204, "1" is substituted into "n" (n <- 1) as an initial value of the counter used for sequentially processing all the reference FPs, and it proceeds to Step S205.

In Step S205, a process of "reading the n-th reference FP (reference $FP_n$) in the list" is performed. In this process, the n-th FP of the file name list of all the reference FPs listed in Step S203 is read, and it proceeds to Step S206.

In Step S206, a process of "acquiring all the retention time points (R2)" is performed. In this process, all of the retention time point information of the reference FP that are read in S205 are acquired, and it proceeds to Step S207.

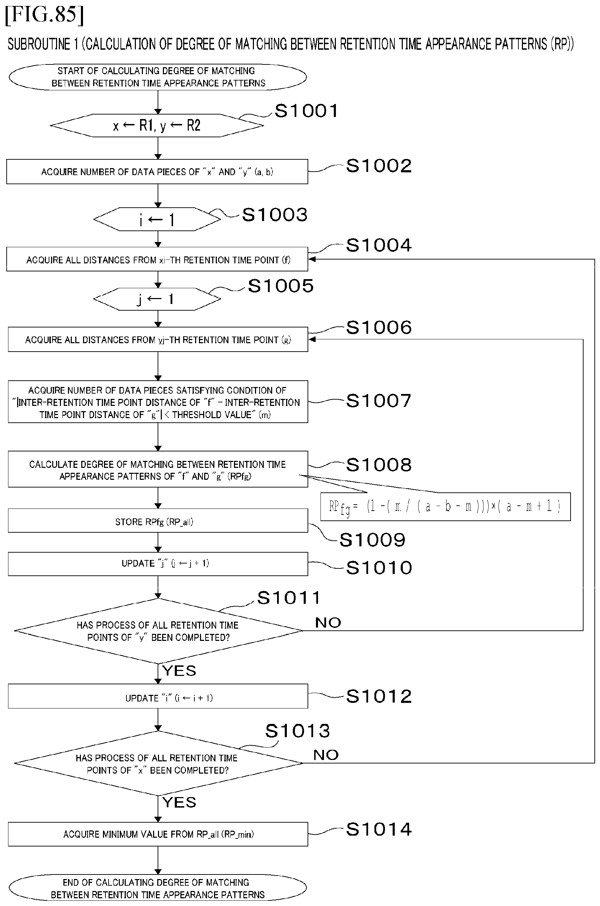

In Step S207, a process of "calculating the degree of matching between retention time appearance patterns of R1 and R2 ($RP_n\_min$)" is performed. In this process, $RP_n\_min$ is calculated based on the retention time point of the target FP that is acquired in Step S202 and the retention time point of the reference FP that is acquired in Step S206, and it proceeds to Step S208. A detailed calculation flow of $RP_n\_min$ will be described with reference to "Subroutine 1" of Fig. 85 separately.

In Step S208, a process of "storing $RP_n\_min$ ($RP_{all\_}min$)" is performed. In this process, $RP_n\_min$ calculated in Step S207 is stored in $RP_{all\_}min$, and it proceeds to Step S209.

In Step S209, a process of "updating n (n <- n+1)" is performed. In this process, in order to transfer the process to the next FP, "n+1" is substituted for n as the update of n, and it proceeds to Step S210.

In Step S210, a determining process "Have all reference FP processes been completed?" is performed. In this process, it is determined whether or not all the reference FPs have been processed. If processed (YES), it proceeds to Step S211. If there are one or more reference FPs that have not been processed (NO), it proceeds to Step S205 in order to perform the process of Steps S205 to S210 for the FPs that have not been processed. Until the process of all the reference FPs are completed, the process of Steps S205 to S210 is repeated.

In Step S211, a process of "selecting a reference FP demonstrating the minimum degree of matching from $RP_{all\_}min$" is performed. In this process, $RP_n\_min$ to RPn_min calculated for all the reference FPs are compared with each other to select a reference FP demonstrating the minimum degree of matching with respect to the retention time appearance pattern of the target FP, and the target FP assigning process 1 is finished.

[S3: Target FP Assigning Process 2]

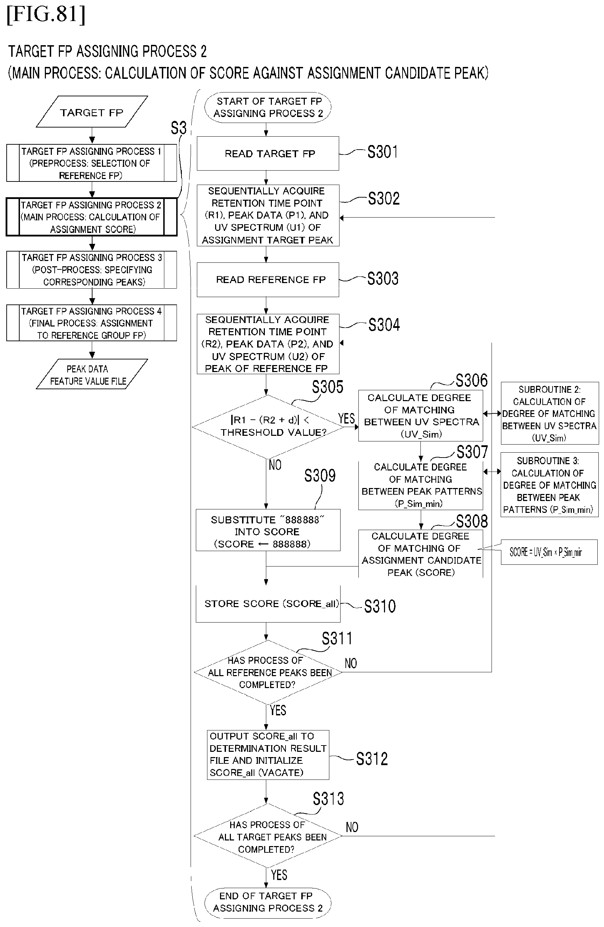

Fig. 81 is a flowchart illustrating details of the "target FP assigning process 2" of Step S3 in Fig. 76. This process is a main process of the assigning process and calculates the degree (SCORE) of matching for each assignment candidate peak based on the degrees of matching between the peak patterns and the UV spectra of the target FP 17 and the reference FP selected in Step S2.

In Step S301, a process of "reading a target FP" is performed. In this process, the FP that is an assignment target is read, and it proceeds to Step S302.

In Step S302, a process of "sequentially acquiring a retention time point (R1), peak data (P1), and a UV spectrum (U1) of an assignment target peak" is performed. In this process, the peaks of the target FP read in Step S301 are sequentially set as the assignment target peak to acquire R1, P1, and U1, and it proceeds to Step S303.

In Step S303, a process of "reading the reference FP" is performed. In this process, the reference FP that is selected in the "Target FP Assigning Process 1" in Fig. 80 is read, and it proceeds to Step S304.

In Step S304, a process of "sequentially acquiring a retention time point (R2), peak data (P2), and a UV spectrum (U2) of a peak of the reference FP" is performed. In this process, R2, P2, and U2 are acquired from the reference FP read in Step S303 for each peak, and it proceeds to Step S305.

In Step S305, a determining process "|R1 - (R2 + d) | < Threshold Value?" is performed. In this process, it is determined whether or not R1 and R2 read in Steps S302 and S304 correspond to each other within the threshold value range. If corresponding (YES), it is determined that the peak of which the retention time point is R2 is an assignment candidate peak of the peak of which the retention time point is R1. Then, in order to calculate the degree of matching for the assignment candidate peak (SCORE), it proceeds to Step S306. If not corresponding (NO), since the peak of which the retention time point is R2 and the peak of which the retention time point is R1 have a great difference in the retention time, it is determined that the peak cannot be set as the assignment candidate peak, and it proceeds to Step S309. In addition, "d" used in this determination process is a value for correcting the retention time points of the peaks of the target FP and the reference FP, and the initial value is set to zero. A difference between retention time points of peaks is acquired whenever being assigned during the progress of the process to update "d" with the value. In addition, the threshold value is an allowable range of the retention time points used for determining whether to be set as an assignment candidate peak.

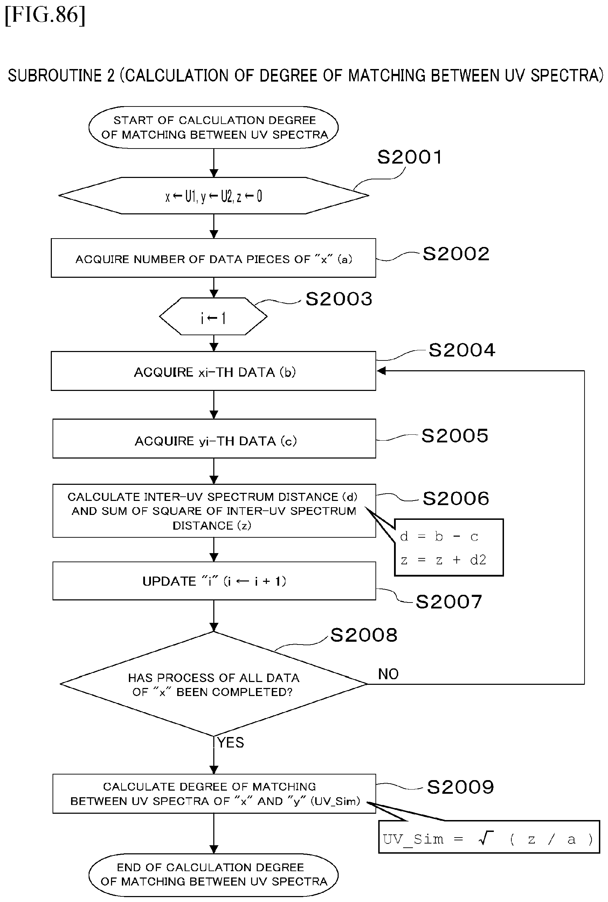

In Step S306, a process of "calculating the degree of matching between UV spectra (UV_Sim)" is performed. In this process, UV_Sim is calculated based on U1 of the assignment target peak acquired in Step S302 and U2 of the assignment candidate peak acquired in S304, and it proceeds to Step S307. In addition, a detailed calculation flow of UV_Sim will be described with reference to "Subroutine 2" in Fig. 86 separately.

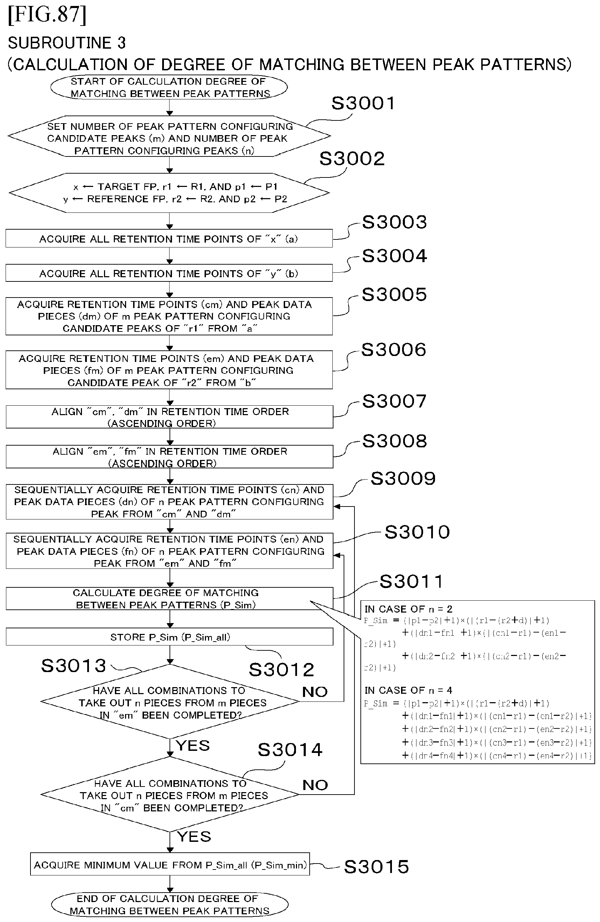

In Step S307, a process of "calculating the degree of matching between peak patterns (P_Sim_min)" is performed. In this process, from R1 and P1 of the assignment target peak acquired in Step S302 and R2 and P2 of the assignment candidate peak acquired in Step S304, peak patterns are comprehensively prepared for these peaks. In addition, P_Sim_min of these peak patterns is calculated, and it proceeds to Step S308. A detailed calculation flow of P_Sim_min will be described with reference to "Subroutine 3" in Fig. 87 separately.

In Step S308, a process of "calculating the degree of matching for the assignment candidate peak (SCORE)" is performed. In this process, from UV_Sim calculated in Step S306 and P_Sim_min calculated in Step S307, SCORE of the assignment target peak and the assignment candidate peak is calculated as:

$$\text{SCORE} = \text{UV\_Sim} \times \text{P\_Sim\_min}.$$

It proceeds to Step S310.

In Step S309, a process of "substituting "888888" into SCORE (SCORE <-888888)" is performed. In this process, SCORE of a peak of an assignment target peak that does not correspond to an assignment candidate peak is set to "888888", and it proceeds to Step S310.

In Step S310, a process of "storing SCORE (SCORE_all)" is performed. In this process, SCORE acquired in Step S308 or S309 is stored in the SCORE_all, and it proceeds to Step S311.

In Step S311, a determining process "Has the process of all reference peaks been completed?" is performed. In this process, it is determined whether or not all the peaks of the reference FP have been processed. If processed (YES), it proceeds to Step S312. If there are one or more peaks that have not been processed (NO), it proceeds to Step S304 in order to perform the process of S304 to S311 for the unprocessed peaks. Until the process of all the peaks is completed, the process of Steps S304 to S311 is repeated.

In Step S312, a process of "outputting the SCORE_all to a determination result file to initialize (vacate) the SCORE_all" is performed. In this process, the SCORE_all is output to the determination result file, and thereafter, the SCORE_all is initialized (vacated), and it proceeds to Step S313.

In Step S313, a determining process "Has the process of all target peaks been completed?" is performed. In this process, it is determined whether all the peaks of the target FP have been processed. If processed (YES), the target FP assigning process 2 is finished. If there are one or more peaks that have not been processed (NO), it proceeds to Step S302 in order to perform the process of Steps S302 to S313 for the unprocessed peaks. Until the process of all the peaks is completed, the process of S302 to S313 is repeated.

Fig. 95 illustrates an output determination result file example 129.

[S4: Target FP Assigning Process 3]

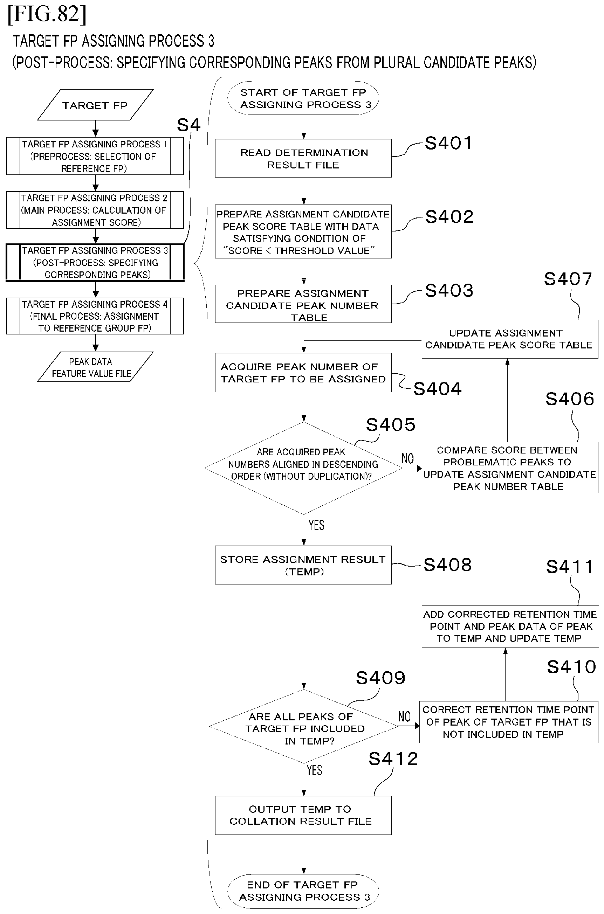

Fig. 82 is a flowchart illustrating the "target FP assigning process 3" of Step S4 in Fig. 76. This process is a post-process of the assignment and specifies the peak of the reference FP corresponding to each peak of the target FP based on the degree of matching of the assignment candidate peak (SCORE) calculated as described above.

In Step S401, a process of "reading the determination result file" is performed. In this process, the determination result file prepared by the "target FP assigning process 2" in Fig. 81 is read, and it proceeds to Step S402.

In Step S402, a process of "preparing an assignment candidate peak score table with data satisfying the condition of "SCORE < Threshold value"" is performed. In this process, an assignment candidate score table 131 is prepared in Fig. 96 (upper diagram) based on the SCORE of the determination result file, and it proceeds to Step S403. This assignment candidate peak score table is a table in which only SCOREs less than the threshold value in the SCORE calculated for the all peaks of the target FP are aligned in an ascending order for each peak of the reference FP. The smaller the value of the SCORE is, the higher the possibility for a peak to be assigned is. In addition, the threshold value is an upper limit value for the SCOREs to determine whether to set as an assignment candidate.

In Step S403, a process of "preparing an assignment candidate peak number table" is performed. In this process, an assignment candidate peak number table 133 illustrated in Fig. 96 (lower diagram) is prepared based on the assignment candidate peak score table, and it proceeds to Step S404. This assignment candidate peak number table is a table that is acquired by substituting each score included in the assignment candidate peak score table into a peak number of the target FP corresponding to the score. Accordingly, this table is a table that sequentially aligns the peak numbers of the target FP to be associated for each peak of the reference FP.

In Step S404, a process of "acquiring the peak numbers of the target FP to be assigned" is performed. In this process, a peak number of the target FP that is located at the highest position is acquired for each peak of the reference FP from the assignment candidate peak number table prepared in Step S403, and it proceeds to Step S405.

In Step S405, a determining process "Are the acquired peak numbers aligned in a descending order (without duplication)?" is performed. In this process, it is determined whether or not the peak numbers of the target FP acquired in Step S404 are aligned in the descending order without duplication. If aligned (YES), it is determined that the peaks of the target FP corresponding to respective peaks of the reference FP can be settled, and it proceeds to Step S408. If not aligned (NO), in order to reconsider one or more problematic peaks of the target FP to be assigned to peaks of the reference FP, it proceeds to Step S406.

In Step S406, a process of "comparing SCOREs of problematic peaks to update the assignment candidate peak number table" is performed. In this process, SCOREs corresponding to the peak numbers of the target FP that have the problem are compared with use of the assignment candidate score table, and the assignment candidate peak number table is updated in which a peak number having a larger SCORE is substituted into a peak number located in the second, and it proceeds to Step S407.

In Step S407, a process of "updating the assignment candidate peak store table" is performed. In this process, in accordance with the updated content of the assignment candidate peak number table in Step S406, the assignment candidate peak score table is updated, and it proceeds to Step S404. Until there is no problem in the peak numbers of the target FP (there is no duplication, or the peak numbers are aligned in the descending order), the process of Steps S404 to S407 is repeated.

In Step S408, a process of "storing an assignment result (TEMP)" is performed. In this process, the peak numbers of all the peaks, the retention time points and the peaks of the reference FP and peak data of the target FP that is specified as the peaks corresponding to these peak of the reference FP are stored in TEMP, and it proceeds to Step S409.

In Step S409, a determining process "Are all the peaks of the target FP included in TEMP?" is performed. In this process, it is determined whether the peak data of all the peaks of the target FP is included in TEMP stored in Step S408. If all included (YES), it is determined that the process for all the peaks of the target FP has been completed, and it proceeds to Step S412. If there is any excluded peak (NO), in order to add peak data of the excluded peak, it proceeds to Step S410. In Step S410, a process of "correcting the retention time point of the peak of the target FP that is not included in TEMP" is performed. In this process, the retention time point of the peak of the target FP (the peak of the target FP that is needed to be corrected) that is excluded from TEMP is corrected as a correction value = k1 + (k2 - k1) * (t0 - t1)/(t2 - t1), wherein:

k1: it is a retention time point of a peak having a shorter retention time point of two reference FP-side peaks that are assigned in the vicinity of a peak of a target FP for which correction is necessary;
k2: it is a retention time point of a peak having a larger retention time point of two reference FP-side peaks that are assigned in the vicinity of the peak of the target FP for which correction is necessary;
t0: it is a retention time point of the peak of the target FP for which correction is necessary;
t1: it is a retention time point of a peak having a shorter retention time point of two target FP-side peaks that are assigned in the vicinity of the peak of the target FP for which correction is necessary; and
t2: it is a retention time point of a peak having a longer retention time point of two target FP-side peaks that are assigned in the vicinity of the peak of the target FP for which correction is necessary, and it proceeds to Step S411.

In Step S411, a process of "adding the corrected retention time point and the peak data thereof to TEMP, and updating TEMP" is performed. In this process, the retention time point of the peak of the target FP corrected in S410 and not included in TEMP is compared with the retention time points of the reference FP in TEMP, to add the corrected retention time point and peak data of the peak of the target FP that is not included in TEMP to a valid position in TEMP and update TEMP, and it proceeds to Step S409. Until all the peaks of the target FP are added, the process of Steps S409 to S411 is repeated.

In Step S412, a process of "outputting TEMP to a collation result file" is performed. In this process, TEMP that specifies the correspondence relation between all the peaks of the reference FP and the all the peaks of the target FP is output as a collation result file, and the target FP assigning process 3 ends.

Fig. 97 illustrates a collation result file example 135 output as described above.

[S5: Target FP Assigning Process 4]

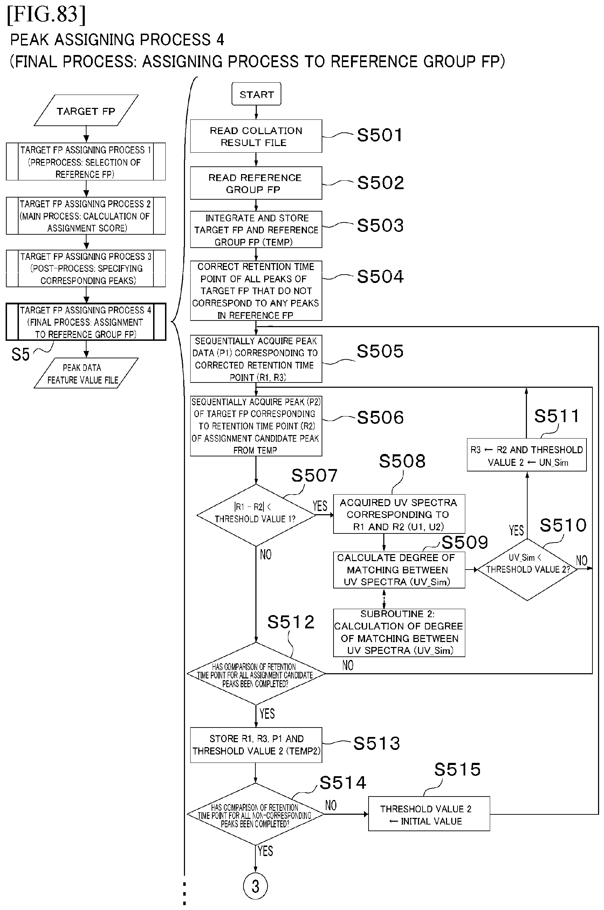

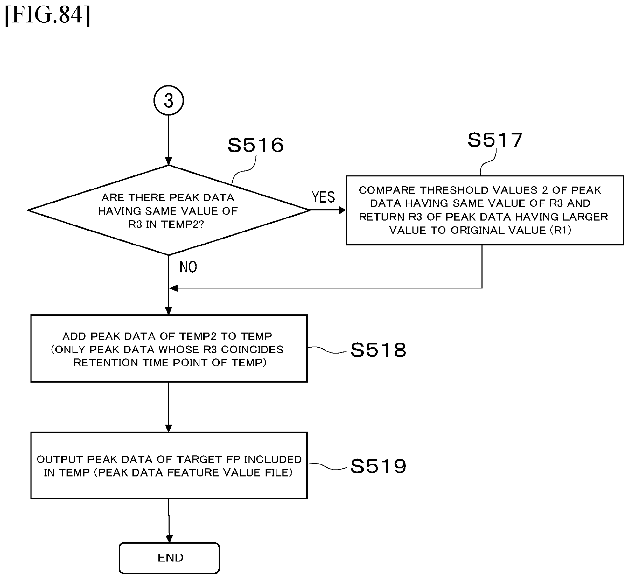

Figs. 83 and 84 are flowcharts illustrating details of the "target FP assigning process 4" of Step S5 in Fig. 76. This process is a final process of the assignment and assigns the peaks of the target FP to the respective peaks of the reference group FP based on the collation result file prepared in Step S4 of Fig. 76. In addition, the reference group FP is a FP that specifies the correspondence relation among all the reference FPs as described above. The reference group FP is data configured by reference group FP peak numbers, reference group retention time points and peak heights similar to the example of the reference group FP data 137 in Fig. 98. As the reference group FP 19 illustrated in Fig. 2, each peak can be denoted by an average value (black point) ± standard deviation (vertical line).

In Step S501, a process of "reading the collation result file" is performed. In this process, the collation result file output in Step S412 illustrated in Fig. 82 is read, and it proceeds to Step S502.

In Step S502, a process of "reading the reference group FP" is performed. In this process, the reference group FP that is a final assignment opponent of each peak of the target FP is read, and it proceeds to Step S503.

In Step S503, a process of "integrating and storing the target FP and the reference group FP (TEMP)" is performed. In this process, two files are integrated based on the peak data of the reference FP that is commonly present in the collation result file and the reference group FP to store the result as TEMP, and it proceeds to Step S504.

In Step S504, a process of "correcting the retention time point of the peak of the target FP that does not correspond to any peaks in the reference FP" is performed. In this process, the retention time points of all the peaks of the target FP that do not correspond to any peaks in the reference FP in the collation result file are corrected to the retention time points of TEMP stored in Step S503, and it proceeds to Step S505. In addition, the correction for the retention time point is performed by the same method as that of Step S410 of the "Target FP Assigning Process 3".

In Step S505, a process of "sequentially acquiring the peak data (P1) corresponding to the corrected retention time point (R1 and R3)" is performed. In this process, peak data pieces of peaks corresponding to retention time points corrected in Step S504 as R1 and R3 are sequentially acquired as P1, and it proceeds to Step S506.

In Step S506, a process of "sequentially acquiring peak data (P2) of the target FP corresponding to retention time point (R2) of assignment candidate peak from TEMP" is performed. In this process, peak data pieces are sequentially acquired as P2 corresponding to retention time points R2 at which no peak of the target FP are assigned from TEMP stored in Step S503, and it proceeds to Step S507.

In Step S507, a determining process "|R1 - R2| < threshold value 1?" is performed. In this process, it is determined whether or not a difference between the retention time points R1 and R2 acquired in Steps S505 and S506 is less than the threshold value 1. If a difference is less than the threshold value (YES), it is determined that there is a possibility that the retention time point of the target FP with the retention time point R1 corresponding to the retention time point of the reference FP with the retention time point R2, and it proceeds to Step S508. If a difference between the retention time points R1 and R2 is the threshold value 1 or more (NO), it is determined that there is no possibility of the correspondence, and it proceeds to Step S512.

In Step S508, a process of "acquiring UV spectra (U1, U2) corresponding to the retention time points R1 and R2" is performed. In this process, the UV spectra corresponding to the peaks of the retention time points of R1 and R2 that are determined to have the possibility of the correspondence in Step S507 are acquired from respective FPs, and it proceeds to Step S509.

In Step S509, a process of "calculating the degree of matching between the UV spectra (UV_Sim)" is performed. In this process, the UV_Sim is calculated using the same method as that of Step S306 of the "Target FP Assigning Process 2" of Step S3 based on the UV spectra U1 and U2 acquired in Step S508, and it proceeds to Step S510. In addition, a detailed calculation flow of the UV_Sim will be additionally described with reference to Subroutine 2 illustrated in Fig. 86 separately.

In Step S510, a determining process "UV_Sim < threshold value 2?" is performed. In this process, it is determined whether the UV_Sim calculated in Step S509 is less than the threshold value 2. If it is less than the threshold value 2 (YES), it is determined that the peak of the UV spectrum U1 corresponds to the peak of U2, and it proceeds to Step S511. If the UV_Sim is the threshold value 2 or more (NO), it is determined that there is no correspondence, and it proceeds to Step S507.

In Step S511, a process of "R3 ← R2, and threshold value 2 ← UV_Sim" is performed. In this process, the retention time point R3 (that is, R1) determined to have the correspondence in Step S510 is updated with R2 that is the retention time point of the corresponding opponent, and thereafter, the threshold value 2 is updated with the value of UV_Sim, and it proceeds to Step S507.

In Step S512, a determining process "Have the retention time points of all the assignment candidate peaks been compared?" is performed. In this process, it is determined whether comparisons of R1 with the retention time points of all the assignment candidate peaks have been completed. If completed (YES), it proceeds to Step S513. If not completed (NO), it proceeds to Step S507.

In Step S513, a process of "storing R1, R3 and P1 as well as the threshold value 2 (TEMP2)" is performed. In this process, the retention time point (R1) determined to have the correspondence in Step S510 and the peak (P1) corresponding to R3 updated to the retention time point (R2) of the corresponding opponent are stored as well as the threshold value 2 at this time (TEMP2), and it proceeds to Step S507.

In Step S514, a determining process "Have the retention time points of all non-corresponding peaks been compared?" is performed. In this process, it is determined whether or not comparisons with the retention time points of the assignment candidate peaks have been completed in the retention time points of all non-corresponding peaks. If completed (YES), it is determined that the assignment process of all the non-corresponding peaks has been completed, and it proceeds to Step S516. If not completed (NO), it is determined that one or more non-corresponding peaks that have not been processed remain, and it proceeds to Step S515.

In Step S515, a process of "threshold value 2 <- initial value" is performed. In this process, the threshold value 2 that is updated to UV_Sim in Step S511 is returned to the initial value, and it proceeds to Step S505.

In Step S516, a determining process "Are there peaks having the same value of R3 present in TEMP2?" is performed. In this process, it is determined whether or not a plurality of non-corresponding peaks are assigned to the same peak in TEMP. If there are non-corresponding peaks assigned to the same peak (YES), it proceeds to Step S517. If such non-corresponding peak is not present (NO), it proceeds to Step S518.

In Step S517, a process of "comparing the threshold values 2 of the peaks having the same values of R3 and returning R3 of the peak having a larger threshold value to its original value (R1) " is performed. In this process, the threshold values 2 of the peaks having the same value of R3 in TEMP2 are compared with each other, to return the value of R3 of the peak having a larger threshold value to its original value (in other words, R1), and it proceeds to Step S518.

In Step S518, a process of "adding a peak of TEMP2 to TEMP (only a peak of whose R3 coincides with the retention time point of TEMP)" is performed. In this process, every peak of which R3 coincides with the retention time point of TEMP is added to TEMP, and it proceeds to Step S519. Every peak of which R3 does not coincide with the retention time point of TEMP is not added, because there is no peak to be an assignment opponent in the reference group FP.

In Step S519, a process of "outputting the peaks of the target FP included in TEMP (peak feature value file)" is performed. In this process, the peak data of the target FP assigned to the reference group FP 137 is output as a peak data feature value file, to finish the target FP assigning process 4.

Fig. 99 shows an example of the peak data feature value file 139 output as described above.

[Subroutine 1]

Fig. 85 is a flowchart that illustrates details of the "Subroutine 1" of the "reference FP selecting process" of Fig. 80. This process calculates the degree of matching between retention time appearance patterns of FPs (for example, a target FP and a reference FP).

In Step S1001, a process of "x <- R1 and y <- R2" is performed. In this process, R1 and R2 acquired in Steps S202 and S206 of Fig. 80 are respectively substituted into "x" and "y", and it proceeds to Step S1002.

In Step S1002, a process of "acquiring the numbers of data "x" and "y" (a, b)" is performed. In this process, the numbers of data "x" and "y" are acquired as "a" and "b," respectively, and it proceeds to Step S1003.

In Step S1003, as an initial value of a counter used for sequentially invoking the retention time points of "x", "1" is substituted into "i" (i <- 1), and it proceeds to Step S1004.

In Step S1004, a process of "acquiring all distances from the xi-th retention time point (f)" is performed. In this process, all distances, from the xi-th retention time point, of retention time points after the xi-th retention time point are acquired as "f", and it proceeds to Step S1005.

In Step S1005, as an initial value of a counter for sequentially invoking the retention time points of "y", "1" is substituted into "j" (j <- 1), and it proceeds to Step S1006.

In Step S1006, a process of "acquiring all distances from the yj-th retention time point (g)" is performed. In this process, all distances, from the yj-th retention time point, of retention time points after the yj-th retention time point are acquired as "g", and it proceeds to Step S1007.

In Step S1007, a process of "acquiring the number of data pieces satisfying a condition of "linter-retention time point distance of "f"- inter-retention time point distance of "g"| < threshold value" (m)" is performed. In this process, inter-retention time point distances "f" and "g" acquired in Steps S1004 and S1006 are compared with each other in a round robin manner, the number of data pieces satisfying the condition of "|inter-retention time point distance of "f" - inter retention time point distance of "g"| < threshold value" is acquired as "m", and it proceeds to Step S1008.

In Step S1008, a process of "calculating the degree of matching between the retention time appearance patterns of "f" and "g" (RP$_{fg}$)" is performed. In this process, RP$_{fg}$ is calculated based on "a" and "b" acquired in Step S1002 and "m" acquired in Step S1007 as:

$$RP_{fg} = (1 - (m/(a + b - m))) \times (a - m + 1).$$

It proceeds to Step S1009.

In Step S1009, a process of "storing RP$_{fg}$ (RP_all)" is performed. In this process, the degree of matching calculated in Step S1008 is stored in RP_all, and it proceeds to Step S1010.

In Step S1010, a process of "updating j (j <- j + 1)" is performed. In this process, in order to perform the process of "y" at the next retention time point, "j + 1" is substituted into "j" as the update of "j", and it proceeds to Step S1011.

In Step S1011, a determining process "Has the process been completed at all the retention time points of "y"?" is performed. In this process, it is determined whether or not the process of all the retention time points of "y" has been completed. If completed (YES), it is determined that the process of all the retention time points has been completed, to proceed to Step S1012. If not completed (NO), it is determined that one or more retention time points that have not been processed remain in "y", to proceed to Step S1006. In other words, the process of Steps S1006 to S 1011 is repeated until all the retention time points of "y" are processed.

In Step S1012, a process of "updating "i" (i <- i + 1)" is performed. In this process, as the update of "i" for bringing the process of "x" to the next retention time point, "i+1" is substituted into "i", and it proceeds to Step S1013.

In Step S1013, a determining process "Has the process been completed at all the retention time points of "x"?" is performed. In this process, it is determined whether or not the process of all the retention time points of "x" has been completed. If completed (YES), it is determined that the process of all the retention time points of "x" has been completed, to proceed to Step S1014. If not completed (NO), it is determined that one ore more retention time points that have not been processed remain in "x", to proceed to Step S1004. In other words, the process of Steps S1004 to S1013 is repeated until all the retention time points of "x" are processed.

In Step S1014, a process of "acquiring a minimum value from RP_all (RP_min)" is performed. In this process, the minimum value in RP_all in which RPs for all the combinations of the retention time appearance patterns of the target FP and the reference FP are stored is acquired as RP_min, and RP_min is input to Step S207 of Fig. 80 to finish the process of calculating the degree of matching between the retention time appearance patterns.

[Subroutine 2]

Fig. 86 is a flowchart that illustrates the "Subroutine 2" of the "target FP assigning process 2" of Fig. 81 in detail. In this process, the degree of matching between the UV spectra is calculated.

In Step S2001, a process of "x <- U1, y <- U2, z <- 0" is performed. In this process, the UV spectra U1 and U2 acquired in Steps S302 and S304 of Fig. 81 are respectively substituted into "x" and "y", and furthermore, "0" is substituted as an initial value of the sum (z) of squares of a distance of the UV spectra, and it proceeds to Step S2002.

In Step S2002, a process of "acquiring the number of data pieces of "x" (a)" is performed. In this process, the number of data pieces of "x" is acquired as "a", and it proceeds to Step S2003.

In Step S2003, a process of "i <- 1" is performed. In this process, "1" is substituted into "i" as an initial value used for sequentially invoking absorbance at each detection wavelength configuring the UV spectra U1 and U2 from "x" and "y", and it proceeds to Step S2004.

In Step S2004, a process of "acquiring the xi-th data (b)" is performed. In this process, the i-th absorbance data of "x" into which the UV spectrum "U1" is substituted is acquired as "b", and it proceeds to Step S2005.

In Step S2005, a process of "acquiring yi-th data (c)" is performed. In this process, the i-th absorbance data of "y" into which UV spectrum U2 is substituted is acquired as "c", and it proceeds to Step S2006.

In Step S2006, a process of "calculating an inter-UV spectra distance (d) and a sum (z) of squares of the inter-UV spectra distance" is performed. In this process, the inter-UV spectra distance "d" and the sum "z" of squares of the inter-UV spectra distance are calculated as:

$$d = b - c;$$

and

$$z = z + d^2.$$

It proceeds to Step S2007.

In Step S2007, a process of "updating i (i <- i + 1)" is performed. In this process, as the update of "i," "i+1" is substituted into "i," to proceed to Step S2008.

In Step S2008, a determining process "Have the process of all data of "x" been completed ?" is performed. In this process, it is determined whether the process of all data of "x" and "y" have been completed. If completed (YES), it is determined that the process of all data of "x" and "y" have been completed, to proceed to Step S2009. If not completed (NO), it is determined that there are one or more data pieces of "x" and "y" that have not been processed, to proceed to Step S2004. In other words, the process of Steps S2004 to S2008 is repeated until all the absorbance data of "x" and "y" is processed.

In Step S2009, a process of "calculating the degree of matching between the UV spectra of "x" and "y" (UV_Sim)" is performed. In this process, UV_Sim is calculated based on the sum "z" of squares of the inter-UV spectra distance and the number "a" of data of "x" as follows:

$$UV\_Sim = \sqrt{(z/a)}.$$

UV_Sim is input to Step S306 of Fig. 81, to finish the process of calculating the degree of matching between UV spectra.

[Subroutine 3]

Fig. 87 is a flowchart of that illustrates details of the "Subroutine 3" of the "target FP assigning process 2" of Fig. 81. In this process, the degrees of matching between peak patterns are calculated.

In Step S3001, a process of "setting the number (m) of peak pattern configuring candidates and the number (n) of peak pattern configuring peaks" is performed. In this process, as setting for comprehensively preparing peak patterns, the number (m) of peak pattern configuring candidates and the number (n) of peak pattern configuring peaks are set, and it proceeds to Step S3002.

In Step S3002, a process of "x ← target FP name, r1 ← R1, p1 ← P1, y ← reference FP name, r2 ← R2, and p2 ←P2" is performed. In this process, the file names of the target FP and the reference FP that are necessary for the process, and the retention time points and the peak data acquired in Steps S302 and S304 of Fig. 81 are substituted into "x," "r1," and "p1," and "y," "r2," and "p2," and it proceeds to Step S3003.

In Step S3003, a process of "acquiring all retention time points of "x" (a)" is performed. In this process, a file (target FP) having a name substituted into "x" in Step S3002 is read, all the retention time points of the file are acquired as "a", and it proceeds to Step S3004.

In Step S3004, a process of "acquiring all retention time points of "y" (b)" is performed. In this process, a file (reference FP) having a name substituted into "y" in Step S3002 is read, all the retention time points of the file are acquired as "b", and it proceeds to Step S3005.

In Step S3005, a process of "acquiring retention time points (cm) and peak data (dm) of m peak pattern configuring candidate peaks of "r1" from "a"" is performed. In this process, retention time points of m peak pattern configuring candidate peaks of "r1" that are the retention time points of the assignment target peaks are acquired as "cm" and the peak data thereof as "dm" from "a", and it proceeds to Step S3006. Here, m peak pattern configuring candidate peaks are m peaks with retention time points close to "r1."

In Step S3006, a process of "acquiring retention time points (em) and peak date (fm) of m peak pattern configuring candidate peaks of "r2" from "b"" is performed. In this process, retention time points of m peak pattern configuring candidate peaks of "r2" that are the retention time points of the assignment target peaks are acquired as "em" and the peak data thereof as "fm" from "b", and it proceeds to Step S3007. Here, m peak pattern configuring candidate peaks are m peaks with retention time points close to "r2".

In Step S3007, a process of "aligning "cm" and "dm" in the retention time order (ascending order)" is performed. In this process, "cm" and "dm" acquired in Step S3005 are rearranged so as to be in the ascending order of the retention time, and it proceeds to Step S3008.

In Step S3008, a process of "aligning "em" and "fm" in the retention time order (ascending order)" is performed. In this process, "em" and "fm" acquired in Step S3006 are rearranged so as to be in the ascending order of the retention time, and it proceeds to Step S3009.

In Step S3009, a process of "sequentially acquiring retention time points (cn) and peak data (dn) of n peak pattern configuring peaks from "cm" and "dm"" is performed. In this process, retention time points are sequentially acquired as "cn" and the peak data thereof as "dn" from "cm" and "dm" of m peak pattern configuring candidate peaks, and it proceeds to Step S3010.

In Step S3010, a process of "sequentially acquiring retention time points (en) and peak data (fn) of n peak pattern configuring peaks from "em" and "fm"" is performed. In this process, retention time points of n peak pattern configuring peaks are sequentially acquired as "en" and the peak data thereof as "fn" from "em" and "fm" of m peak pattern configuring candidate peak, and it proceeds to Step S3011.

In Step S3011, a process of "calculating the degree of matching between peak patterns (P_Sim)" is performed. In this process, the degree (P_Sim) of matching between peak patterns is calculated based on "r1" and "p1" of the assignment target peaks, "cn" and "dn" of n peak pattern configuring peaks, "r2" and "p2" of the assignment candidate peaks, and "en" and "fn" of n peak pattern configuring peaks, which have been acquired until now as:

$$P\_Sim = (|p1 - p2| + 1) \times (|(r1 - (r2 + d)| + 1) + (|dn1 - fn1| + 1) \times (|(cn1 - r1) - (en1 - r2)| + 1)$$

$$+ (|dn2 - fn2| + 1) \times (|(cn2 - r1) - (en2 - r2)| + 1)$$

$$+ (|dn3 - fn3| + 1) \times (|(cn3 - r1) - (en3 - r2)| + 1)$$

$$+ (|dn4 - fn4| + 1) \times (|(cn4 - r1) - (en4 - r2)| + 1)$$

in the case of n = 4 as an example as represented in Fig. 64, and it proceeds to Step S3012.

In Step S3012, a process of "storing P_Sim (P_Sim all)" is performed. In this process, P_Sim calculated in Step S3011 is sequentially stored in P_Sim-all, and it proceeds to Step S3013.

In Step S3013, a determining process "Have all the combinations to take out n pieces from m pieces included in "em" been completed?" is performed. In this process, it is determined whether or not the process has been completed for all the combinations to take out n peak pattern configuration peaks out from m peak pattern configuring candidate peaks. If completed (YES), it is determined that the preparation of comprehensive peak patterns and the calculation of the degrees of matching for the patterns have been completed for the assignment candidate peaks, to proceed to Step S3014. If not completed (NO), it is determined that one or more combinations to take out n pieces out from m pieces have not been completed, to proceed to Step S3010. In other words, the process of Steps S3010 to S3013 is repeated until the process is completed for all the combinations to take out n pieces out from m pieces.

In Step S3014, a process of determining "Have all the combinations to take out m pieces from n pieces included in "cm" been completed?" is performed. In this process, it is determined whether or not the process has been completed for all the combinations to take out n peak pattern configuring peaks from m peak pattern configuring candidate peaks of the assignment target peak. If completed (YES), it is determined that the preparation of comprehensive peak patterns and the calculation of the degrees of matching for the patterns have been completed for the assignment candidate peak, to proceed to Step S3015. If not completed (NO), it is determined that one or more combinations to take out n pieces from m pieces has not been completed, to proceed to Step S3009. In other words, the process of Steps S3009 to S3014 is repeated until the process is completed for all the combinations to take out n pieces out from m pieces.

In Step S3015, a process of "acquiring a minimum value from P_Sim_all (P_Sim_ min)" is performed. In this process, the minimum value of the P_Sim-all stored in S3012 is acquired as P_Sim_min, and the P_Sim_min is input to Step S307 of Fig. 81 to finish the process of calculating the degree of matching between peak patterns.

[Preparation of Reference Group FP]

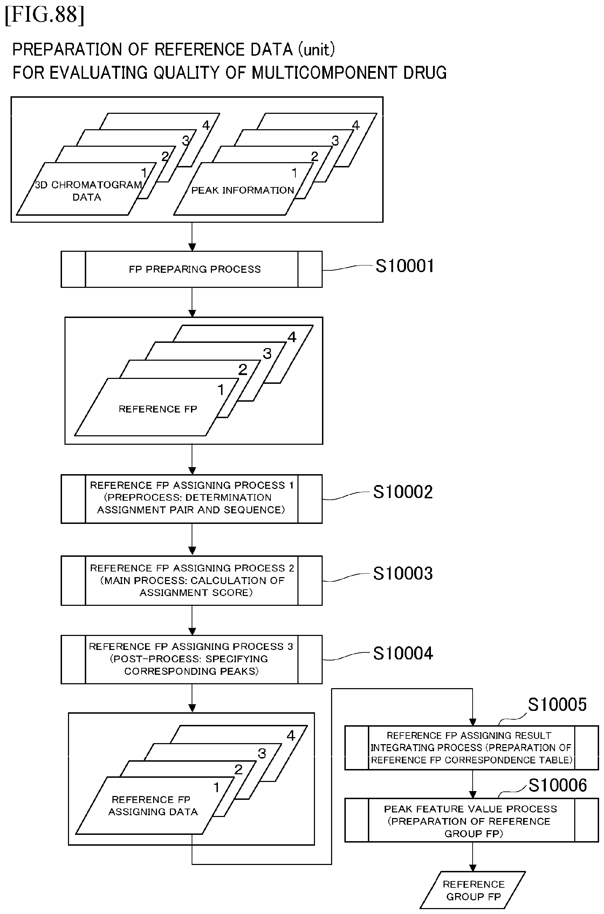

The reference FP feature value file is prepared for comparing the target FP feature value data with the reference FP feature value data as illustrated in Figs. 88 to 91.

Fig. 88 is a flowchart that is used for preparing a reference FP feature value file. It realizes a FP preparing function of a reference FP preparing part, a reference FP peak assigning function of a reference FP peak assigning part, a reference FP assigning result integrating function of a reference FP assigning result integrating part, and a reference FP peak feature value preparing function of a reference FP peak feature value preparing unit in a computer.

The reference FP preparing function is realized in Step S10001. The reference FP peak assigning function is realized in Steps S10002, S10003, and S10004. The reference FP assigning result integrating function is realized in Step S10005. The reference FP peak feature value preparing function is realized in Step S10006.

Steps 510001 to S10004 correspond to Steps S1 to S4 relating to the preparation of the target FP feature value integrating file illustrated in Fig. 76.

In Step 510001, the "FP preparing process" is performed according to a 3D chromatogram and peak information at a specific detection wavelength as inputs.

Both the 3D chromatograph and the peak data are provided for each one of a plurality of evaluation reference drug (reference kampo medicine) that are evaluation criteria.

In Step S10001, the reference FP preparing part of the computer functions and a reference FP is prepared similarly to the target FP 17 (Fig. 2) based on the 3D chromatogram and the peak information, and data of the reference FP is output as a file.

In Step S10002, the "reference FP assigning process 1" is performed according to all reference FPs output in Step S10001 as inputs.

In Step S10002, the reference FP peak assigning part of the computer functions, and, for all the reference FPs, a combination is selected from among the all reference FPs in order to calculate assignment scores for the selected combination in the selected order, and it proceeds to Step S10003.

In Step S10003, the "reference FP assigning process 2" is performed according to the selected combination of the reference FPs as an input.

In Step S10003, for all the peaks of the combination of the reference FPs that is selected in Step S2, peak patterns are comprehensively prepared as illustrated in Figs. 23 to 61. Then, the degrees of matching between the peak patterns (P_Sim illustrated in Fig. 63 or 64) are calculated. In addition, the degrees of matching between UV spectra (UV_Sim illustrated in Fig. 66) of the peaks of the selected combination of the reference FPs are calculated. Furthermore, the degrees of matching of the assignment candidate peaks (SCORE illustrated in Fig. 67) are calculated based on these two degrees of matching. The calculation result is output as a determination result file (see the determination result file example 129 illustrated in Fig. 95).

In Step S10004, the "reference FP assigning process 3" is performed according to the determination result file output in Step S10003 as input.

In Step S10004, between the reference FPs in the selected combinations, peaks of the reference FPs in the selected combinations, which correspond to each other are specified based on the degree of matching between the assignment candidate peaks (SCORE). The result is output as the reference FP assigning data for each reference FP.

In Step S10005, the "reference FP assigning result integrating process" is performed according to all the reference FP assigning data output in Step S10004 is received as input.

In Step S10005, the reference FP assigning result integrating part of the computer functions to prepare a reference FP correspondence table by integrating all the FP assigning data with reference to the peak correspondence relation of the individual reference FP specified by the reference FP peak assigning part, and it proceeds to Step S10006.

In Step S10006, the reference FP peak feature value preparing part of the computer functions to prepare a peak feature value (reference group FP) according to the all reference FPs based on the reference FP correspondence table that is prepared by the reference FP assigning result integrating part. In the process at the reference FP peak feature value preparing part, statistic values (a maximum value, a minimum value, a medium value, an average value, and the like) are calculated for each peak (column) in the reference FP correspondence table, to select the peak (column) based on the calculated information. The selected peak (column) is output as the reference group FP (see the reference group FP example 137 illustrated in Fig. 98).

[S10005: Preparation of Reference FP Correspondence Table]

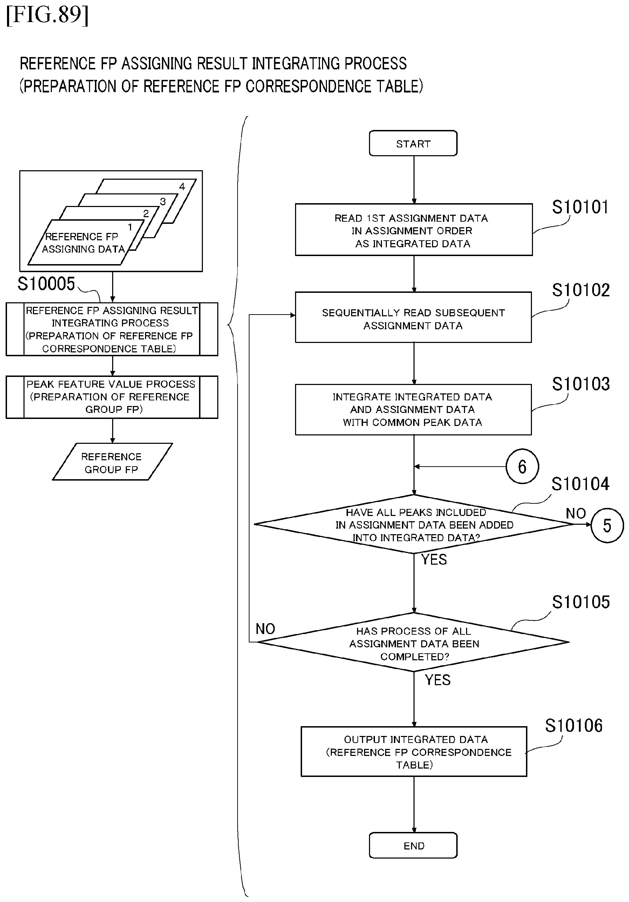

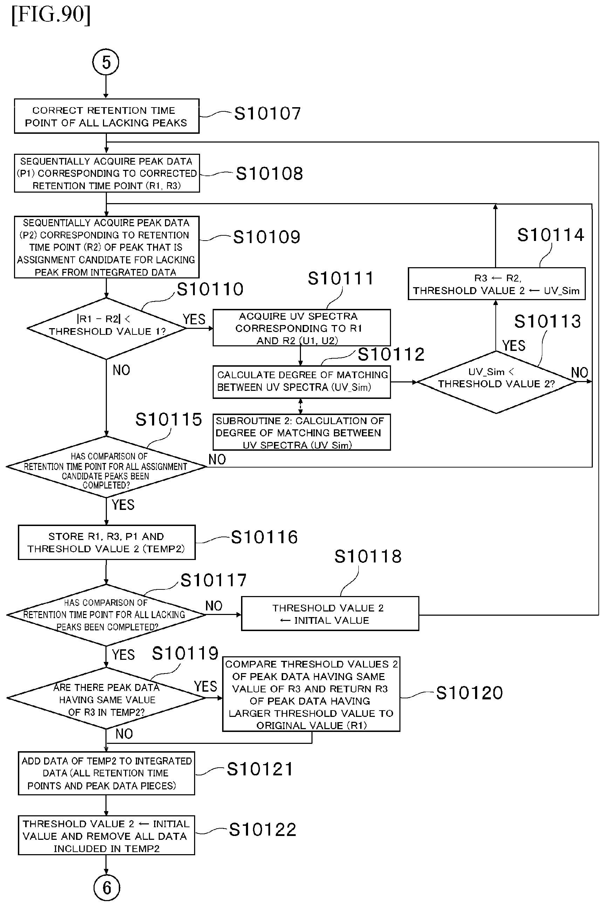

Figs. 89 and 90 are flowcharts that illustrate details of the "reference FP assigning result integrating process illustrated in Step S10005 (preparation of a reference FP correspondence table)."

In Step S10101, a process of "reading the 1st assignment data in the assignment order as integrated data" is performed. In this process, the reference FP assigning data, in which the assignment process is performed first to specify the correspondence relation of peaks in Step S10004, is read as the integrated data. Then, it proceeds to Step S 1 0 1 02.

In Step S10102, a process of "sequentially reading subsequent assignment data" is performed. In this process, at first the reference FP assigning data, in which the assignment process is secondarily performed to specify the correspondence relation of peaks in Step S10004, is read as integrated data. Then, it proceeds to Step S10103.

In Step S10103, a process of "integrating the integrated data and the assignment data with common peak data" is performed. In this process, the two files are integrated based on the peak data of the reference FP commonly-existing in the integrated data and the assignment data, the integrated data is updated as a result thereof, and it proceeds to Step S10104.

In Step S10104, a determining process "Have all the peaks included in the assignment data been added to the integrated data?" is performed. In this process, it is determined whether or not all the peaks in the assignment data have been added to the integrated data. If added (YES), it proceeds to Step S10105. If there is one or more peaks (lacking peaks) that have not been added (NO), in order to add the lacking peaks to the integrated data, it proceeds to Step S10107. In addition, in the process (S10107 to S10120) of adding the lacking peaks to the integrated data, the same process as that of Steps S504 to S517 in S5 (target FP assigning process 4) is performed.

In Step S10121, a process of "adding data of TEMP2 to the integrated data (all the retention time points and peaks)" is performed. In this process, all the retention time points (R3) and the peaks (P1) in TEMP2 are added to corresponding positions in the integrated data, and it proceeds to Step S10122.

In Step S10122, a process of "threshold value 2 <- initial value, and deleting all the data in TEMP2" is performed. In this process, the threshold value 2 updated to UV_Sim is returned to the original value, all the data are deleted from TEMP2 storing data such as retention time points and peaks of all the lacking peaks and the like, and it is returned to Step S10104.

In Step S10105 to which it proceeds from Step S10104, a determining process "Has the process of all the assignment data been completed?" is performed. In this process, it is determined whether or not the process of all reference data has been completed. If completed (YES), in order to output a reference FP correspondence table that is an integration result of all the assignment data, it proceeds to Step S10106. If not completed (NO), it is returned to Step S10102 to sequentially process the remaining assignment data.

In Step S10106, a process of "outputting the integrated data (reference FP correspondence table)" is performed. In this process, the result integrating all the assignment data is output as the reference FP correspondence table, to finish the process of preparing the reference FP correspondence table.

[S10006: Peak Feature Value Process]

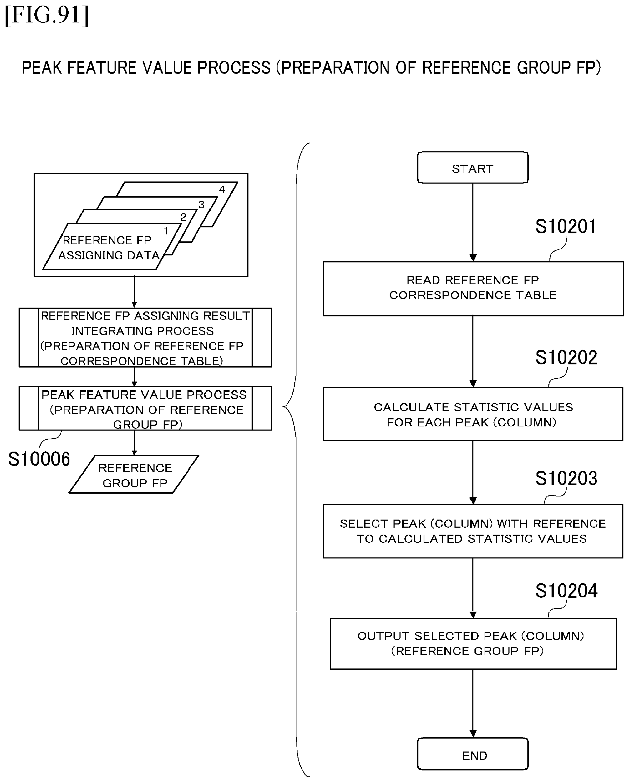

Fig. 91 is a flowchart that illustrates details of the "peak feature value process (preparation of a reference group FP)" of Step S10006 in Fig. 88.

In Step 510201, a process of "reading the reference FP correspondence table" is performed. In this process, the reference FP correspondence table prepared in Step S10005 is read to proceed to Step S10202.

In Step S10202, a process of "calculating statistic values for each peak (column)" is performed. In this process, the statistic values (a maximum value, a minimum value, a medium value, an average value, a variance, a standard deviation, an existence number, and an existence ratio) are calculated for each peak (column) of the reference FP correspondence table. Then, it proceeds to Step S10203.

In Step S10203, a process of "selecting a peak (column) with reference to the calculated statistic values" is performed. In this process, a peak is selected with reference to the statistic values calculated in Step S10102, to proceeds to Step S10204.

In Step S10204, a process of "outputting the selected peak (column) (reference group FP)" is performed. In this process, the selecting result of the peak (column) according to the statistic amounts is output as the reference group FP to finish the process of preparing the reference group FP.

Fig. 98 illustrates a reference FP correspondence table example 137 output as described above.

[Effects of Embodiment 1]

In Embodiment 1 of the present invention, the FP preparing step 113 preparing target FP 17 that comprises peaks, retention time points and UV spectra of the peaks detected from the 3D chromatogram 15 of the multicomponent drug that is the evaluation target at a specific wavelength, for example, 203 nm; the reference FP selecting step 115 selecting a reference FP that is appropriate to peak assignment of the target FP 17 from among a plurality of reference FPs; a peak pattern preparing step 117 preparing peak patterns that comprises, for example, three peaks including two peaks that are present at least on one of sides located in front and in the rear in a time axis direction for each peak of the target FP and the selected reference FP; the peak assigning step 119 comparing the peak patterns and the UV spectra of the peaks to specify corresponding peaks; and the evaluating step 121 evaluating the assigned peak of the target FP by comparison with the peaks of the plurality of reference FPs, for example, with use of MT method.

By processing the 3D chromatogram 15 of the multicomponent drug that is an evaluation target through these five steps (113, 115, 117, 119, and 121), it can improve the accuracy and the efficiency of the quality evaluation of the evaluation target drug.

The target FP 17 prepared by the FP preparing step 113, similarly to the 3D chromatogram 15, is configured as three dimensional information (peaks, retention time points, and UV spectra). Accordingly, the target FP 17 is data directly succeeding to the information unique to the drug. In spite of that, the data volume is compressed at the ratio of about 1/70, compared to the 3D chromatogram 15, the amount of information to be processed can be greatly reduced to increase the processing speed.

The FP preparing step 113 prepares a FP by composing a plurality of FPs at different detection wavelengths.

Accordingly, for even a multicomponent drug acquired by combining components all of which cannot be detected using one wavelength, a quality evaluation including all the components can be performed by composing FPs having a plurality of detection wavelengths.

The FP preparing step 113 prepares a FP that includes all the peaks detected in the 3D chromatogram. Accordingly, the FP preparing step is suited for an evaluation of the quality of a kampo medicine that is a multicomponent drug.

The reference FP selecting step 115 compares retention time appearance patterns of FPs with each other, to select a reference FP having a high degree of matching between patterns as a reference FP that is appropriate to the assignment. Accordingly, in the peak assigning step 119, the assignment process can be performed between FPs having similar patterns, whereby assignment can be performed with high accuracy.

The peak pattern preparing step 117 comprehensively prepares peak patterns with use of a plurality of peripheral peaks for each of the assignment target peak and the assignment candidate peak. Accordingly, even if there is a difference between the whole patterns of the target FP and the reference FP more or less, assignment can be performed through the peak assigning step 119 with high accuracy.

In the peak assigning step 119, in addition to the degree of matching between peak patterns prepared by the peak pattern preparing step 117, the degree of matching between UV spectra of the assignment target peak and the assignment candidate peak is used for specifying the peak to be assigned. Accordingly, assignment can be performed with high accuracy.

The peak assigning step 119 assigns all the peaks of the target FP to the peaks of the reference FP all together. Accordingly, the assignment process can be performed with high efficiency.

The evaluating step 121 collects a FP that is composed by multiple components as multi-dimensional data as a MD value in one dimension by MT method, to easily compare and evaluate a plurality of evaluation target lots. Accordingly, it is suited for evaluating a multicomponent based drug that is composed of multiple components.

The evaluating program for a multicomponent drug according to this embodiment of the present invention realizes the functions in a computer to improve the accuracy and the efficiency of the evaluation.

The evaluating apparatus for a multicomponent drug according to this embodiment of the present invention operates the units 3, 5, 7, 9 and 11 to improve the accuracy and the efficiency of the evaluation.

[Modified Examples of Calculation of Degree of Matching between Peak Patterns (P_Sim)]

In the case of Figs. 63, 64, and 87, the calculation of the degree of matching between peak patterns (P_Sim) is performed based on a difference between peak heights of comparison targets in the above-described embodiment in which the FPs are prepared with use of peak heights.

In the evaluating method, the evaluating program and the evaluating apparatus, there may be a case where a peak represents a maximum value of a signal strength (height) as described above or a case where a peak represents an area value (peak area) of a signal strength in a form of a height.

In other words, even in the case where the FP is prepared with use of peak areas, the area values are represented in a form of heights to prepare the FP. Accordingly, the FP has the same representation as that of the case where the FP is prepared with use of the peak heights as in the above-described embodiment. Therefore, similar to the case where the FP is prepared with use of the peak heights, the FP can be evaluated by the process of the above-described embodiment.

However, in the case where the FP is prepared with use of the peak areas, differences between the peak values of comparison targets are larger. Accordingly, it is appropriate that the calculation is made based on a ratio so as to make the handling thereof easy.

Hereinafter, the degree of matching between peak patterns (P_Sim) that is calculated based on the ratios will be represented for exemplary cases where n = 2 and n = 4.

In the case where n = 2, the calculation is represented as follows:

$$\mathrm{P\_Sim} = (p1/p2^{\#1}) \times (|(r1 - (r2 + d)| + 1)$$

$$+ (dn1/fn1^{\#1}) \times (|(cn1 - r1) - (en1 - r2)| + 1)$$

$$+ (dn2/fn2^{\#1}) \times (|(cn2 - r1) - (en2 - r2)| + 1).$$

In a case where n = 4, the calculation is represented as follows :

$$P\_Sim = (p1/p2^{\#1}) \times (|(r1 - (r2 + d)| + 1)$$

$$+ (dn1/fn1^{\#1}) \times (|(cn1 - r1) - (en1 - r2)| + 1)$$

$$+ (dn2/fn2^{\#1}) \times (|(cn2 - r1) - (en2 - r2)| + 1)$$

$$+ (dn3/fn3^{\#1}) \times (|(cn3 - r1) - (en3 - r2)| + 1)$$

$$+ (dn4/fn4^{\#1}) \times (|(cn4 - r1) - (en4 - r2)| + 1).$$

Here, $^{\#1}$ represents a ratio (larger value/smaller value) of two comparison target values.

In addition, also in the case where the FP is prepared by means of the peak heights, the degree of matching between peak patterns (P_Sim) can be calculated based on a ratio, and, also in the case where the FP is prepared by means of the peak areas, similarly to the case of a difference between the peak heights, the degree of matching between peak patterns (P_Sim) can be acquired based on a difference between peak area values.

[Modified Example of Subroutine 2]

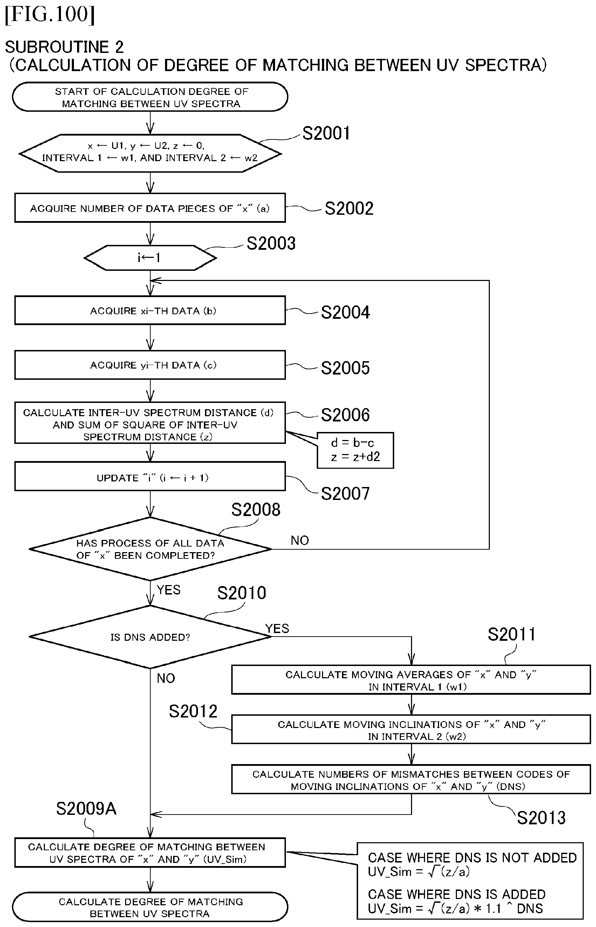

Fig. 100 is a modified example of the "Subroutine 2" that is applied instead of that illustrated in Fig. 86 and is a flowchart illustrating details of the modified example of Subroutine 2 in the "target FP assigning process 2" illustrated in Fig. 81. The degree of matching between UV spectra is calculated by the process according to this modified example.

In the modified example of this Subroutine 2, a process of adding inclination information in moving average of a UV pattern (DNS) to the RMSD of Subroutine 2 in Fig. 86 can be performed. The DNS is represented in an equation to be described later and is defined as the number of mismatches of inclination codes (+/-) when the moving inclinations of the moving average values in the UV pattern are compared between two patterns. In other words, the DNS is a value that represents an evaluation of the matching state of the positions of the maximum and minimum values of the UV patterns.

By adding the DNS information to the RMSD, the degree of matching between waveforms of UV spectra can be calculated more accurately.

In Subroutine 2 according to the modified example of Fig. 100, Steps S2001 to S2008 are almost the same as those of Subroutine 2 in Fig. 86. However, in Step S2001, initial setting of "Interval 1 <- w1 and Interval 2 <- w2" is additionally performed, to be used for calculating the moving average and the moving inclination to be described later.

In Subroutine 2 of this modified example, Steps S2010 to S2013 are added so as to add the DNS, so that it enables Step S2009A to calculate the degree of matching to which the DNS is added.

In Step S2010, a determining process of "Is the DNS added?" is performed. If the DNS is determined to be added (YES), it proceeds to Step S2011. If the DNS is determined not to be added (NO), it proceeds to Step S2009A. The determination whether the DNS is added or not is based on, for example, an initial setting. For example, if the FP is prepared by means of peak areas, the DNS is set to be added; and if the FP is prepared by means of peak heights, the DNS is set to be not added.

However, also in the case of the above-described embodiment in which the FP is prepared by means of peak heights, the degree of matching between UV patterns can be calculated through a process to which the DNS is added; and also in the case where the FP is prepared by means of peak areas, the degree of matching between UV patterns can be calculated through the process of the above-described embodiment to which the DNS is not added.

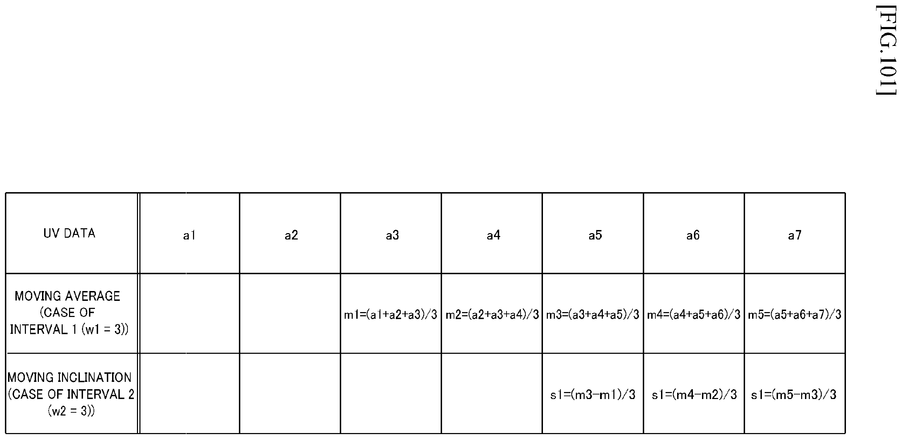

In Step S2011, a process of "calculating the moving averages of "x" and "y" in interval 1 (w1)" is performed, to find the moving averages for interval 1 (w1). Interval 1 (w1) is an interval relating to the wavelength of the UV data. In a case where w1 = 3 in the initial setting of Step S2001, interval 1 (3) is set and the average of the UV intensities of three wavelengths is acquired. More specifically, description will be made later with reference to a table represented in Fig. 101.

In Step S2012, the process of "calculating the moving inclinations of "x" and "y" in interval 2 (w2)" is performed to acquire the moving inclinations in interval 2 (w2). Interval 2 (w2) is an interval relating to the moving average acquired in Step S2011. If w2 = 3 in the initial setting performed in Step S2001, interval 2 (3) is set to acquire inclinations of ($\pm$) over the three moving averages based on the moving averages calculated in Step S2011. More specifically, description will be made later with reference to a table illustrated in Fig. 101.

In Step S2013, a process of "calculating the number of mismatches between the codes of the moving inclinations of "x" and "y" (DNS)" is performed, to calculate the number of matches in the inclinations of ($\pm$) based on the moving inclinations calculated in Step S2012. The moving inclination of (+) represents rising to the right in Fig. 66, and the moving inclination of (-) represents falling to the right.

When proceeding from Step S2013 to Step S2009A, the degree of matching to which the DNS is added is calculated in the process of Step S2009A.

In Step S2009A, a process of "calculating the degree of matching between UV spectra of "x" and "y" (UV_Sim)" is performed. In the calculation process of the degree of matching to which the DNS is added, the UV_Sim is calculated based on the sum "z" of squares of inter-UV spectrum distances, the number "a" of data of "x" and the DNS as:

$$UV\_Sim = \sqrt{(z/a)} \times 1.1^{DNS}.$$

This UV_Sim is input to Step S306 in Fig. 81, to finish the process of calculating the degree of matching between UV spectra.

In addition, the process performed in a case where it proceeds from Step S2010 to Step S2009A is the same as that of Step S2009 in Fig. 86.

Fig. 101 is a table illustrating a calculating example of moving averages and moving inclinations.

In Fig. 101, the upper row represents an example of UV data, the intermediate row represents an example of calculation of moving averages, and the lower row represents an example of calculation of moving inclinations. As the example of the UV data, the UV intensity is represented as a1 to a7 instead of specific numeric values. For example, the UV intensity of 220 nm is a1, the UV intensity of 221 nm is a2, and the like. Also in the example of calculation of the moving averages and moving inclinations, UV intensities a1 to a7 are used instead of specific numeric values.

For the example of the interval 1 (w1 = 3), the moving averages are calculated as m1, m2... as respective values calculated for an interval (a1, a2, a3), an interval (a2, a3, a4)... in Step S2012 (see Fig. 100). In addition, for the example of the interval 2 (3), the moving inclinations are calculated as s1... as respective values calculated for an interval (m1, m2, m3), an interval (m2, m3, m4)... in Step S2013 (see Fig. 100). For example, a difference m3 - m1 between the moving averages is the moving inclination, and ($\pm$) thereof are extracted.

In this way, when preparing the FP by means of peak areas, in the assignment process to the reference group FP and the reference FP assigning result integrating process, the degree of matching between UV patterns can be calculated through the process to which the DNS is added. With this calculation, even if a distance (dis) between two corresponding points illustrated in Fig. 66 is larger relative to the FP prepared by means of peak heights, the handing thereof can be easily performed, thereby calculating the degree of matching between UV patterns with high accuracy.

[Others]

Although this embodiment of the present invention is applied to an evaluation of a kampo medicine as a multicomponent drug, it can be also applied to an evaluation of other multicomponent materials.

An evaluating method for a pattern according to the present invention includes: a pattern acquiring step acquiring a target pattern of an evaluation target whose peaks change in a time series; a peak pattern preparing step preparing individual peak patterns, for each peak, of the target pattern and a reference pattern that corresponds to the target pattern and is evaluation criteria, with use of n+1 peaks that include n peaks being present on at least one of sides located in front and in the rear of each peak in a time axis direction; a peak assigning step comparing the individual peak patterns to specify corresponding peaks; and an evaluating step evaluating the peaks that are specified and assigned in the peak assigning step by comparison with peaks of a plurality of reference patterns that are evaluation criteria. The method is broadly applicable to an evaluation of equivalency between a target pattern and a reference pattern, and the like.

Although all the peaks on the 3D chromatogram are set as targets in the FP of the embodiment, the FP may be prepared with the exclusion of fine data such as peaks each having a peak area corresponding to 5% or less on the 3D chromatogram.

In the above-described embodiment, the FP is prepared based on the peak heights, to acquire evaluations in Figs. 70 to 74. However, even if the FP is prepared based on peak areas, MD values are acquired by MT method through the same sequence as that of the above-described embodiment that is prepared based on the peak heights, to acquire the evaluations as illustrated in Figs. 70 to 74 in the same way.

The chromatogram is not limited to the 3D chromatogram, and a FP that is configured by peaks and retention time points, in which UV spectra are not included, may be used. In such a case, the process can be performed similarly to the above-described embodiment with the exception of the degree of matching between UV spectra.

DESCRIPTION OF THE NUMERALS

1 Evaluating apparatus for a multicomponent drug (Evaluating apparatus for a pattern)

3 FP preparing part (FP acquiring part)

5 Reference FP selecting part (Reference pattern selecting part)

7 Peak pattern preparing part

9 Peak assigning part

11 Evaluating part

13 Kampo medicine

15 3D chromatogram

17 Target FP (Target pattern)

19 Reference group FP

21 Target FP assigned to a reference group FP

23 Evaluation result of a target FP

25 UV spectrum of a peak included into a target FP

27 FP of Drug A

29 FP of Drug B

31 FP of Drug C

33 Target FP (retention time from 10.0 to 14.5 minutes)

35, 37, 39, 41, 43, 45, 47, 49, 51, 53 Each peak included into a target FP (retention time from 10.0 to 14.5 minutes)

55 Reference FP (retention time from 10.0 to 14.5 minutes)

57, 59, 61, 63, 65, 67, 69, 71, 73, 75, 77 Each peak included into a reference FP (retention time from 10.0 to 14.5 minutes)

79 Target FP retention time appearance pattern

81 Reference FP retention time appearance pattern

83 Degree of matching between retention time appearance distances

85 Degree of matching between retention time appearance patterns

87 Peak pattern of a target FP assignment target peak (three peaks)

89, 91, 93, 95 Peak pattern of reference FP assignment candidate peaks (three peaks)

97 Peak pattern of a target FP assignment target peak (five peaks)

99, 101, 103, 105 Peak pattern of reference FP assignment candidate peaks (five peaks)

107 UV spectrum of an assignment target peak

111 UV spectrum of a assignment candidate peak

113 FP preparing step (peak pattern acquiring step)

115 Reference FP selecting step (reference pattern selecting step)

117 Peak pattern preparing step

119 Peak assigning step

121 Evaluating step

123 3D chromatogram data example

125 Peak information data example

127 FP data example

129 Determination result file example

131 Assignment candidate peak score table

133 Assignment candidate peak number table

135 Collation result file example

137 Reference group FP data example

139 Target FP peak feature value file example

**Claims**

1.  An evaluating method for a pattern comprising:

    a fingerprint (FP) preparing step preparing a target fingerprint (33) that comprises peaks (35, 37, 39, 41, 43, 45, 47, 49, 51, 53) and retention time points of the peaks detected from a chromatogram of a multicomponent material that is an evaluation target;
    a peak pattern preparing step configured to
    prepare a peak pattern (87) for an assignment target peak (45) of the target fingerprint (33), the peak pattern (87) being configured by retention time and intensity of n+1 peaks (43, 45, 47) which include n peripheral peaks (43, 47) being present on at least one of the sides located in the front and in the rear of the assignment target peak (45) in a time axis direction; and

prepare peak patterns (89, 91, 93, 95) for respective assignment candidate peaks (65, 67, 69, 71), which are peaks having differences in appearance time point relative to the assignment target peak (45) within a set range, of a reference fingerprint (55) that corresponds to the target fingerprint (33) and comprises peaks (57, 59, 61, 63, 65, 67, 69, 71, 73, 75, 77) and retention time points of the peaks detected from a chromatogram of a multicomponent material determined in advance as a normal product that is evaluation criteria, each of the peak patterns (89, 91, 93 and 95) being configured by retention time and intensity of n+1 peaks (63, 65, 67; 65, 67, 69; 67, 69, 71; 69, 71, 73) which include n peripheral peaks (63, 67; 65, 69; 67, 71; 69, 73) being present on at least one of the sides located in the front and in the rear of the corresponding assignment candidate peak (65, 67, 69, 71) in the time axis direction;

a peak assigning step comparing the peak pattern (87) for the assignment target peak (45) with the peak patterns (89, 91, 93, 95) for the assignment candidate peaks (65, 67, 69, 71) to calculate the degree of matching between the peak pattern for the assignment target peak and the peak patterns for the assignment candidate peaks, to specify and assign corresponding peaks (45, 65) of the target fingerprint (33) and the reference fingerprint (55); and

an evaluating step evaluating the peaks that are specified and assigned in the peak assigning step by comparison with peaks of a plurality of reference fingerprints (55) that are evaluation criteria whose peaks change in a time series.

2. The method according to claim 1, **characterized in that**

the fingerprint (FP) preparing step prepares a target fingerprint (FP) (33) that comprises peaks, retention time points and UV spectra of the peaks detected from a 3D chromatogram of the multicomponent material that is the evaluation target, and

the peak pattern preparing step prepares individual peak patterns, for each peak, of the target fingerprint (FP) (33) and a reference fingerprint (FP) (55) that corresponds to the target fingerprint (FP) and comprises peaks, retention time points and UV spectra of the peaks detected from a 3D chromatogram of the multicomponent material that is the evaluation criteria, with use of n+1 peaks that include n peaks being present on at least one of the sides located in the front and in the rear of each peak in a time axis direction.

3. The method according to claim 1 or 2, **characterized in that**
the fingerprint (FP) preparing step prepares the target fingerprint (FP) (33) by composing a plurality of fingerprints (FP) that are detected at different detection wavelengths from the chromatogram or the 3D chromatogram of the evaluation target.

4. The method according to any one of claims 1 to 3, **characterized in that**
the fingerprint (FP) preparing step extracts all the peaks of the chromatogram or the 3D chromatogram of the evaluation target to prepare the target fingerprint (FP) (33).

5. The method according to any one of claims 1 to 4, **characterized in that**
the peak pattern preparing step comprehensively prepares peak patterns for both a peak being an assignment target of the target fingerprint (FP) (33) and a peak being an assignment candidate of the reference fingerprint (FP) (55) by changing peaks configuring the peak patterns.

6. The method according to any one of claims 1 to 5, **characterized in that**
the peak pattern preparing step prepares the peak patterns for all the peaks of the target fingerprint (FP) (33) and the reference fingerprint (FP) (55).

7. The method according to any one of claims 1 to 6, **characterized in that**
the peak assigning step performs the specifying of the peaks based on a degree of matching between the individual peak patterns calculated from all peaks configuring the peak patterns and the retention time points thereof.

8. The method according to claim 2, **characterized in that**
the peak assigning step performs the specifying of the peaks in consideration of comparison between UV spectra of the peaks of the target fingerprint (FP) (33) and the reference fingerprint (FP) (55).

9. The method according to claim 8, **characterized in that**
the peak assigning step performs the specifying of the peaks based on a degree of matching of the peaks calculated by synthesizing a degree of matching between the individual peak patterns calculated from all peaks configuring the peak patterns and the retention time points thereof and a degree of matching between the UV spectra.

10. The method according to any one of claims 1 to 9, **characterized in that**
the peak assigning step performs the specifying with respect to all the peaks of the target fingerprint (FP) (33) and the reference fingerprint (FP) (55).

11. The method according to any one of claims 1 to 10, **characterized in that**
the peak assigning step specifies the peaks of the target fingerprint (FP) (33) by associating the peaks of the target fingerprint (FP) assigned to the reference fingerprint (FP) (55) with respective peaks of a reference group fingerprint (FP) that is based on the plurality of reference fingerprints (FP).

12. The method according to any one of claims 1 to 11, **characterized in that**
the evaluating step acquires an acquired evaluation (MD) value by a calculation method for quality engineering (MT method) with use of the peaks of the target fingerprint (FP) (33) assigned to the reference group fingerprint (FP)as a feature value and evaluates the acquired evaluation (MD) value by comparison with a plurality of acquired evaluation (MD) values that are evaluation criteria.

13. The method according to any one of claims 1 to 12, **characterized in that**
a reference fingerprint (FP) selecting step selects the reference fingerprint (FP)that is used in the peak pattern preparing step from among the plurality of reference fingerprint (FP)s.

14. The method according to claim 13 **characterized in that**
the reference fingerprint (FP) selecting step compares the retention time points and an appearance pattern of the peaks of the target fingerprint (FP) (33) with those of all the plurality of reference fingerprints (FP) to select a reference fingerprint (FP) that is appropriate as an assignment opponent of the target fingerprint (FP).

15. The method according to any one of claims 1 to 14, **characterized in that**
the multicomponent material is a multicomponent drug that is one of a crude drug, a combination of crude drugs, an extract thereof, and a kampo medicine.

16. An evaluating program for performing an evaluating method for a pattern of claim 1 that causes a computer to execute functions comprising:

a fingerprint (FP) preparing function preparing a target fingerprint (33) that comprises peaks (35, 37, 39, 41, 43, 45, 47, 49, 51, 53) and retention time points of the peaks detected from a chromatogram of a multicomponent material that is an evaluation target;
a peak pattern preparing function configured to
prepare a peak pattern (87) for an assignment target peak (45) of the target fingerprint (33), the peak pattern (87) being configured by retention time and intensity of n+1 peaks (43, 45, 47) which include n peripheral peaks (43, 47) being present on at least one of the sides located in the front and in the rear of the assignment target peak (45) in a time axis direction; and
prepare peak patterns (89, 91, 93, 95) for respective assignment candidate peaks (65, 67, 69, 71), which are peaks having differences in appearance time point relative to the assignment target peak (45) within a set range, of a reference fingerprint (55) that corresponds to the target fingerprint (33) and comprises peaks (57, 59, 61, 63, 65, 67, 69, 71, 73, 75, 77) and retention time points of the peaks detected from a chromatogram of a multicomponent material determined in advance as a normal product that is evaluation criteria, each of the peak patterns (89, 91, 93 and 95) being configured by retention time and intensity of n+1 peaks (63, 65, 67; 65, 67, 69; 67, 69, 71; 69, 71, 73) which include n peripheral peaks (63, 67; 65, 69; 67, 71; 69, 73) being present on at least one of the sides located in the front and in the rear of the corresponding assignment candidate peak (65, 67, 69, 71) in the time axis direction;
a peak assigning function comparing the peak pattern (87) for the assignment target peak (45) with the peak patterns (89, 91, 93, 95) for the assignment candidate peaks (65, 67, 69, 71) to calculate the degree of matching between the peak pattern for the assignment target peak and the peak patterns for the assignment candidate peaks, to specify and assign corresponding peaks (45, 65) of the target fingerprint (33) and the reference fingerprint (55); and
an evaluating function evaluating the peaks that are specified and assigned in the peak assigning function by comparison with peaks of a plurality of reference fingerprints (55) that are evaluation criteria whose peaks change in a time series.

17. The evaluating program for a multicomponent material according to claim 16, **characterized in that**

the fingerprint (FP) preparing function prepares a target fingerprint (FP) (33) that comprises peaks, retention time points and UV spectra of the peaks detected from a 3D chromatogram of the multicomponent material that is the evaluation target, and
the peak pattern preparing function prepares individual peak patterns, for each peak, of the target fingerprint (FP) (33) and a reference fingerprint (FP) (55) that corresponds to the target fingerprint (FP) and comprises peaks, retention time points and UV spectra of the peaks detected from a 3D chromatogram of the multicomponent material that is the evaluation criteria, with use of n+1 peaks that include n peaks being present on at least one of the sides located in the front and in the rear of each peak in a time axis direction.

18. The evaluating program for a multicomponent material according to claim 17, **characterized in that**
the peak assigning function performs the specifying of the peaks in consideration of comparison between UV spectra of the peaks of the target fingerprint (FP) (33) and the reference fingerprint (FP) (55).

19. The evaluating program for a multicomponent material according to claim 18, **characterized in that**
the peak assigning function performs the specifying of the peaks based on a degree of matching of the peaks calculated by synthesizing a degree of matching between the individual peak patterns calculated from all peaks configuring the peak patterns and the retention time points thereof and a degree of matching between the UV spectra.

20. An evaluating apparatus for a pattern comprising:

a fingerprint (FP) preparing part configured to prepare a target fingerprint (33) that comprises peaks (35, 37, 39, 41, 43, 45, 47, 49, 51, 53) and retention time points of the peaks detected from a chromatogram of a multicomponent material that is an evaluation target;
a peak pattern preparing part to configured to
prepare a peak pattern (87) for an assignment target peak (45) of the target fingerprint (33), the peak pattern (87) being configured by retention time and intensity of n+1 peaks (43, 45, 47) which include n peripheral peaks (43, 47) being present on at least one of the sides located in the front and in the rear of the assignment target peak (45) in a time axis direction; and
prepare peak patterns (89, 91, 93, 95) for respective assignment candidate peaks (65, 67, 69, 71), which are peaks having differences in appearance time point relative to the assignment target peak (45) within a set range, of a reference fingerprint (55) that corresponds to the target fingerprint (33) and comprises peaks (57, 59, 61, 63, 65, 67, 69, 71, 73, 75, 77) and retention time points of the peaks detected from a chromatogram of a multicomponent material determined in advance as a normal product that is evaluation criteria, each of the peak patterns (89, 91, 93 and 95) being configured by retention time and intensity of n+1 peaks (63, 65, 67; 65, 67, 69; 67, 69, 71; 69, 71, 73) which include n peripheral peaks (63, 67; 65, 69; 67, 71; 69, 73) being present on at least one of the sides located in the front and in the rear of the corresponding assignment candidate peak (65, 67, 69, 71) in the time axis direction;
a peak assigning part configured to compare the peak pattern (87) for the assignment target peak (45) with the peak patterns (89, 91, 93, 95) for the assignment candidate peaks (65, 67, 69, 71) to calculate the degree of matching between the peak pattern for the assignment target peak and the peak patterns for the assignment candidate peaks, to specify and assign corresponding peaks (45, 65) of the target fingerprint (33) and the reference fingerprint (55); and
an evaluating part configured to evaluate the peaks that are specified and assigned in the peak assigning step by comparison with peaks of a plurality of reference fingerprints (55) that are evaluation criteria whose peaks change in a time series.

21. The evaluating apparatus for a multicomponent material according to claim 20, **characterized in that**

the fingerprint (FP) preparing part (3) prepares a target fingerprint (FP) that comprises peaks, retention time points and UV spectra of the peaks detected from a 3D chromatogram of the multicomponent material that is the evaluation target, and
the peak pattern preparing part (7) prepares individual peak patterns, for each peak, of the target fingerprint (FP) and a reference fingerprint (FP) that corresponds to the target fingerprint (FP) and comprises peaks, retention time points and UV spectra of the peaks detected from a 3D chromatogram of the multicomponent material that is the evaluation criteria, with use of n+1 peaks that include n peaks being present on at least one of the sides located in the front and in the rear of each peak in a time axis direction.

22. The evaluating apparatus for a multicomponent material according to claim 21, **characterized in that** the peak assigning part (9) performs the specifying of the peaks in consideration of comparison between UV spectra of the peaks of the target fingerprint (FP) and the reference fingerprint (FP).

23. The evaluating apparatus for a multicomponent material according to claim 22, **characterized in that** the peak assigning part (9) performs the specifying of the peaks based on a degree of matching of the peaks calculated by synthesizing a degree of matching between the respective patterns calculated from all peaks configuring the peak patterns and the retention time points thereof and a degree of matching between the UV spectra.

**Patentansprüche**

1. Ein Bewertungsverfahren für ein Muster aufweisend:

ein Fingerabdruck(FP)-Erstellungsschritt des Erstellens eines Vorgabefingerabdrucks (33), der Spitzenwerte (35, 37, 39, 41, 43, 45, 47, 49, 51, 53) und Retentionszeitpunkte der Spitzenwerte aufweist, welche von einem Chromatogramm eines Multikomponenten-Stoffes, der eine Bewertungsvorgabe ist, detektiert werden; einen Spitzenwertmuster-Erstellungsschritt, der konfiguriert ist, um ein Spitzenwertmuster (87) für einen Zuordnungsvorgabespitzenwert (45) des Vorgabefingerabdrucks (33) zu erstellen, wobei das Spitzenwertmuster (87) durch die Retentionszeit und die Intensität von n+1 Spitzenwerten (43, 45, 47) konfiguriert ist, die n periphere Spitzenwerte (43, 47) umfassen, die auf wenigstens einer der Seiten an der Vorderseite und der Rückseite des Zuordnungsvorgabespitzenwerts (45) in einer Richtung einer Zeitachse angeordnet auftreten; und Spitzenwertmuster (89, 91, 93, 95) für betreffende Zuordnungskandidatenspitzenwerte (65, 67, 69, 71), welche Spitzenwerte sind, die Unterschiede in dem Erscheinungszeitpunkt in Bezug auf den Zuordnungsvorgabespitzenwert (45) innerhalb eines vorgegebenen Bereichs haben, eines Referenzfingerabdrucks (55) zu erstellen, welcher dem Vorgabefingerabdruck (33) entspricht und Spitzenwerte (75, 59, 61, 63, 65, 67, 69, 71, 73, 75, 77) und Retentionszeitpunkte der Spitzenwerte aufweist, welche von einem Chromatogramm eines Multikomponenten-Stoffes bestimmt werden, welcher im Voraus als ein normales Produkt, das ein Bewertungskriterium ist, bestimmt wird, wobei jedes der Spitzenwertmuster (89, 91, 93 und 95) durch die Retentionszeit und die Intensität von n+1 Spitzenwerten (63, 65, 67; 65, 67, 69; 67, 69, 71; 69, 71, 73) konfiguriert ist, welche n periphere Spitzenwerte (63, 67; 65, 69; 67, 71; 69, 73) umfassen, welche auf wenigstens einer der Seiten an der Vorderseite und der Rückseite der betreffenden Zuordnungskandidatenspitzenwerte (65, 67, 69, 71) in der Richtung der Zeitachse angeordnet auftreten; einen Spitzenwert-Zuordnungsschritt, welcher das Spitzenwertmuster (87) für den Zuordnungsvorgabespitzenwert (45) mit den Spitzenwertmustern (89, 91, 93, 95) für die Zuordnungskandidatenspitzenwerte (65, 67, 69, 71) vergleicht, um den Grad der Übereinstimmung zwischen dem Vorgabemuster für den Zuordnungsvorgabespitzenwert und den Spitzenwertmustern für die Zuordnungskandidatenspitzenwerte zu berechnen, um betreffende Spitzenwerte (45, 65) des Vorgabefingerabdrucks (33) und des Referenzfingerabdrucks (55) zu spezifizieren und zuzuordnen; und einen Bewertungsschritt des Bewertens der Spitzenwerte, die in dem Spitzenwert-Zuordnungsschritt durch den Vergleich mit Spitzenwerten einer Vielzahl von Referenzfingerabdrücken (55), die Bewertungskriterien sind, deren Spitzenwerte sich in einer Zeitreihe verändern, spezifiziert und zugeordnet werden.

2. Das Verfahren nach Anspruch 1, **dadurch gekennzeichnet, dass**

der Fingerabdruck(FP)-Erstellungsschritt einen Vorgabefingerabdruck (FP) (33) erstellt, welcher Spitzenwerte, Retentionszeitpunkte und UV-Spektren der Spitzenwerte aufweist, welche von einem 3D-Chromatogramm eines Multikomponenten-Stoffes, der ein Bewertungskriterium ist, detektiert werden, und der Spitzenwertmuster-Erstellungsschritt individuelle Spitzenwertmuster für jeden Spitzenwert des Vorgabefingerabdruck (FP) (33) und einen Referenzfingerabdruck (FP) (55) erstellt, welcher dem Vorgabefingerabdruck (FP) entspricht und Spitzenwerte, Retentionszeitpunkte und UV-Spektren der Spitzenwerte aufweist, welche von einem 3D-Chromatogramm des Multikomponenten-Stoffes, der das Bewertungskriterium ist, detektiert werden, unter Verwendung von n+1 Spitzenwerten, die n Spitzenwerte umfassen, welche auf wenigstens einer der Seiten an der Vorderseite und der Rückseite jedes Spitzenwertes in der Richtung der Zeitachse angeordnet auftreten.

3. Das Verfahren nach Anspruch 1 oder 2, **dadurch gekennzeichnet, dass** der Fingerabdruck(FP)-Erstellungsschritt den Vorgabefingerabdruck (FP) (33) durch das Zusammenstellen einer Vielzahl von Fingerabdrücken (FP) erstellt, welche an unterschiedlichen Detektionswellenlängen von dem Chromatogramm oder dem 3D-Chromatogramm der Bewertungsvorgabe detektiert werden.

4. Das Verfahren nach einem der Ansprüche 1 bis 3, **dadurch gekennzeichnet, dass** der Fingerabdruck(FP)-Erstellungsschritt sämtliche Spitzenwerte des Chromatogramms oder 3D-Chromatogramms der Bewertungsvorgabe extrahiert, um den Vorgabefingerabdruck (FP) (33) zu erstellen.

5. Das Verfahren nach einem der Ansprüche 1 bis 4, **dadurch gekennzeichnet, dass** der Spitzenwertmuster-Erstellungsschritt sowohl für einen Spitzenwert (45), der eine Zuordnungsvorgabe des Vorgabefingerabdrucks (FP) (33) ist, als auch für einen Spitzenwert, der ein Zuordnungskandidat des Referenzfingerabdrucks (FP) (55) ist, Spitzenwertmuster durch Verändern von die Spitzenwertmuster konfigurierenden Spitzenwerten umfassend erstellt.

6. Das Verfahren nach einem der Ansprüche 1 bis 5, **dadurch gekennzeichnet, dass** der Spitzenwertmuster-Erstellungsschritt die Spitzenwertmuster für sämtliche Spitzenwerte des Vorgabefingerabdrucks (FP) (33) und des Referenzfingerabdrucks (55) erstellt.

7. Das Verfahren nach einem der Ansprüche 1 bis 6, **dadurch gekennzeichnet, dass** der Spitzenwert-Zuordnungsschritt das Spezifizieren der Spitzenwerte auf der Grundlage eines Grades der Übereinstimmung der individuellen Spitzenwertmuster durchführt, welche von sämtlichen die Spitzenwertmuster konfigurierenden Spitzenwerten und deren Retentionszeitpunkten berechnet werden.

8. Das Verfahren nach Anspruch 2, **dadurch gekennzeichnet, dass** der Spitzenwert-Zuordnungsschritt das Spezifizieren der Spitzenwerte unter Berücksichtigung des Vergleichs der UV-Spektren der Spitzenwerte des Vorgabefingerabdrucks (FP) (33) mit dem Referenzfingerabdruck (FP) (55) durchführt.

9. Das Verfahren nach Anspruch 8, **dadurch gekennzeichnet, dass** der Spitzenwert-Zuordnungsschritt das Spezifizieren der Spitzenwerte auf der Grundlage eines Grades der Übereinstimmung der Spitzenwerte durchführt, welche durch das Synthetisieren eines Grades der Übereinstimmung der individuellen Spitzenwertmuster, welche aus sämtlichen die Spitzenwertmuster konfigurierenden Spitzenwerten und deren Retentionszeitpunkten berechnet werden, und eines Grades der Übereinstimmung der UV-Spektren berechnet werden.

10. Das Verfahren nach einem der Ansprüche 1 bis 9, **dadurch gekennzeichnet, dass** der Spitzenwert-Zuordnungsschritt das Spezifizieren in Bezug auf sämtliche Spitzenwerte des Vorgabefingerabdrucks (FP) (33) und des Referenzfingerabdrucks (FP) (55) durchführt.

11. Das Verfahren nach einem der Ansprüche 1 bis 10, **dadurch gekennzeichnet, dass** der Spitzenwert-Zuordnungsschritt die Spitzenwerte des Vorgabefingerabdrucks (FP) (33) durch das Assoziieren der Spitzenwerte des dem Referenzfingerabdruck (FP) (55) zugeordneten Vorgabefingerabdrucks (FP) mit betreffenden Spitzenwerten eines auf einer Vielzahl von Referenzfingerabdrücken (FP) beruhenden Referenzgruppenfingerabdrucks (FP) spezifiziert.

12. Das Verfahren nach einem der Ansprüche 1 bis 11, **dadurch gekennzeichnet, dass** der Bewertungsschritt einen erworbenen Bewertungs(MD)-Wert durch ein Berechnungsverfahren zur Qualitätstechnik (MT-Verfahren) unter Verwendung der Spitzenwerte des dem Referenzgruppenfingerabdruck (FP) als ein Merkmalswert zugeordneten Vorgabefingerabdrucks (FP) (33) erwirbt und den erworbenen Bewertungs(MD)-Wert durch den Vergleich mit einer Vielzahl von erworbenen Bewertungs(MD)-Werten, die Bewertungskriterien sind, bewertet.

13. Das Verfahren nach einem der Ansprüche 1 bis 12, **dadurch gekennzeichnet, dass** ein Referenzfingerabdruck(FP)-Auswahlschritt den in dem Vorgabemuster-Erstellungsschritt verwendeten Referenzfingerabdruck (FP) aus der Vielzahl von Referenzfingerabdrücken (FPs) auswählt.

14. Das Verfahren nach Anspruch 13, **dadurch gekennzeichnet, dass** der Referenzfingerabdruck(FP)-Auswahlschritt die Retentionszeitpunkte und ein Erscheinungsmuster der Spitzenwerte des Vorgabefingerabdrucks (FP) (33) mit denjenigen sämtlicher Vielzahlen von Referenzfingerabdrücken (FP) vergleicht, um einen Referenzfingerabdruck (FP) als einen als Zuordnungsgegner geeigneten Vorgabefingerabdruck (FP) auszuwählen.

**15.** Das Verfahren nach Anspruch 14, **dadurch gekennzeichnet, dass** der Multikomponenten-Stoff ein Multikomponentenmedikament ist, das eines von einem Rohmedikament, einer Kombination von Rohmedikamenten, einem Extrakt davon und einer Pflanzenheilkunde-Medizin (KAMPO-Medizin) ist.

**16.** Ein Bewertungsprogramm zum Durchführen eines Bewertungsverfahrens für ein Muster nach Anspruch 1, welches einen Computer zur Durchführung von Funktionen veranlasst, die aufweisen:

eine Fingerabdruck(FP)-Erstellungsfunktion des Erstellens eines Vorgabefingerabdrucks (33), der Spitzenwerte (35, 37, 39, 41, 43, 45, 47, 49, 51, 53) und Retentionszeitpunkte der Spitzenwerte aufweist, welche von einem Chromatogramm eines Multikomponenten-Stoffes, der eine Bewertungsvorgabe ist, detektiert werden;
eine Spitzenwertmuster-Erstellungsfunktion, die konfiguriert ist, um
ein Spitzenwertmuster (87) für einen Zuordnungsvorgabespitzenwert (45) des Vorgabefingerabdrucks (33) zu erstellen, wobei das Spitzenwertmuster (87) durch die Retentionszeit und die Intensität von n+1 Spitzenwerten (43, 45, 47) konfiguriert ist, die n periphere Spitzenwerte (43, 47) umfassen, die auf wenigstens einer der Seiten an der Vorderseite und der Rückseite des Zuordnungsvorgabespitzenwerts (45) in einer Richtung einer Zeitachse angeordnet auftreten; und
Spitzenwertmuster (89, 91, 93, 95) für betreffende Zuordnungskandidatenspitzenwerte (65, 67, 69, 71), welche Spitzenwerte sind, die Unterschiede in dem Erscheinungszeitpunkt in Bezug auf den Zuordnungsvorgabespitzenwert (45) innerhalb eines vorgegebenen Bereichs haben, eines Referenzfingerabdrucks (55) zu erstellen, welcher dem Vorgabefingerabdruck (33) entspricht und Spitzenwerte (75, 59, 61, 63, 65, 67, 69, 71, 73, 75, 77) und Retentionszeitpunkte der Spitzenwerte aufweist, welche von einem Chromatogramm eines Multikomponenten-Stoffes bestimmt werden, welcher im Voraus als ein normales Produkt, das ein Bewertungskriterium ist, bestimmt wird, wobei jedes der Spitzenwertmuster (89, 91, 93 und 95) durch die Retentionszeit und die Intensität von n+1 Spitzenwerten (63, 65, 67; 65, 67, 69; 67, 69, 71; 69, 71, 73) konfiguriert ist, welche n periphere Spitzenwerte (63, 67; 65, 69; 67, 71; 69, 73) umfassen, welche auf wenigstens einer der Seiten an der Vorderseite und der Rückseite der betreffenden Zuordnungskandidatenspitzenwerte (65, 67, 69, 71) in der Richtung der Zeitachse angeordnet auftreten;
eine Spitzenwert-Zuordnungsfunktion, welche das Spitzenwertmuster (87) für den Zuordnungsvorgabespitzenwert (45) mit den Spitzenwertmustern (89, 91, 93, 95) für die Zuordnungskandidatenspitzenwerte (65, 67, 69, 71) vergleicht, um den Grad der Übereinstimmung zwischen dem Vorgabemuster für den Zuordnungsvorgabespitzenwert und den Spitzenwertmustern für die Zuordnungskandidatenspitzenwerte zu berechnen, um betreffende Spitzenwerte (45, 65) des Vorgabefingerabdrucks (33) und des Referenzfingerabdrucks (55) zu spezifizieren und zuzuordnen; und
eine Bewertungsfunktion des Bewertens der Spitzenwerte, die in dem Spitzenwert-Zuordnungsschritt durch den Vergleich mit Spitzenwerten einer Vielzahl von Referenzfingerabdrücken (55), die Bewertungskriterien sind, deren Spitzenwerte sich in einer Zeitreihe verändern, spezifiziert und zugeordnet werden.

**17.** Das Bewertungsprogramm für einen Multikomponenten-Stoff nach Anspruch 16, **dadurch gekennzeichnet, dass**

die Fingerabdruck(FP)-Erstellungsfunktion einen Vorgabefingerabdruck (FP) (33) erstellt, welcher Spitzenwerte, Retentionszeitpunkte und UV-Spektren der Spitzenwerte aufweist, welche von einem 3D-Chromatogramm eines Multikomponenten-Stoffes, der ein Bewertungskriterium ist, detektiert werden, und
die Spitzenwertmuster-Erstellungsfunktion individuelle Spitzenwertmuster für jeden Spitzenwert des Vorgabefingerabdruck (FP) (33) und einen Referenzfingerabdruck (FP) (55) erstellt, welcher dem Vorgabefingerabdruck (FP) entspricht und Spitzenwerte, Retentionszeitpunkte und UV-Spektren der Spitzenwerte aufweist, welche von einem 3D-Chromatogramm des Multikomponenten-Stoffes, der das Bewertungskriterium ist, detektiert werden, unter Verwendung von n+1 Spitzenwerten, die n Spitzenwerte umfassen, welche auf wenigstens einer der Seiten an der Vorderseite und der Rückseite jedes Spitzenwertes in der Richtung der Zeitachse angeordnet auftreten.

**18.** Das Bewertungsprogramm für einen Multikomponenten-Stoff nach Anspruch 17, **dadurch gekennzeichnet, dass** die Spitzenwert-Zuordnungsfunktion das Spezifizieren der Spitzenwerte unter Berücksichtigung des Vergleichs der UV-Spektren der Spitzenwerte des Vorgabefingerabdrucks (FP) (33) mit dem Referenzfingerabdruck (FP) (55) durchführt.

**19.** Das Bewertungsprogramm für einen Multikomponenten-Stoff nach Anspruch 18, **dadurch gekennzeichnet, dass** die Spitzenwert-Zuordnungsfunktion das Spezifizieren der Spitzenwerte auf der Grundlage eines Grades der Übereinstimmung der Spitzenwerte durchführt, welche durch das Synthetisieren eines Grades der Übereinstimmung der individuellen Spitzenwertmuster, welche aus sämtlichen die Spitzenwertmuster konfigurierenden Spitzenwerten und deren Retentionszeitpunkten berechnet werden, und eines Grades der Übereinstimmung der UV-Spektren berechnet werden.

20. Eine Bewertungsvorrichtung für ein Muster aufweisend:

ein Fingerabdruck(FP)-Erstellungsteil, der konfiguriert ist, um einen Vorgabefingerabdruck (33) zu erstellen, welcher Spitzenwerte (35, 37, 39, 41, 43, 45, 47, 49, 51, 53) und Retentionszeitpunkte der Spitzenwerte aufweist, welche von einem Chromatogramm eines Multikomponenten-Stoffes, der eine Bewertungsvorgabe ist, detektiert werden;

einen Spitzenwertmuster-Erstellungsteil, der konfiguriert ist, um

ein Spitzenwertmuster (87) für einen Zuordnungsvorgabespitzenwert (45) des Vorgabefingerabdrucks (33) zu erstellen, wobei das Spitzenwertmuster (87) durch die Retentionszeit und die Intensität von n+1 Spitzenwerten (43, 45, 47) konfiguriert ist, die n periphere Spitzenwerte (43, 47) umfassen, die auf wenigstens einer der Seiten an der Vorderseite und der Rückseite des Zuordnungsvorgabespitzenwerts (45) in einer Richtung einer Zeitachse angeordnet auftreten; und

Spitzenwertmuster (89, 91, 93, 95) für betreffende Zuordnungskandidatenspitzenwerte (65, 67, 69, 71), welche Spitzenwerte sind, die Unterschiede in dem Erscheinungszeitpunkt in Bezug auf den Zuordnungsvorgabespitzenwert (45) innerhalb eines vorgegebenen Bereichs haben, eines Referenzfingerabdrucks (55) zu erstellen, welcher dem Vorgabefingerabdruck (33) entspricht und Spitzenwerte (75, 59, 61, 63, 65, 67, 69, 71, 73, 75, 77) und Retentionszeitpunkte der Spitzenwerte aufweist, welche von einem Chromatogramm eines Multikomponenten-Stoffes bestimmt werden, welcher im Voraus als ein normales Produkt, das ein Bewertungskriterium ist, bestimmt wird, wobei jedes der Spitzenwertmuster (89, 91, 93 und 95) durch die Retentionszeit und die Intensität von n+1 Spitzenwerten (63, 65, 67; 65, 67, 69; 67, 69, 71; 69, 71, 73) konfiguriert ist, welche n periphere Spitzenwerte (63, 67; 65, 69; 67, 71; 69, 73) umfassen, welche auf wenigstens einer der Seiten an der Vorderseite und der Rückseite der betreffenden Zuordnungskandidatenspitzenwerte (65, 67, 69, 71) in der Richtung der Zeitachse angeordnet auftreten;

ein Spitzenwert-Zuordnungsteil, welcher konfiguriert ist, um das Spitzenwertmuster (87) für den Zuordnungsvorgabespitzenwert (45) mit den Spitzenwertmustern (89, 91, 93, 95) für die Zuordnungskandidatenspitzenwerte (65, 67, 69, 71) zu vergleichen, um den Grad der Übereinstimmung zwischen dem Vorgabemuster für den Zuordnungsvorgabespitzenwert und den Spitzenwertmustern für die Zuordnungskandidatenspitzenwerte zu berechnen, um betreffende Spitzenwerte (45, 65) des Vorgabefingerabdrucks (33) und des Referenzfingerabdrucks (55) zu spezifizieren und zuzuordnen; und

ein Bewertungsteil, der konfiguriert ist, um die Spitzenwerte zu bewerten, welche in dem Spitzenwert-Zuordnungsschritt durch den Vergleich mit Spitzenwerten einer Vielzahl von Referenzfingerabdrücken (55), die Bewertungskriterien sind, deren Spitzenwerte sich in einer Zeitreihe verändern, spezifiziert und zugeordnet werden.

21. Das Bewertungsprogramm für einen Multikomponenten-Stoff nach Anspruch 20, **dadurch gekennzeichnet, dass**

der Fingerabdruck(FP)-Erstellungsteil einen Vorgabefingerabdruck (FP) (33) erstellt, welcher Spitzenwerte, Retentionszeitpunkte und UV-Spektren der Spitzenwerte aufweist, welche von einem 3D-Chromatogramm eines Multikomponenten-Stoffes, der ein Bewertungskriterium ist, detektiert werden, und

der Spitzenwertmuster-Erstellungsteil individuelle Spitzenwertmuster für jeden Spitzenwert des Vorgabefingerabdruck (FP) (33) und einen Referenzfingerabdruck (FP) (55) erstellt, welcher dem Vorgabefingerabdruck (FP) entspricht und Spitzenwerte, Retentionszeitpunkte und UV-Spektren der Spitzenwerte aufweist, welche von einem 3D-Chromatogramm des Multikomponenten-Stoffes, der das Bewertungskriterium ist, detektiert werden, unter Verwendung von n+1 Spitzenwerten, die n Spitzenwerte umfassen, welche auf wenigstens einer der Seiten an der Vorderseite und der Rückseite jedes Spitzenwertes in der Richtung der Zeitachse angeordnet auftreten.

22. Das Bewertungsprogramm für einen Multikomponenten-Stoff nach Anspruch 21, **dadurch gekennzeichnet, dass** der Spitzenwert-Zuordnungsteil (9) das Spezifizieren der Spitzenwerte unter Berücksichtigung des Vergleichs der UV-Spektren der Spitzenwerte des Vorgabefingerabdrucks (FP) mit dem Referenzfingerabdruck (FP) durchführt.

23. Das Bewertungsprogramm für einen Multikomponenten-Stoff nach Anspruch 22, **dadurch gekennzeichnet, dass** der Spitzenwert-Zuordnungsteil (9) das Spezifizieren der Spitzenwerte auf der Grundlage eines Grades der Übereinstimmung der Spitzenwerte durchführt, welche durch das Synthetisieren eines Grades der Übereinstimmung der individuellen Spitzenwertmuster, welche aus sämtlichen die Spitzenwertmuster konfigurierenden Spitzenwerten und deren Retentionszeitpunkten berechnet werden, und eines Grades der Übereinstimmung der UV-Spektren berechnet werden.

**Revendications**

1.  Procédé d'évaluation d'un motif comprenant :

    une étape de préparation d'empreinte digitale (FP) préparant une empreinte digitale cible (33) qui comprend des pics (35, 37, 39, 41, 43, 45, 47, 49, 51, 53) et des points de temps de rétention des pics détectés à partir d'un chromatogramme d'un matériau à plusieurs composants qui est une cible d'évaluation ;
    une étape de préparation de motif de pic configurée pour
    préparer un motif de pic (87) pour un pic cible d'attribution (45) de l'empreinte digitale cible (33), le motif de pic (87) étant configuré par le temps de rétention et l'intensité de n+1 pics (43, 45, 47) qui incluent n pics périphériques (43, 47) étant présents sur au moins l'un des côtés situés à l'avant et à l'arrière du pic cible d'attribution (45) dans un sens de l'axe temporel ; et
    préparer des motifs de pic (89, 91, 93, 95) pour des pics candidats d'attribution (65, 67, 69, 71) respectifs qui sont des pics ayant des différences de point de temps d'aspect par rapport au pic cible d'attribution (45) au sein d'une plage définie, d'une empreinte digitale de référence (55) qui correspond à l'empreinte digitale cible (33) et comprend des pics (57, 59, 61, 63, 65, 67, 69, 71, 73, 75, 77) et des points de temps de rétention des pics détectés à partir d'un chromatogramme d'un matériau à plusieurs composants déterminé à l'avance comme un produit normal qui est le critère d'évaluation, chacun des motifs de pic (89, 91, 93 et 95) étant configuré par le temps de rétention et l'intensité de n+1 pics (63, 65, 67 ; 65, 67, 69 ; 67, 69, 71 ; 69, 71, 73) qui incluent n pics périphériques (63, 67 ; 65, 69 ; 67, 71 ; 69, 73) étant présents sur au moins l'un des côtés situés à l'avant et à l'arrière du pic candidat d'attribution (65, 67, 69, 71) correspondant dans le sens de l'axe temporel ;
    une étape d'attribution de pic comparant le motif de pic (87) pour le pic cible d'attribution (45) avec les motifs de pics (89, 91, 93, 95) pour les pics candidats d'attribution (65, 67, 69, 71) pour calculer le degré de correspondance entre le motif de pic pour le pic cible d'attribution et les motifs de pics pour les pics candidats d'attribution, pour spécifier et attribuer les pics (45, 65) correspondants de l'empreinte digitale cible (33) et de l'empreinte digitale de référence (55) ; et
    une étape d'évaluation évaluant les pics qui sont spécifiés et attribués dans l'étape d'attribution de pics par comparaison avec les pics d'une pluralité d'empreintes digitales de référence (55) qui sont des critères d'évaluation dont les pics changent dans une série temporelle.

2.  Procédé selon la revendication 1, **caractérisé en ce que**

    l'étape de préparation de l'empreinte digitale (FP) prépare une empreinte digitale (FP) cible (33) qui comprend des pics, des points de temps de rétention et des spectres UV des pics détectés à partir d'un chromatogramme 3D du matériau à plusieurs composants qui est la cible d'évaluation, et
    l'étape de préparation de motifs de pics prépare des motifs de pics individuels, pour chaque pic, de l'empreinte digitale (FP) cible (33) et d'une empreinte digitale (FP) de référence (55) qui correspond à l'empreinte digitale (FP) cible et comprend des pics, des points de temps de rétention et des spectres UV des pics détectés à partir d'un chromatogramme 3D du matériau à plusieurs composants qui est le critère d'évaluation, avec l'utilisation de n + 1 pics qui incluent n pics étant présents sur au moins l'un des côtés situés à l'avant et à l'arrière de chaque pic dans un sens de l'axe temporel.

3.  Procédé selon la revendication 1 ou 2, **caractérisé en ce que**
    l'étape de préparation de l'empreinte digitale (FP) prépare l'empreinte digitale (FP) cible (33) en composant une pluralité d'empreintes digitales (FP) qui sont détectées à différentes longueurs d'ondes de détection à partir du chromatogramme ou du chromatogramme 3D de la cible d'évaluation.

4.  Procédé selon l'une quelconque des revendications 1 à 3, **caractérisé en ce que**
    l'étape de préparation de l'empreinte digitale (FP) extrait tous les pics du chromatogramme ou du chromatogramme 3D de la cible d'évaluation pour préparer l'empreinte digitale (FP) cible (33).

5.  Procédé selon l'une quelconque des revendications 1 à 4, **caractérisé en ce que**
    l'étape de préparation de motifs de pics prépare globalement des motifs de pics pour à la fois un pic étant une cible d'attribution de l'empreinte digitale (FP) cible (33) et un pic étant un candidat d'attribution de l'empreinte digitale (FP) de référence (55) en changeant les pics configurant les motifs de pics.

6. Procédé selon l'une quelconque des revendications 1 à 5, **caractérisé en ce que**
l'étape de préparation de motifs de pics prépare les motifs de pics pour tous les pics de l'empreinte digitale (FP) cible (33) et de l'empreinte digitale (FP) de référence (55).

7. Procédé selon l'une quelconque des revendications 1 à 6, **caractérisé en ce que**
l'étape d'attribution de pics réalise la spécification des pics sur la base d'un degré de correspondance entre les motifs de pics individuels calculé à partir de tous les pics configurant les motifs de pics et des points de temps de rétention de ceux-ci.

8. Procédé selon la revendication 2, **caractérisé en ce que**
l'étape d'attribution de pics réalise la spécification des pics eu égard à la comparaison entre les spectres UV des pics de l'empreinte digitale (FP) cible (33) et de l'empreinte digitale (FP) de référence (55).

9. Procédé selon la revendication 8, **caractérisé en ce que**
l'étape d'attribution de pics réalise la spécification des pics sur la base d'un degré de correspondance des pics calculé en synthétisant un degré de correspondance entre les motifs de pics individuels calculé à partir de tous les pics configurant les motifs de pics et les points de temps de rétention de ceux-ci et un degré de correspondance entre les spectres UV.

10. Procédé selon l'une quelconque des revendications 1 à 9, **caractérisé en ce que**
l'étape d'attribution de pics réalise la spécification par rapport à tous les pics de l'empreinte digitale (FP) cible (33) et de l'empreinte digitale (FP) de référence (55).

11. Procédé selon l'une quelconque des revendications 1 à 10, **caractérisé en ce que**
l'étape d'attribution de pics spécifie les pics de l'empreinte digitale (FP) cible (33) en associant les pics de l'empreinte digitale (FP) cible attribués à l'empreinte digitale (FP) de référence (55) à des pics respectifs d'une empreinte digitale (FP) de groupe de référence qui est basée sur la pluralité d'empreintes digitales (FP) de référence.

12. Procédé selon l'une quelconque des revendications 1 à 11, **caractérisé en ce que**
l'étape d'évaluation acquiert une valeur d'évaluation acquise (MD) par un procédé de calcul pour l'ingénierie de la qualité (procédé MT) avec utilisation des pics de l'empreinte digitale (FP) cible (33) attribués à l'empreinte digitale (FP) de groupe de référence an tant que valeur caractéristique et évalue la valeur d'évaluation acquise (MD) par comparaison avec une pluralité de valeurs d'évaluation (MD) acquises qui sont des critères d'évaluation.

13. Procédé selon l'une quelconque des revendications 1 à 12, **caractérisé en ce que**
une étape de sélection d'empreinte digitale (FP) de référence sélectionne l'empreinte digitale (FP) de référence qui est utilisée dans l'étape de préparation de motifs de pics parmi la pluralité d'empreintes digitales (FP) de référence.

14. Procédé selon la revendication 13 **caractérisé en ce que**
l'étape de sélection d'empreinte digitale (FP) de référence compare les points de temps de rétention et un motif d'aspect des pics de l'empreinte digitale (FP) cible (33) à ceux de l'ensemble de la pluralité d'empreintes digitales (FP) de référence pour sélectionner une empreinte digitale (FP) de référence qui est appropriée en tant qu'opposant d'attribution de l'empreinte digitale (FP) cible.

15. Procédé selon l'une quelconque des revendications 1 à 14, **caractérisé en ce que**
le matériau à plusieurs composants est un médicament à plusieurs composants qui est l'un d'un médicament brut, d'une combinaison de médicaments bruts, d'un extrait de ceux-ci et d'un médicament Kampo.

16. Programme d'évaluation pour réaliser un procédé d'évaluation d'un motif selon la revendication 1 qui amène un ordinateur à exécuter des fonctions comprenant :

une fonction de préparation d'empreinte digitale (FP) préparant une empreinte digitale cible (33) qui comprend des pics (35, 37, 39, 41, 43, 45, 47, 49, 51, 53) et des points de temps de rétention des pics détectés à partir d'un chromatogramme d'un matériau à plusieurs composants qui est une cible d'évaluation ;
une fonction de préparation de motif de pics configurée pour
préparer un motif de pics (87) pour un pic cible d'attribution (45) de l'empreinte digitale cible (33), le motif de pics (87) étant configuré par le temps de rétention et l'intensité de n+1 pics (43, 45, 47) qui incluent n pics périphériques (43, 47) étant présents sur au moins l'un des côtés situés à l'avant et à l'arrière du pic cible d'attribution (45) dans un sens de l'axe temporel ; et

préparer des motifs de pics (89, 91, 93, 95) pour des pics candidats d'attribution (65, 67, 69, 71) respectifs, qui sont des pics ayant des différences de point de temps d'aspect par rapport au pic cible d'attribution (45) au sein d'une plage définie, d'une empreinte digitale de référence (55) qui correspond à l'empreinte digitale cible (33) et comprend des pics (57, 59, 61, 63, 65, 67, 69, 71, 73, 75, 77) et des points de temps de rétention des pics détectés à partir d'un chromatogramme d'un matériau à plusieurs composants déterminé à l'avance comme un produit normal qui est un critère d'évaluation, chacun des motifs de pics (89, 91, 93 et 95) étant configuré par un temps de rétention et une intensité de n+1 pics (63, 65, 67 ; 65, 67, 69 ; 67, 69, 71 ; 69, 71, 73) qui incluent n pics périphériques (63, 67 ; 65, 69 ; 67, 71 ; 69, 73) étant présent sur au moins l'un des côtés situés à l'avant et à l'arrière du pic candidat d'attribution (65, 67, 69, 71) correspondant dans un sens de l'axe temporel ;

une fonction d'attribution de pic comparant le motif de pic (87) pour le pic cible d'attribution (45) aux motifs de pics (89, 91, 93, 95) pour les pics candidats d'attribution (65, 67, 69, 71) pour calculer le degré de correspondance entre le motif de pic pour le pic cible d'attribution et les motifs de pics pour les pics candidats d'attribution, pour spécifier et attribuer des pics (45, 65) correspondants de l'empreinte digitale cible (33) et de l'empreinte digitale de référence (55) ; et

une fonction d'évaluation évaluant les pics qui sont spécifiés et attribués dans la fonction d'attribution de pic par comparaison avec des pics d'une pluralité d'empreintes digitales de référence (55) qui sont des critères d'évaluation dont les pics changent dans une série temporelle.

17. Programme d'évaluation d'un matériau à plusieurs composants selon la revendication 16, **caractérisé en ce que**

la fonction de préparation de l'empreinte digitale (FP) prépare une empreinte digitale (FP) cible (33) qui comprend des pics, des points de temps de rétention et des spectres UV des pics détectés à partir d'un chromatogramme 3D du matériau à plusieurs composants qui est la cible d'évaluation, et

la fonction de préparation de motifs de pics prépare des motifs de pics individuels, pour chaque pic, de l'empreinte digitale (FP) cible (33) et d'une empreinte digitale de référence (FP) (55) qui correspond à l'empreinte digitale (FP) cible et comprend des pics, des points de temps de rétention et des spectres UV des pics détectés à partir d'un chromatogramme 3D du matériau à plusieurs composants qui est le critère d'évaluation, avec l'utilisation de n+1 pics qui incluent n pics étant présents sur au moins l'un des côtés situés à l'avant et à l'arrière de chaque pic dans un sens de l'axe temporel.

18. Programme d'évaluation d'un matériau à plusieurs composants selon la revendication 17, **caractérisé en ce que** la fonction d'attribution de pics réalise la spécification des pics eu égard à la comparaison entre les spectres UV des pics de l'empreinte digitale (FP) cible (33) et de l'empreinte digitale (FP) de référence (55).

19. Programme d'évaluation d'un matériau à plusieurs composants selon la revendication 18, **caractérisé en ce que** la fonction d'attribution de pics réalise la spécification des pics sur la base d'un degré de correspondance des pics calculé en synthétisant un degré de correspondance entre les motifs de pics individuels calculé à partir de l'ensemble des pics configurant les motifs de pics et les points de temps de rétention de ceux-ci et d'un degré de correspondance entre les spectres UV.

20. Appareil d'évaluation d'un motif comprenant :

une partie préparation d'empreinte digitale (FP) configurée pour préparer une empreinte digitale (FP) cible (33) qui comprend des pics (35, 37, 39, 41, 43, 45, 47, 49, 51, 53) et des points de temps de rétention des pics détectés à partir d'un chromatogramme d'un matériau à plusieurs composants qui est une cible d'évaluation ;

une partie préparation de motif de pics configurée pour

préparer un motif de pic (87) pour un pic cible d'attribution (45) de l'empreinte digitale cible (33), le motif de pic (87) étant configuré par le temps de rétention et l'intensité de n+1 pics (43, 45, 47) qui incluent n pics périphériques (43, 47) étant présents sur au moins l'un des côtés situés à l'avant et à l'arrière du pic cible d'attribution (45) dans un sens de l'axe temporel ; et

préparer des motifs de pics (89, 91, 93, 95) pour des pics candidats d'attribution (65, 67, 69, 71) respectifs, qui sont des pics ayant des différences de point de temps d'aspect par rapport au pic cible d'attribution (45) au sein d'une plage définie, d'une empreinte digitale de référence (55) qui correspond à l'empreinte digitale cible (33) et comprend des pics (57, 59, 61, 63, 65, 67, 69, 71, 73, 75, 77) et des points de temps de rétention des pics détectés à partir d'un chromatogramme d'un matériau à plusieurs composants déterminé à l'avance comme un produit normal qui est le critère d'évaluation, chacun des motifs de pics (89, 91, 93 et 95) étant configuré par le temps de rétention et l'intensité de n+1 pics (63, 65, 67 ; 65, 67, 69 ; 67, 69, 71 ; 69, 71, 73) qui incluent n pics périphériques (63, 67 ; 65, 69 ; 67, 71 ; 69, 73) étant présents sur au moins l'un des côtés situés à l'avant et à l'arrière du pic candidat d'attribution (65, 67, 69, 71) correspondant dans le sens de l'axe temporel ;

une partie attribution de pics configurée pour comparer le motif de pic (87) pour le pic cible d'attribution (45) aux motifs de pics (89, 91, 93, 95) pour les pics candidats d'attribution (65, 67, 69, 71) pour calculer le degré de correspondance entre le motif de pic pour le pic cible d'attribution et les motifs de pics pour les pics candidats d'attribution, pour spécifier et attribuer des pics (45, 65) correspondants de l'empreinte digitale cible (33) et de l'empreinte digitale de référence (55) ; et

une partie évaluation configurée pour évaluer les pics qui sont spécifiés et attribués dans l'étape d'attribution de pics par comparaison avec des pics d'une pluralité d'empreintes digitales de référence (55) qui sont des critères d'évaluation dont les pics changent dans une série temporelle.

21. Appareil d'évaluation d'un matériau à plusieurs composants selon la revendication 20, **caractérisé en ce que**

la partie préparation de l'empreinte digitale (FP) (3) prépare une empreinte digitale (FP) cible qui comprend des pics, des points de temps de rétention et des spectres UV des pics détectés à partir d'un chromatogramme du matériau à plusieurs composants qui est la cible d'évaluation, et

la partie préparation de motifs de pics (7) prépare des motifs de pics individuels, pour chaque pic, de l'empreinte digitale (FP) cible et d'une empreinte digitale (FP) de référence qui correspond à l'empreinte digitale (FP) cible et comprend des pics, des points de temps de rétention et des spectres UV des pics détectés à partir d'un chromatogramme 3D du matériau à plusieurs composants qui est le critère d'évaluation, avec l'utilisation de n+1 pics qui incluent n pics étant présents sur au moins l'un des côtés situés à l'avant et à l'arrière de chaque pic dans un sens de l'axe temporel.

22. Appareil d'évaluation d'un matériau à plusieurs composants selon la revendication 21, **caractérisé en ce que**
la partie attribution de pics (9) réalise la spécification des pics eu égard à la comparaison entre les spectres UV des pics de l'empreinte digitale (FP) cible et de l'empreinte digitale (FP) de référence.

23. Appareil d'évaluation d'un matériau à plusieurs composants selon la revendication 22, **caractérisé en ce que**
la partie attribution de pics (9) réalise la spécification des pics sur la base d'un degré de correspondance des pics calculé en synthétisant un degré de correspondance entre les motifs respectifs calculé à partir de l'ensemble de pics configurant les motifs de pics et les points de temps de rétention de ceux-ci et un degré de correspondance entre les spectres UV.

[FIG.1]

1

EVALUATING APPARATUS FOR A MULTICOMPONENT DRUG

FP PREPARING PART — 3

REFERENCE FP SELECTING PART — 5

PEAK PATTERN PREPARING PART — 7

PEAK ASSIGNING PART — 9

EVALUATING PART — 11

[FIG.2]

[FIG.3]

[FIG.4]

(A) DRUG A

(B) DRUG B

(C) DRUG C

[FIG.5]

[FIG.6]

TARGET FP RETENTION TIME APPEARANCE PATTERN

|      | 10.2 | 10.5 | 10.8 | 11.1 | 11.6 | 12.1 | 12.8 | 13.1 | 13.6 | 14.0 |
|------|------|------|------|------|------|------|------|------|------|------|
| 10.2 | 0.0  | 0.3  | 0.6  | 0.9  | 1.4  | 1.9  | 2.6  | 2.9  | 3.4  | 3.8  |
| 10.5 |      | 0.0  | 0.3  | 0.6  | 1.1  | 1.6  | 2.3  | 2.6  | 3.1  | 3.5  |
| 10.8 |      |      | 0.0  | 0.3  | 0.8  | 1.3  | 2.0  | 2.3  | 2.8  | 3.2  |
| 11.1 |      |      |      | 0.0  | 0.5  | 1.0  | 1.7  | 2.0  | 2.5  | 2.9  |
| 11.6 |      |      |      |      | 0.0  | 0.5  | 1.2  | 1.5  | 2.0  | 2.4  |
| 12.1 |      |      |      |      |      | 0.0  | 0.7  | 1.0  | 1.5  | 1.9  |
| 12.8 |      |      |      |      |      |      | 0.0  | 0.3  | 0.8  | 1.2  |
| 13.1 |      |      |      |      |      |      |      | 0.0  | 0.5  | 0.9  |
| 13.6 |      |      |      |      |      |      |      |      | 0.0  | 0.4  |
| 14.0 |      |      |      |      |      |      |      |      |      | 0.0  |

[FIG.7]

Reference FP retention time chart with labeled peaks 55, 57, 59, 61, 63, 65, 67, 69, 71, 73, 75, 77 at retention times 10.1, 10.4, 10.7, 11.1, 11.7, 12.3, 12.7, 13.1, 13.6, 14.1, 14.4

REFERENCE FP RETENTION TIME APPEARANCE PATTERN

| | 10.1 | 10.4 | 10.7 | 11.1 | 11.7 | 12.3 | 12.7 | 13.1 | 13.6 | 14.1 | 14.4 |
|------|------|------|------|------|------|------|------|------|------|------|------|
| 10.1 | 0.0 | 0.3 | 0.6 | 1.0 | 1.6 | 2.2 | 2.6 | 3.0 | 3.5 | 4.0 | 4.3 |
| 10.4 | | 0.0 | 0.3 | 0.7 | 1.3 | 1.9 | 2.3 | 2.7 | 3.2 | 3.7 | 4.0 |
| 10.7 | | | 0.0 | 0.4 | 1.0 | 1.6 | 2.0 | 2.4 | 2.9 | 3.4 | 3.7 |
| 11.1 | | | | 0.0 | 0.6 | 1.2 | 1.6 | 2.0 | 2.5 | 3.0 | 3.3 |
| 11.7 | | | | | 0.0 | 0.6 | 1.0 | 1.4 | 1.9 | 2.4 | 2.7 |
| 12.3 | | | | | | 0.0 | 0.4 | 0.8 | 1.3 | 1.8 | 2.1 |
| 12.7 | | | | | | | 0.0 | 0.4 | 0.9 | 1.4 | 1.7 |
| 13.1 | | | | | | | | 0.0 | 0.5 | 1.0 | 1.3 |
| 13.6 | | | | | | | | | 0.0 | 0.5 | 0.8 |
| 14.1 | | | | | | | | | | 0.0 | 0.3 |
| 14.4 | | | | | | | | | | | 0.0 |

81

[FIG.8]

**83**

NUMBER OF MATCHES IN RETENTION TIME POINT APPEARANCE DISTANCE

| | | REFERENCE FP RETENTION TIME APPEARANCE PATTERN | | | | | | | | | |
| --- | --- | --- | --- | --- | --- | --- | --- | --- | --- | --- | --- |
| | | 1ROW | 2ROW | 3ROW | 4ROW | 5ROW | 6ROW | 7ROW | 8ROW | 9ROW | 10ROW |
| TARGET FP RETENTION TIME APPEARANCE PATTERN | 1ROW | ⑦ | ⑦ | 7 | 6 | 6 | 5 | 4 | 4 | 3 | 2 |
| | 2ROW | 9 | 6 | 6 | 6 | 5 | 2 | 3 | 3 | 2 | 2 |
| | 3ROW | 3 | 8 | 5 | 4 | 5 | 5 | 4 | 2 | 2 | 2 |
| | 4ROW | 6 | 2 | 7 | 6 | 5 | 4 | 4 | 3 | 2 | 1 |
| | 5ROW | 3 | 4 | 5 | 6 | 5 | 4 | 3 | 3 | 2 | 1 |
| | 6ROW | 4 | 3 | 4 | 4 | 5 | 3 | 3 | 2 | 2 | 1 |
| | 7ROW | 2 | 4 | 2 | 2 | 1 | 4 | 3 | 2 | 2 | 2 |
| | 8ROW | 3 | 1 | 3 | 2 | 3 | 3 | 3 | 3 | 3 | 1 |
| | 9ROW | 2 | 2 | 2 | 1 | 1 | 2 | 2 | 2 | 2 | 2 |

[FIG.9]

**85**

DEGREE OF MATCHING IN RETENTION TIME APPEARANCE PATTERN

| | | REFERENCE FP RETENTION TIME APPEARANCE PATTERN | | | | | | | | | |
| --- | --- | --- | --- | --- | --- | --- | --- | --- | --- | --- | --- |
| | | 1ROW | 2ROW | 3ROW | 4ROW | 5ROW | 6ROW | 7ROW | 8ROW | 9ROW | 10ROW |
| TARGET FP RETENTION TIME APPEARANCE PATTERN | 1ROW | 2.00 | 2.00 | 2.00 | 3.00 | 3.00 | 4.13 | 5.35 | 5.35 | 6.67 | 8.05 |
| | 2ROW | ⓪.50 | 3.00 | 3.00 | 3.00 | 4.13 | 8.05 | 6.67 | 6.67 | 8.05 | 8.05 |
| | 3ROW | 6.67 | 1.15 | 4.13 | 5.35 | 4.13 | 4.13 | 5.35 | 8.05 | 8.05 | 8.05 |
| | 4ROW | 3.00 | 8.05 | 2.00 | 3.00 | 4.13 | 5.35 | 5.35 | 6.67 | 8.05 | 9.50 |
| | 5ROW | 6.67 | 5.35 | 4.13 | 3.00 | 4.13 | 5.35 | 6.67 | 6.67 | 8.05 | 9.50 |
| | 6ROW | 5.35 | 6.67 | 5.35 | 5.35 | 4.13 | 6.67 | 6.67 | 8.05 | 8.05 | 9.50 |
| | 7ROW | 8.05 | 5.35 | 8.05 | 8.05 | 9.50 | 5.35 | 6.67 | 8.05 | 8.05 | 8.05 |
| | 8ROW | 6.67 | 9.50 | 6.67 | 8.05 | 6.67 | 6.67 | 6.67 | 6.67 | 6.67 | 9.50 |
| | 9ROW | 8.05 | 8.05 | 8.05 | 9.50 | 9.50 | 8.05 | 8.05 | 8.05 | 8.05 | 8.05 |

DEGREE OF MATCHING BETWEEN RETENTION TIME APPEARANCE PATTERNS =
(1 − (NUMBER OF MATCHES IN APPEARANCE DISTANCE/(NUMBER OF PEAKS OF TARGET FP + NUMBER OF PEAKS OF REFERENCE FP − NUMBER OF MATCHES IN APPEARANCE DISTANCE)) × (NUMBER OF PEAKS OF TARGET FP − NUMBER OF MATCHES IN APPEARANCE DISTANCE + 1)

[FIG.10]

[FIG.11]

[FIG.12]

[FIG.13]

[FIG.14]

TARGET FP 33

35  37  39  41  43  45  47  49  51  53

ASSIGNMENT TARGET PEAK

10.2  10.5  10.8  11.1  11.6  12.1  12.8  13.1  13.6  14.0

67

ALLOWABLE RANGE

REFERENCE FP 55

57  59  61  63  65  69  71  73  75  77

10.1  10.4  10.7  11.1  11.7  12.3  12.7  13.1  13.6  14.1  14.4

ASSIGNMENT CANDIDATE PEAKS

[FIG.15]

TARGET FP 33

87  41  35  37  39  43  45  47  49  51  53

10.2  10.5  10.8  11.1  11.6  12.1  12.8  13.1  13.6  14.0

89  67

REFERENCE FP 55

61  57  59  63  65  69  71  73  75  77

10.1  10.4  10.7  11.1  11.7  12.3  12.7  13.1  13.6  14.1  14.4

[FIG.16]

[FIG.17]

[FIG.18]

[FIG.19]

[FIG.20]

[FIG.21]

[FIG.22]

[FIG.23]

[FIG.24]

PREPARE PEAK PATTERN WITH USE OF THREE PEAKS INCLUDING
ARBITRARY TWO OF FOUR PEAK PATTERN CONFIGURING CANDIDATE PEAKS

$$_4C_2 = 6 \text{ PATTERNS}$$

[FIG.25]

PREPARE PEAK PATTERN WITH USE OF THREE PEAKS INCLUDING
ARBITRARY TWO OF FOUR PEAK PATTERN CONFIGURING CANDIDATE PEAKS

$$_4C_2 = 6 \text{ PATTERNS}$$

[FIG.26]

## COMPARISON OF COMPREHENSIVE PEAK PATTERNS 45 → 65 (1/36)

[FIG.27]

## COMPARISON OF COMPREHENSIVE PEAK PATTERNS 45 → 65 (2/36)

[FIG.28]

## COMPARISON OF COMPREHENSIVE PEAK PATTERNS 45 → 65 (3/36)

[FIG.29]

## COMPARISON OF COMPREHENSIVE PEAK PATTERNS 45 → 65 (4/36)

[FIG.30]

**COMPARISON OF COMPREHENSIVE PEAK PATTERNS 45 → 65 (5/36)**

[FIG.31]

**COMPARISON OF COMPREHENSIVE PEAK PATTERNS 45 → 65 (6/36)**

[FIG.32]

COMPARISON OF COMPREHENSIVE PEAK PATTERNS 45 → 65 (7/36)

[FIG.33]

COMPARISON OF COMPREHENSIVE PEAK PATTERNS 45 → 65 (8/36)

[FIG.34]

**COMPARISON OF COMPREHENSIVE PEAK PATTERNS 45 → 65 (9/36)**

[FIG.35]

**COMPARISON OF COMPREHENSIVE PEAK PATTERNS 45 → 65 (10/36)**

[FIG.36]

## COMPARISON OF COMPREHENSIVE PEAK PATTERNS 45 → 65 (11/36)

[FIG.37]

## COMPARISON OF COMPREHENSIVE PEAK PATTERNS 45 → 65 (12/36)

[FIG.38]

**COMPARISON OF COMPREHENSIVE PEAK PATTERNS 45 → 65 (13/36)**

[FIG.39]

**COMPARISON OF COMPREHENSIVE PEAK PATTERNS 45 → 65 (14/36)**

[FIG.40]

## COMPARISON OF COMPREHENSIVE PEAK PATTERNS 45 → 65 (15/36)

[FIG.41]

## COMPARISON OF COMPREHENSIVE PEAK PATTERNS 45 → 65 (16/36)

[FIG.42]

## COMPARISON OF COMPREHENSIVE PEAK PATTERNS 45 → 65 (17/36)

[FIG.43]

## COMPARISON OF COMPREHENSIVE PEAK PATTERNS 45 → 65 (18/36)

[FIG.44]

COMPARISON OF COMPREHENSIVE PEAK PATTERNS 45 → 65 (19/36)

[FIG.45]

COMPARISON OF COMPREHENSIVE PEAK PATTERNS 45 → 65 (20/36)

## [FIG.46]

**COMPARISON OF COMPREHENSIVE PEAK PATTERNS 45 → 65 (21/36)**

## [FIG.47]

**COMPARISON OF COMPREHENSIVE PEAK PATTERNS 45 → 65 (22/36)**

[FIG.48]

## COMPARISON OF COMPREHENSIVE PEAK PATTERNS 45 → 65 (23/36)

[FIG.49]

## COMPARISON OF COMPREHENSIVE PEAK PATTERNS 45 → 65 (24/36)

[FIG.50]

## COMPARISON OF COMPREHENSIVE PEAK PATTERNS 45 → 65 (25/36)

[FIG.51]

## COMPARISON OF COMPREHENSIVE PEAK PATTERNS 45 → 65 (26/36)

[FIG.52]

## COMPARISON OF COMPREHENSIVE PEAK PATTERNS 45 → 65 (27/36)

[FIG.53]

## COMPARISON OF COMPREHENSIVE PEAK PATTERNS 45 → 65 (28/36)

[FIG.54]

**COMPARISON OF COMPREHENSIVE PEAK PATTERNS 45 → 65 (29/36)**

[FIG.55]

**COMPARISON OF COMPREHENSIVE PEAK PATTERNS 45 → 65 (30/36)**

[FIG.56]

## COMPARISON OF COMPREHENSIVE PEAK PATTERNS 45 → 65 (31/36)

[FIG.57]

## COMPARISON OF COMPREHENSIVE PEAK PATTERNS 45 → 65 (32/36)

[FIG.58]

**COMPARISON OF COMPREHENSIVE PEAK PATTERNS 45 → 65 (33/36)**

[FIG.59]

**COMPARISON OF COMPREHENSIVE PEAK PATTERNS 45 → 65 (34/36)**

[FIG.60]

## COMPARISON OF COMPREHENSIVE PEAK PATTERNS 45 → 65 (35/36)

[FIG.61]

## COMPARISON OF COMPREHENSIVE PEAK PATTERNS 45 → 65 (36/36)

[FIG.62]

[FIG.63]

$$P\_Sim(45-67) = (|p1-p2|+1) * (|(r1-(r2+d)|+1)$$
$$+ (|dn1-fn1|+1) * (|(cn1-r1) - (en1-r2)|+1)$$
$$+ (|dn2-fn2|+1) * (|(cn2-r1) - (en2-r2)|+1)$$

[FIG.64]

$$P\_Sim(45-67) = (|p1-p2|+1) * (|(r1-(r2+d)|+1)$$
$$+ (|dn1-fn1|+1) * (|(cn1-r1) - (en1-r2)|+1)$$
$$+ (|dn2-fn2|+1) * (|(cn2-r1) - (en2-r2)|+1)$$
$$+ (|dn3-fn3|+1) * (|(cn3-r1) - (en3-r2)|+1)$$
$$+ (|dn4-fn4|+1) * (|(cn4-r1) - (en4-r2)|+1)$$

[FIG.65]

UV SPECTRUM OF 45

UV SPECTRUM OF 67

[FIG.66]

$$UV\_Sim(45-67) = RMSD \ (107 \ vs \ 111)$$

[FIG.67]

SCORE(45-67) = P_Sim_min(45-67) * UV_Sim(45-67)

[FIG.68]

[FIG.69]

ASSIGNMENT RESULT OF TARGET FP

[FIG.70]

[FIG.71]

[FIG.72]

[FIG.73]

[FIG.74]

[FIG.75]

```
┌─────────────────────────────────────┐
│         FP PREPARING STEP            │——113
└─────────────────────────────────────┘
                  │
                  ▼
┌─────────────────────────────────────┐
│     REFERENCE FP SELECTING STEP      │——115
└─────────────────────────────────────┘
                  │
                  ▼
┌─────────────────────────────────────┐
│     PEAK PATTERN PREPARING STEP      │——117
└─────────────────────────────────────┘
                  │
                  ▼
┌─────────────────────────────────────┐
│        PEAK ASSIGNING STEP           │——119
└─────────────────────────────────────┘
                  │
                  ▼
┌─────────────────────────────────────┐
│         EVALUATING STEP              │——121
└─────────────────────────────────────┘
```

[FIG.76]

QUALITY EVALUATING SYSTEM FOR MULTICOMPONENT DRUG

## [FIG.77]

FP PREPARING PROCESS (CASE OF SINGLE WAVELENGTH)

[FIG.78]

FP PREPARING PROCESS (CASE OF PLURALITY OF WAVELENGTHS)

PEAK DATA AT EACH WAVELENGTH

UV220
UV210
UV200

3D CHROMATOGRAM DATA
PEAK INFORMATION

S1

FP PREPARING PROCESS

TARGET FP

START OF FP PREPARING PROCESS

PREPARE FP FOR EACH WAVELENGTH — S110

LIST FPs ACCORDING TO NUMBER OF PEAKS (DESCENDING ORDER) — S111

n ← 1 — S112

READ n-TH FP IN LIST — S113

ACQUIRE ALL RETENTION TIME POINTS (X) — S114

UPDATE n (n ← n+1) — S115

READ n-th FP IN LIST — S116

ACQUIRE ALL RETENTION TIME POINTS (Y) — S117

INTEGRATE X AND Y WITHOUT DUPLICATION (Z) — S118

UPDATE X (X←Z) — S119

S120

HAS PROCESS OF ALL FPs BEEN COMPLETED?  NO

YES

2

[FIG.79]

[FIG.80]

TARGET FP ASSIGNING PROCESS 1
(PREPROCESS: SELECTION OF REFERENCE FP)

START OF TARGET FP
ASSIGNING PROCESS 1

READ TARGET FP — S201

ACQUIRE ALL RETENTION
TIME POINTS (R1) — S202

TARGET FP / S2

LIST FILE NAMES OF ALL
REFERENCE FPs — S203

TARGET FP ASSIGNING PROCESS 1
(PREPROCESS: SELECTION OF
REFERENCE FP)

S204

n ← 1

TARGET FP ASSIGNING PROCESS 2
(MAIN PROCESS: CALCULATION OF
ASSIGNMENT SCORE)

S205

READ n–TH REFERENCE FP
IN LIST (REFERENCE FPn)

TARGET FP ASSIGNING PROCESS 3
(POST-PROCESS: SPECIFYING
CORRESPONDING PEAKS)

S206

ACQUIRE ALL RETENTION
TIME POINTS (R2)

TARGET FP ASSIGNING PROCESS 4
(FINAL PROCESS: ASSIGNMENT
TO REFERENCE GROUP FP)

S207

CALCULATE DEGREE OF
MATCHING BETWEEN RETENTION
TIME APPEARANCE PATTERNS
OF R1 AND R2 (RPn_min)

PEAK DATA
FEATURE VALUE FILE

S208

STORE RPn_min (RPall_min)

SUBROUTINE 1:
CALCULATION OF DEGREE
OF MATCHING BETWEEN
RETENTION TIME
APPEARANCE PATTERNS

S209

UPDATE n (n ← n+1)

S210

HAS PROCESS OF
ALL REFERENCE FPs
BEEN COMPLETED?    NO

YES

SELECT REFERENCE FP WITH
MINIMUM DEGREE OF
MATCHING FROM RPall_min — S211

END OF TARGET FP
ASSIGNING PROCESS 1

[FIG.81]

TARGET FP ASSIGNING PROCESS 2
(MAIN PROCESS: CALCULATION OF SCORE AGAINST ASSIGNMENT CANDIDATE PEAK)

/ TARGET FP /

S3

TARGET FP ASSIGNING PROCESS 1
(PREPROCESS: SELECTION OF
REFERENCE FP)

TARGET FP ASSIGNING PROCESS 2
(MAIN PROCESS: CALCULATION OF
ASSIGNMENT SCORE)

TARGET FP ASSIGNING PROCESS 3
(POST-PROCESS: SPECIFYING
CORRESPONDING PEAKS)

TARGET FP ASSIGNING PROCESS 4
(FINAL PROCESS: ASSIGNMENT
TO REFERENCE GROUP FP)

/ PEAK DATA
FEATURE VALUE FILE /

( START OF TARGET FP
ASSIGNING PROCESS 2 )

READ TARGET FP — S301

SEQUENTIALLY ACQUIRE
RETENTION TIME POINT
(R1), PEAK DATA (P1), AND
UV SPECTRUM (U1) OF
ASSIGNMENT TARGET PEAK — S302

READ REFERENCE FP — S303

SEQUENTIALLY ACQUIRE
RETENTION TIME POINT
(R2), PEAK DATA (P2), AND
UV SPECTRUM (U2) OF
PEAK OF REFERENCE FP — S304

S305

$|R1 - (R2 + d)| <$ THRESHOLD VALUE?

YES

S306
CALCULATE DEGREE
OF MATCHING
BETWEEN UV SPECTRA
(UV_Sim)

SUBROUTINE 2:
CALCULATION OF
DEGREE OF MATCHING
BETWEEN UV SPECTRA
(UV_Sim)

NO

S309

SUBSTITUTE "888888"
INTO SCORE
(SCORE ← 888888)

S307
CALCULATE DEGREE
OF MATCHING
BETWEEN PEAK PATTERNS
(P_Sim_min)

SUBROUTINE 3:
CALCULATION OF
DEGREE OF MATCHING
BETWEEN PEAK
PATTERNS (P_Sim_min)

S308
CALCULATE DEGREE
OF MATCHING OF
ASSIGNMENT CANDIDATE
PEAK (SCORE)

SCORE = UV_Sim × P_Sim_min

STORE SCORE (SCORE_all) — S310

S311

HAS PROCESS OF
ALL REFERENCE PEAKS BEEN
COMPLETED?

NO

YES

OUTPUT SCORE_all TO
DETERMINATION RESULT
FILE AND INITIALIZE
SCORE_all (VACATE) — S312

S313

HAS PROCESS OF
ALL TARGET PEAKS BEEN
COMPLETED?

NO

YES

( END OF TARGET FP
ASSIGNING PROCESS 2 )

## [FIG.82]

TARGET FP ASSIGNING PROCESS 3
(POST-PROCESS: SPECIFYING CORRESPONDING PEAKS FROM PLURAL CANDIDATE PEAKS)

[FIG.83]
PEAK ASSIGNING PROCESS 4
(FINAL PROCESS: ASSIGNING PROCESS TO REFERENCE GROUP FP)

TARGET FP

TARGET FP ASSIGNING PROCESS 1
(PREPROCESS: SELECTION OF
REFERENCE FP)

TARGET FP ASSIGNING PROCESS 2
(MAIN PROCESS: CALCULATION OF
ASSIGNMENT SCORE)

TARGET FP ASSIGNING PROCESS 3
(POST-PROCESS: SPECIFYING
CORRESPONDING PEAKS)

TARGET FP ASSIGNING PROCESS 4
(FINAL PROCESS: ASSIGNMENT
TO REFERENCE GROUP FP)

S5

PEAK DATA
FEATURE VALUE FILE

START

READ COLLATION
RESULT FILE — S501

READ REFERENCE
GROUP FP — S502

INTEGRATE AND STORE
TARGET FP AND REFERENCE
GROUP FP (TEMP) — S503

CORRECT RETENTION TIME
POINT OF ALL PEAKS OF
TARGET FP THAT DO NOT
CORRESPOND TO ANY PEAKS
IN REFERENCE FP — S504

SEQUENTIALLY ACQUIRE PEAK
DATA (P1) CORRESPONDING TO
CORRECTED RETENTION TIME
POINT (R1, R3) — S505

SEQUENTIALLY ACQUIRE PEAK (P2)
OF TARGET FP CORRESPONDING
TO RETENTION TIME POINT (R2)
OF ASSIGNMENT CANDIDATE PEAK
FROM TEMP — S506

S507
$|R1 - R2| <$ THRESHOLD VALUE 1?

YES

S508
ACQUIRED UV SPECTRA
CORRESPONDING TO
R1 AND R2 (U1, U2)

S509
CALCULATE DEGREE OF
MATCHING BETWEEN
UV SPECTRA (UV_Sim)

SUBROUTINE 2:
CALCULATION OF DEGREE
OF MATCHING BETWEEN
UV SPECTRA (UV_Sim)

S510
UV_Sim < THRESHOLD VALUE 2?

YES

NO

S511
R3 ← R2 AND THRESHOLD
VALUE 2 ← UN_Sim

NO

S512
HAS COMPARISON OF RETENTION
TIME POINT FOR ALL ASSIGNMENT CANDIDATE
PEAKS BEEN COMPLETED?

NO

YES

STORE R1, R3, P1 AND
THRESHOLD VALUE 2 (TEMP2) — S513

S514
HAS COMPARISON OF RETENTION
TIME POINT FOR ALL NON-CORRESPONDING
PEAKS BEEN COMPLETED?

NO

S515
THRESHOLD VALUE 2
← INITIAL VALUE

YES

3

[FIG.84]

```
                    ┌───┐
                    │ 3 │
                    └─┬─┘
                      │
             S516     ▼                          S517
                  ╱───────────╲      ┌─────────────────────────────────┐
                 ╱ ARE THERE   ╲     │ COMPARE THRESHOLD VALUES 2 OF PEAK│
                ╱  PEAK DATA     ╲YES │ DATA HAVING SAME VALUE OF R3 AND  │
               ╲ HAVING SAME VALUE OF╱│ RETURN R3 OF PEAK DATA HAVING     │
                ╲  R3 IN TEMP2? ╱     │ LARGER VALUE TO ORIGINAL VALUE(R1)│
                 ╲───────────╱        └─────────────────────────────────┘
                      │ NO                          │
                      ▼◄───────────────────────────┘
            ┌──────────────────────────┐
            │ ADD PEAK DATA OF TEMP2 TO │
            │ TEMP (ONLY PEAK DATA WHOSE│   S518
            │ R3 COINCIDES RETENTION    │
            │ TIME POINT OF TEMP)       │
            └──────────────────────────┘
                      │
                      ▼
            ┌──────────────────────────┐
            │ OUTPUT PEAK DATA OF TARGET│   S519
            │ FP INCLUDED IN TEMP (PEAK │
            │ DATA FEATURE VALUE FILE)  │
            └──────────────────────────┘
                      │
                      ▼
                 ┌─────────┐
                 │   END   │
                 └─────────┘
```

## [FIG.85]

SUBROUTINE 1 (CALCULATION OF DEGREE OF MATCHING BETWEEN RETENTION TIME APPEARANCE PATTERNS (RP))

START OF CALCULATING DEGREE OF MATCHING
BETWEEN RETENTION TIME APPEARANCE PATTERNS

$x \leftarrow R1, y \leftarrow R2$ — S1001

ACQUIRE NUMBER OF DATA PIECES OF "x" AND "y" (a, b) — S1002

$i \leftarrow 1$ — S1003

ACQUIRE ALL DISTANCES FROM $x_i$-TH RETENTION TIME POINT (f) — S1004

$j \leftarrow 1$ — S1005

ACQUIRE ALL DISTANCES FROM $y_j$-TH RETENTION TIME POINT (g) — S1006

ACQUIRE NUMBER OF DATA PIECES SATISFYING CONDITION OF
"|INTER-RETENTION TIME POINT DISTANCE OF "f" – INTER-RETENTION
TIME POINT DISTANCE OF "g"| < THRESHOLD VALUE" (m) — S1007

CALCULATE DEGREE OF MATCHING BETWEEN RETENTION TIME
APPEARANCE PATTERNS OF "f" AND "g" (RPfg) — S1008

$$RP_{fg} = (1 - (m / (a - b - m))) \times (a - m + 1)$$

STORE RPfg (RP_all) — S1009

UPDATE "j" (j ← j + 1) — S1010

S1011
HAS PROCESS OF ALL RETENTION TIME
POINTS OF "y" BEEN COMPLETED?　　NO

YES

UPDATE "i" (i ← i + 1) — S1012

S1013
HAS PROCESS OF ALL RETENTION TIME
POINTS OF "x" BEEN COMPLETED?　　NO

YES

ACQUIRE MINIMUM VALUE FROM RP_all (RP_min) — S1014

END OF CALCULATING DEGREE OF MATCHING
BETWEEN RETENTION TIME APPEARANCE PATTERNS

[FIG.86]

SUBROUTINE 2 (CALCULATION OF DEGREE OF MATCHING BETWEEN UV SPECTRA)

```
        ╭─────────────────────────────╮
       │   START OF CALCULATION DEGREE  │
       │  OF MATCHING BETWEEN UV SPECTRA │
        ╰─────────────────────────────╯
                      │
                      ▼                        S2001
       ╱──────────────────────────────╲
       ⟨      x ← U1, y ← U2, z ← 0      ⟩
       ╲──────────────────────────────╱
                      │
                      ▼
       ┌──────────────────────────────┐
       │ ACQUIRE NUMBER OF DATA PIECES  │      S2002
       │          OF "x" (a)            │
       └──────────────────────────────┘
                      │
                      ▼            S2003
              ⟨─────────────⟩
              ⟨    i ← 1     ⟩
              ⟨─────────────⟩
                      │                        S2004
                      ▼
       ┌──────────────────────────────┐  ◄───────────┐
       │    ACQUIRE xi-TH DATA (b)      │              │
       └──────────────────────────────┘              │
                      │                               │
                      ▼                        S2005  │
       ┌──────────────────────────────┐              │
       │    ACQUIRE yi-TH DATA (c)      │              │
       └──────────────────────────────┘              │
                      │                               │
                      ▼                        S2006  │
       ┌──────────────────────────────┐              │
       │ CALCULATE INTER-UV SPECTRUM    │   ┌──────────┐
       │ DISTANCE (d) AND SUM OF SQUARE │   │ d = b - c │
       │ OF INTER-UV SPECTRUM           │   │ z = z + d2│
       │ DISTANCE (z)                   │   └──────────┘
       └──────────────────────────────┘              │
                      │                               │
                      ▼                               │
       ┌──────────────────────────────┐              │
       │      UPDATE "i" (i ← i + 1)    │      S2007   │
       └──────────────────────────────┘              │
                      │           S2008               │
                      ▼                               │
            ╱───────────────────╲                     │
          ╱   HAS PROCESS OF ALL   ╲     NO            │
          ⟨   DATA OF "x" BEEN       ⟩───────────────┘
          ╲   COMPLETED?           ╱
            ╲───────────────────╱
                      │ YES
                      ▼                        S2009
       ┌──────────────────────────────┐
       │    CALCULATE DEGREE OF MATCHING│   ┌─────────────────────┐
       │ BETWEEN UV SPECTRA OF "x" AND  │   │ UV_Sim = √ ( z / a ) │
       │        "y" (UV_Sim)            │   └─────────────────────┘
       └──────────────────────────────┘
                      │
                      ▼
        ╭─────────────────────────────╮
       │    END OF CALCULATION DEGREE   │
       │  OF MATCHING BETWEEN UV SPECTRA │
        ╰─────────────────────────────╯
```

## [FIG.87]

SUBROUTINE 3
(CALCULATION OF DEGREE OF MATCHING BETWEEN PEAK PATTERNS)

START OF CALCULATION DEGREE OF
MATCHING BETWEEN PEAK PATTERNS — S3001

SET NUMBER OF PEAK PATTERN CONFIGURING
CANDIDATE PEAKS (m) AND NUMBER OF PEAK
PATTERN CONFIGURING PEAKS (n) — S3002

x ← TARGET FP, r1 ← R1, AND p1 ← P1
y ← REFERENCE FP, r2 ← R2, AND p2 ← P2

ACQUIRE ALL RETENTION TIME POINTS OF "x" (a) — S3003

ACQUIRE ALL RETENTION TIME POINTS OF "y" (b) — S3004

ACQUIRE RETENTION TIME POINTS (cm) AND PEAK DATA
PIECES (dm) OF m PEAK PATTERN CONFIGURING
CANDIDATE PEAKS OF "r1" FROM "a" — S3005

ACQUIRE RETENTION TIME POINTS (em) AND PEAK DATA
PIECES (fm) OF m PEAK PATTERN CONFIGURING
CANDIDATE PEAK OF "r2" FROM "b" — S3006

ALIGN "cm", "dm" IN RETENTION TIME ORDER
(ASCENDING ORDER) — S3007

ALIGN "em", "fm" IN RETENTION TIME ORDER
(ASCENDING ORDER) — S3008

SEQUENTIALLY ACQUIRE RETENTION TIME POINTS (cn) AND
PEAK DATA PIECES (dn) OF n PEAK PATTERN CONFIGURING
PEAK FROM "cm" AND "dm" — S3009

SEQUENTIALLY ACQUIRE RETENTION TIME POINTS (en) AND
PEAK DATA PIECES (fn) OF n PEAK PATTERN CONFIGURING
PEAK FROM "em" AND "fm" — S3010

CALCULATE DEGREE OF MATCHING
BETWEEN PEAK PATTERNS (P_Sim) — S3011

STORE P_Sim (P_Sim_all)

S3013 — HAVE ALL COMBINATIONS TO
TAKE OUT n PIECES FROM m PIECES
IN "em" BEEN COMPLETED? — NO — S3012

YES

S3014 — HAVE ALL COMBINATIONS TO
TAKE OUT n PIECES FROM m PIECES
IN "cm" BEEN COMPLETED? — NO

YES

ACQUIRE MINIMUM VALUE FROM P_Sim_all (P_Sim_min) — S3015

END OF CALCULATION DEGREE OF
MATCHING BETWEEN PEAK PATTERNS

IN CASE OF n = 2
$$P\_Sim = (|p1-p2|+1) \times (|(r1-(r2+d)|+1)$$
$$+ (|dn1-fn1| +1) \times (|(cn1-r1)-(en1-r2)|+1)$$
$$+ (|dn2-fn2| +1) \times (|(cn2-r1)-(en2-r2)|+1)$$

IN CASE OF n = 4
$$P\_Sim = (|p1-p2|+1) \times (|(r1-(r2+d)|+1)$$
$$+ (|dn1-fn1|+1) \times (|(cn1-r1)-(cn1-r2)|+1)$$
$$+ (|dn2-fn2|+1) \times (|(cn2-r1)-(en2-r2)|+1)$$
$$+ (|dn3-fn3|+1) \times (|(cn3-r1)-(en3-r2)|+1)$$
$$+ (|dn4-fn4|+1) \times (|(cn4-r1)-(en4-r2)|+1)$$

[FIG.88]

PREPARATION OF REFERENCE DATA (unit)
FOR EVALUATING QUALITY OF MULTICOMPONENT DRUG

## [FIG.89]

REFERENCE FP ASSIGNING RESULT INTEGRATING PROCESS
(PREPARATION OF REFERENCE FP CORRESPONDENCE TABLE)

EP 2 717 049 B1

[FIG.90]

(5)

CORRECT RETENTION TIME POINT OF ALL LACKING PEAKS — S10107

SEQUENTIALLY ACQUIRE PEAK DATA (P1) CORRESPONDING TO CORRECTED RETENTION TIME POINT (R1, R3) — S10108

SEQUENTIALLY ACQUIRE PEAK DATA (P2) CORRESPONDING TO RETENTION TIME POINT (R2) OF PEAK THAT IS ASSIGNMENT CANDIDATE FOR LACKING PEAK FROM INTEGRATED DATA — S10109

$|R1 - R2| <$ THRESHOLD VALUE 1? — S10110

YES → ACQUIRE UV SPECTRA CORRESPONDING TO R1 AND R2 (U1, U2) — S10111

CALCULATE DEGREE OF MATCHING BETWEEN UV SPECTRA (UV_Sim) — S10112

SUBROUTINE 2: CALCULATION OF DEGREE OF MATCHING BETWEEN UV SPECTRA (UV_Sim)

UV_Sim < THRESHOLD VALUE 2? — S10113

YES → R3 ← R2, THRESHOLD VALUE 2 ← UV_Sim — S10114

NO

S10115 HAS COMPARISON OF RETENTION TIME POINT FOR ALL ASSIGNMENT CANDIDATE PEAKS BEEN COMPLETED?

NO

YES

STORE R1, R3, P1 AND THRESHOLD VALUE 2 (TEMP2) — S10116

HAS COMPARISON OF RETENTION TIME POINT FOR ALL LACKING PEAKS BEEN COMPLETED? — S10117

NO → THRESHOLD VALUE 2 ← INITIAL VALUE — S10118

YES

ARE THERE PEAK DATA HAVING SAME VALUE OF R3 IN TEMP2? — S10119

YES → COMPARE THRESHOLD VALUES 2 OF PEAK DATA HAVING SAME VALUE OF R3 AND RETURN R3 OF PEAK DATA HAVING LARGER THRESHOLD VALUE TO ORIGINAL VALUE (R1) — S10120

NO

ADD DATA OF TEMP2 TO INTEGRATED DATA (ALL RETENTION TIME POINTS AND PEAK DATA PIECES) — S10121

THRESHOLD VALUE 2 ← INITIAL VALUE AND REMOVE ALL DATA INCLUDED IN TEMP2 — S10122

(6)

[FIG.91]

PEAK FEATURE VALUE PROCESS (PREPARATION OF REFERENCE GROUP FP)

REFERENCE FP ASSIGNING DATA

1 2 3 4

↓

REFERENCE FP ASSIGNING RESULT INTEGRATING PROCESS (PREPARATION OF REFERENCE FP CORRESPONDENCE TABLE)

↓

PEAK FEATURE VALUE PROCESS (PREPARATION OF REFERENCE GROUP FP)

S10006

↓

REFERENCE GROUP FP

START

↓

**S10201**

READ REFERENCE FP CORRESPONDENCE TABLE

↓

**S10202**

CALCULATE STATISTIC VALUES FOR EACH PEAK (COLUMN)

↓

**S10203**

SELECT PEAK (COLUMN) WITH REFERENCE TO CALCULATED STATISTIC VALUES

↓

**S10204**

OUTPUT SELECTED PEAK (COLUMN) (REFERENCE GROUP FP)

↓

END

[FIG.92]

123

DETECTION WAVELENGTH (nm) →

| DAD-Data | 203 | 204 | 205 | 206 | 207 | ... | 296 | 297 | 298 | 299 | 300 |
|---|---|---|---|---|---|---|---|---|---|---|---|
| 2.002167 | 0.236511 | 0.184536 | 0.198364 | 0.132561 | 0.192642 | ... | 0.031471 | 0.034332 | -0.00715 | 0.031471 | 0.000954 |
| 2.008833 | 0.24128 | 0.193119 | 0.205994 | 0.142574 | 0.206947 | ... | 0.026703 | 0.034332 | -0.00572 | 0.039577 | -0.00095 |
| 2.0155 | 0.247955 | 0.199795 | 0.216007 | 0.157356 | 0.22316 | ... | 0.01812 | 0.030994 | 0.001431 | 0.048637 | -0.00238 |
| 2.022167 | 0.258923 | 0.208378 | 0.226498 | 0.173569 | 0.239372 | ... | 0.011444 | 0.027657 | 0.009537 | 0.052929 | -0.00238 |
| 2.028833 | 0.270367 | 0.222206 | 0.237942 | 0.189304 | 0.252724 | ... | 0.008583 | 0.023842 | 0.016689 | 0.053406 | 0.000954 |
| 2.0355 | 0.283241 | 0.240326 | 0.249386 | 0.208855 | 0.263214 | ... | 0.010967 | 0.017643 | 0.024319 | 0.050545 | 0.007153 |
| 2.042167 | 0.300884 | 0.257969 | 0.259876 | 0.230789 | 0.271797 | ... | 0.015259 | 0.013828 | 0.032902 | 0.044823 | 0.014782 |
| 2.048833 | 0.323772 | 0.273705 | 0.271797 | 0.252247 | 0.281811 | ... | 0.021935 | 0.015736 | 0.03767 | 0.039577 | 0.021935 |
| 2.0555 | 0.352383 | 0.289917 | 0.283241 | 0.272274 | 0.293732 | ... | 0.027657 | 0.024319 | 0.035286 | 0.039577 | 0.025749 |
| 2.062167 | 0.382423 | 0.306129 | 0.298977 | 0.293255 | 0.306129 | ... | 0.030994 | 0.033855 | 0.030518 | 0.039577 | 0.026226 |
| 2.068833 | 0.418663 | 0.323772 | 0.325203 | 0.321388 | 0.319958 | ... | 0.030041 | 0.044823 | 0.027657 | 0.0453 | 0.025272 |
| 2.0755 | 0.460625 | 0.346661 | 0.36335 | 0.352383 | 0.339985 | ... | 0.023365 | 0.059128 | 0.025749 | 0.052929 | 0.025272 |
| 2.082167 | 0.511169 | 0.383377 | 0.412464 | 0.387669 | 0.366688 | ... | 0.015736 | 0.069141 | 0.021458 | 0.060081 | 0.02861 |
| 2.088833 | 0.570297 | 0.435352 | 0.46587 | 0.431538 | 0.402451 | ... | 0.01049 | 0.071526 | 0.016212 | 0.066757 | 0.030994 |
| ... | ... | ... | ... | ... | ... | ... | ... | ... | ... | ... | ... |
| 59.94883 | 268.1746 | 208.0026 | 160.8496 | 125.2108 | 98.0444 | ... | 0.685215 | 0.713348 | 0.657558 | 0.6814 | 0.639915 |
| 59.9555 | 268.3458 | 208.1509 | 160.9607 | 125.2985 | 98.11354 | ... | 0.684738 | 0.716209 | 0.657082 | 0.680447 | 0.638485 |
| 59.96217 | 268.5189 | 208.2896 | 161.0661 | 125.3839 | 98.176 | ... | 0.683308 | 0.712395 | 0.656605 | 0.679016 | 0.638962 |
| 59.96883 | 268.693 | 208.4146 | 161.1671 | 125.4654 | 98.23656 | ... | 0.681877 | 0.702381 | 0.658989 | 0.676632 | 0.638485 |
| 59.9755 | 268.858 | 208.528 | 161.2616 | 125.5417 | 98.29617 | ... | 0.6814 | 0.690937 | 0.665665 | 0.674725 | 0.639439 |
| 59.98217 | 269.0167 | 208.6339 | 161.3479 | 125.6094 | 98.35434 | ... | 0.67997 | 0.681877 | 0.671387 | 0.677586 | 0.641823 |
| 59.98883 | 269.1703 | 208.7336 | 161.428 | 125.67 | 98.40822 | ... | 0.677586 | 0.674725 | 0.672817 | 0.681877 | 0.644684 |
| 59.9955 | 269.3124 | 208.8246 | 161.5 | 125.7191 | 98.45686 | ... | 0.674725 | 0.67091 | 0.671864 | 0.684738 | 0.646114 |

SIGNAL INTENSITY

RETENTION TIME (MINUTE)

[FIG.93]

EP 2 717 049 B1

125

| HeaderName | HeaderValue | Flags | PeakType | RetTime | Area | Height | Width | ··· | AreaPercent |
|---|---|---|---|---|---|---|---|---|---|
| NumberOfRows | 118 | 2 | 8 | 3.737342119 | 149.1629486 | 24.95453835 | 0.089736186 | ··· | 1.65592471 |
| NumberOfCol | 21 | 32 | 8 | 3.925331116 | 9.433002472 | 1.965691447 | 0.075986251 | ··· | 0.104719986 |
| NumberOfHead | 9 | 0 | 8 | 4.19194603 | 76.68452454 | 7.853988171 | 0.139910638 | ··· | 0.851309257 |
| Modified | | 0 | 8 | 4.611811161 | 13.87264156 | 1.980707884 | 0.111711942 | ··· | 0.154006408 |
| DateTime | | 2 | 8 | 4.96133709 | 140.9757385 | 14.70096874 | 0.137878135 | ··· | 1.565034823 |
| IntegStart | 0 | 34 | 8 | 5.159340382 | 99.85542297 | 11.33316708 | 0.128793851 | ··· | 1.10853978 |
| IntegEnd | 0 | 32 | 8 | 5.294465542 | 53.13053131 | 8.221708298 | 0.09939263 | ··· | 0.589825828 |
| Errors | 0 | 0 | 8 | 5.789111137 | 30.95188141 | 3.765951157 | 0.129886866 | ··· | 0.3436107 |
| IntegMode | 2 | 2 | 8 | 6.094525814 | 31.32414055 | 3.210045815 | 0.147845194 | ··· | 0.347743316 |
| | | 34 | 8 | 6.300107956 | 31.41418457 | 3.775012255 | 0.121085465 | ··· | 0.348742935 |
| | | 34 | 8 | 6.729168415 | 337.5288696 | 25.2769146 | 0.181787893 | ··· | 3.747059177 |
| | | 32 | 8 | 7.14134407 | 128.023819 | 8.847929955 | 0.196809128 | ··· | 1.421249762 |
| | | · | · | · | · | · | · | · · · | · |
| | | · | · | · | · | · | · | · · · | · |
| | | · | · | · | · | · | · | · · · | · |
| | | 0 | 8 | 53.9373436 | 15.90642643 | 1.939949751 | 0.127659678 | ··· | 0.176584365 |
| | | 0 | 8 | 54.65077972 | 184.8139801 | 19.59948349 | 0.144011214 | ··· | 2.05170278 |
| | | 0 | 8 | 55.67518234 | 27.47281837 | 3.244963169 | 0.132773459 | ··· | 0.304988063 |
| | | 0 | 8 | 56.46019745 | 28.83968353 | 2.449590445 | 0.169354796 | ··· | 0.320162246 |
| | | 0 | 8 | 58.57313156 | 27.80234528 | 3.295254707 | 0.132432669 | ··· | 0.308646289 |
| | | 0 | 8 | 59.13926315 | 56.54888153 | 5.344749451 | 0.15743646 | ··· | 0.627774465 |
| | | | | ↑ | ↑ | ↑ | | | |
| | | | | RETENTION TIME (MINUTE) | PEAK AREA | PEAK HEIGHT | | | |

[FIG.94]

EP 2 717 049 B1

~127

| RETENTION TIME (MINUTES) | PEAK HEIGHT | 220 | 221 | 222 | 223 | 224 | · · · | 297 | 298 | 299 | 300 |
|---|---|---|---|---|---|---|---|---|---|---|---|
| 3.737342119 | 24.95453835 | 1 | 0.946988 | 0.893759 | 0.840576 | 0.78823 | · · · | 0.102586 | 0.100418 | 0.098715 | 0.096144 |
| 3.925331116 | 1.965691447 | 1 | 0.96683 | 0.933862 | 0.899785 | 0.870409 | · · · | 0.148963 | 0.145538 | 0.142684 | 0.139663 |
| 4.19194603 | 7.853988171 | 1 | 0.951353 | 0.905206 | 0.861315 | 0.822883 | · · · | 0.858285 | 0.854974 | 0.853741 | 0.848492 |
| 4.611811161 | 1.980707884 | 1 | 0.966231 | 0.934717 | 0.902057 | 0.875049 | · · · | 0.536362 | 0.505956 | 0.476373 | 0.444965 |
| 4.96133709 | 14.70096874 | 1 | 0.955319 | 0.911156 | 0.86973 | 0.833565 | · · · | 0.207648 | 0.205572 | 0.206256 | 0.203732 |
| 5.159340382 | 11.33316708 | 1 | 0.931257 | 0.872526 | 0.82505 | 0.784413 | · · · | 0.307071 | 0.303897 | 0.303275 | 0.302065 |
| 5.294465542 | 8.221708298 | 1 | 0.964049 | 0.924894 | 0.884593 | 0.847809 | · · · | 0.425285 | 0.425389 | 0.427005 | 0.425129 |
| 5.789111137 | 3.765951157 | 1 | 0.952489 | 0.907016 | 0.860134 | 0.816736 | · · · | 0.213023 | 0.211059 | 0.209317 | 0.207167 |
| 6.094525814 | 3.210045815 | 1 | 0.962636 | 0.923002 | 0.885678 | 0.852812 | · · · | 0.154766 | 0.149538 | 0.148322 | 0.146499 |
| 6.300107956 | 3.775012255 | 1 | 0.961981 | 0.924493 | 0.885414 | 0.849061 | · · · | 0.161201 | 0.157036 | 0.157225 | 0.154385 |
| 6.729168415 | 25.2769146 | 1 | 0.963209 | 0.929871 | 0.898246 | 0.870401 | · · · | 0.151135 | 0.147084 | 0.146431 | 0.146295 |
| 7.14134407 | 8.847929955 | 1 | 0.962426 | 0.927794 | 0.892649 | 0.86298 | · · · | 0.232462 | 0.226918 | 0.229348 | 0.227739 |
| · | · | · | · | · | · | · | · · · | · | · | · | · |
| · | · | · | · | · | · | · | · · · | · | · | · | · |
| · | · | · | · | · | · | · | · · · | · | · | · | · |
| 47.04151535 | 21.10215187 | 1 | 0.946357 | 0.898223 | 0.85577 | 0.821346 | · · · | 0.010336 | 0.003716 | 0.002477 | −0.00068 |
| 47.262146 | 12.78098965 | 1 | 0.949657 | 0.903068 | 0.858554 | 0.822637 | · · · | −0.00652 | −0.01092 | −0.00954 | −0.01201 |
| 48.20507431 | 14.44223309 | 1 | 0.966953 | 0.938847 | 0.912835 | 0.889156 | · · · | 0.016335 | 0.011633 | 0.010432 | 0.008717 |
| 48.8165741 | 0.599506497 | 1 | 0.91256 | 0.839808 | 0.770762 | 0.707069 | · · · | −0.00419 | −0.00796 | −0.00323 | −0.00631 |
| 53.9373436 | 1.939949751 | 1 | 0.913891 | 0.839232 | 0.771945 | 0.711194 | · · · | 0.028755 | 0.023475 | 0.02933 | 0.024207 |
| 54.65077972 | 19.59948349 | 1 | 0.969395 | 0.940957 | 0.915986 | 0.892413 | · · · | 0.031448 | 0.027065 | 0.026584 | 0.023791 |
| 55.67518234 | 3.244963169 | 1 | 0.918351 | 0.839363 | 0.77256 | 0.718174 | · · · | 0.035946 | 0.032818 | 0.033752 | 0.029644 |
| 56.46019745 | 2.449590445 | 1 | 0.923736 | 0.850274 | 0.788056 | 0.736999 | · · · | 0.095911 | 0.087768 | 0.085269 | 0.077858 |
| 58.57313156 | 3.295254707 | 1 | 0.921651 | 0.849726 | 0.781768 | 0.714406 | · · · | 0.04279 | 0.042018 | 0.043071 | 0.041386 |
| 59.13926315 | 5.344749451 | 1 | 0.918037 | 0.841855 | 0.775269 | 0.718988 | · · · | 0.051443 | 0.050853 | 0.050617 | 0.048258 |

UV SPECTRUM

(DATA NORMALIZED WITH USE OF MAXIMUM VALUE OF "1" IN UV SPECTRUM OF DETECTION WAVELENGTH OF 220 TO 300 nm)

[FIG.95]

DETERMINATION RESULT FILE

129

| TARGET FP | | | REFERENCE FP | | | | | |
|---|---|---|---|---|---|---|---|---|
| PEAK NUMBER | RETENTION | PEAK HEIGHT | 6 | 5 | 4 | 3 | 2 | 1 ← PEAK NUMBER |
| | | | 5.30 | 5.14 | 4.96 | 4.19 | 3.92 | 3.73 ← RETENTION |
| | | | 6.32 | 11.78 | 11.70 | 5.85 | 2.06 | 24.11 ← PEAK HEIGHT |
| 7 | 5.79 | 6.02 | 114.46 | 888888 | 888888 | 888888 | 888888 | 888888 |
| 6 | 5.18 | 17.28 | 27.09 | 4.05 | 36.11 | 888888 | 888888 | 888888 |
| 5 | 4.91 | 12.07 | 148.07 | 27.67 | 3.27 | 888888 | 888888 | 888888 |
| 4 | 4.58 | 3.12 | 888888 | 888888 | 399.00 | 96.93 | 888888 | 888888 |
| 3 | 4.17 | 5.26 | 888888 | 888888 | 888888 | 4.59 | 194.22 | 800.99 |
| 2 | 3.87 | 2.63 | 888888 | 888888 | 888888 | 178.46 | 1.07 | 83.51 |
| 1 | 3.70 | 26.53 | 888888 | 888888 | 888888 | 706.02 | 103.15 | 0.59 |

PEAK NUMBER   RETENTION   PEAK HEIGHT

EP 2 717 049 B1

[FIG.96]

EP 2 717 049 B1

**131**

ASSIGNMENT CANDIDATE PEAK SCORE TABLE

| REFERENCE FP | | | | | | |
|---|---|---|---|---|---|---|
| 6 | 5 | 4 | 3 | 2 | 1 | ← PEAK NUMBER |
| 5.30 | 5.14 | 4.96 | 4.19 | 3.92 | 3.73 | ← RETENTION |
| 6.32 | 11.78 | 11.70 | 5.85 | 2.06 | 24.11 | ← PEAK HEIGHT |
| 27.09 | 4.05 | 3.27 | 4.59 | 1.07 | 0.59 | ← 1ST CANDIDATE (SCORE) |
| | 27.67 | 36.11 | 96.93 | | 83.51 | ← 2ND CANDIDATE (SCORE) |
| | | | | | | ← 3RD CANDIDATE (SCORE) |

SUBSTITUTE BY PEAK NUMBER
OF CORRESPONDING REFERENCE FP3

ASSIGNMENT CANDIDATE
PEAK NUMBER TABLE

**133**

| REFERENCE FP | | | | | | |
|---|---|---|---|---|---|---|
| 6 | 5 | 4 | 3 | 2 | 1 | ← PEAK NUMBER |
| 5.30 | 5.14 | 4.96 | 4.19 | 3.92 | 3.73 | ← RETENTION |
| 6.32 | 11.78 | 11.70 | 5.85 | 2.06 | 24.11 | ← PEAK HEIGHT |
| 6 | 6 | 5 | 3 | 2 | 1 | ← 1ST CANDIDATE (NUMBER) |
| | 5 | 6 | 4 | | 2 | ← 2ND CANDIDATE (NUMBER) |
| | | | | | | ← 3RD CANDIDATE (NUMBER) |

[FIG.97]

EP 2 717 049 B1

COLLATION RESULT FILE

135

| REFERENCE FP PEAK NUMBER | REFERENCE FP RETENTION TIME | REFERENCE FP PEAK DATA | TARGET FP PEAK DATA |
|---|---|---|---|
|  | 5.77 | 0.00 | 6.02 |
| 6 | 5.30 | 6.32 | 0.00 |
| 5 | 5.14 | 11.78 | 17.28 |
| 4 | 4.96 | 11.70 | 12.07 |
|  | 4.62 | 0.00 | 3.12 |
| 3 | 4.19 | 5.85 | 5.26 |
| 2 | 3.92 | 2.06 | 2.63 |
| 1 | 3.73 | 24.11 | 26.53 |

[FIG.98]

EP 2 717 049 B1

137

| | P1 | P2 | P3 | P4 | P5 | P6 | · · · | P59 | P60 | P61 | P62 | ← REFERENCE GROUP FP PEAK NUMBER |
|---|---|---|---|---|---|---|---|---|---|---|---|---|
| | 3.74 | 4.19 | 4.97 | 5.15 | 5.3 | 5.79 | · · · | 54.63 | 55.65 | 58.56 | 59.13 | ← REFERENCE GROUP FP RETENTION TIME (MINUTE) |
| REFERENCE FP01 | 24.95454 | 7.853988 | 14.70097 | 11.33317 | 8.221708 | 3.765951 | · · · | 19.59948 | 3.244963 | 3.295255 | 5.344749 | |
| REFERENCE FP02 | 24.6409 | 5.85494 | 11.60693 | 13.23757 | 6.280517 | 4.881429 | · · · | 29.02091 | 4.153991 | 5.682264 | 6.406809 | |
| REFERENCE FP03 | 23.41309 | 6.5214 | 12.75416 | 10.8282 | 6.856488 | 6.392059 | · · · | 30.33544 | 3.53835 | 4.372816 | 6.136991 | |
| REFERENCE FP04 | 24.1606 | 7.157524 | 12.22046 | 11.29149 | 6.495505 | 5.647078 | · · · | 26.83015 | 4.332538 | 3.193285 | 5.196698 | |
| REFERENCE FP05 | 24.39492 | 5.397262 | 11.57015 | 12.50578 | 7.025057 | 3.999343 | · · · | 24.84871 | 3.971856 | 4.557617 | 6.042276 | |
| REFERENCE FP06 | 23.79346 | 6.513698 | 12.60938 | 11.06297 | 6.917242 | 5.876327 | · · · | 31.78834 | 3.642261 | 3.717728 | 6.79227 | |
| · | · | · | · | · | · | · | · · · | · | · | · | · | PEAK HEIGHT |
| · | · | · | · | · | · | · | · · · | · | · | · | · | |
| · | · | · | · | · | · | · | · · · | · | · | · | · | |
| REFERENCE FP74 | 24.54318 | 7.229315 | 12.54595 | 11.2069 | 6.476972 | 5.93869 | · · · | 28.67843 | 3.249248 | 3.390216 | 4.883753 | |
| REFERENCE FP75 | 24.29594 | 4.890856 | 9.386555 | 12.18806 | 5.953234 | 5.645267 | · · · | 31.22406 | 3.530962 | 3.596855 | 6.64429 | |
| REFERENCE FP76 | 23.70935 | 6.477376 | 12.64439 | 10.87211 | 6.865796 | 5.588339 | · · · | 31.43591 | 4.058423 | 4.047005 | 6.010718 | |
| REFERENCE FP77 | 24.38124 | 6.815894 | 12.57119 | 10.90901 | 6.628489 | 5.446465 | · · · | 27.19673 | 3.325539 | 2.983221 | 5.730851 | |
| REFERENCE FP78 | 25.64983 | 5.101483 | 9.62847 | 12.87496 | 6.267442 | 7.07402 | · · · | 32.01024 | 3.428246 | 4.078995 | 6.910386 | |
| REFERENCE FP79 | 24.56647 | 6.390944 | 11.81665 | 11.60368 | 6.251297 | 4.853024 | · · · | 30.00299 | 3.375931 | 3.21543 | 5.605225 | |
| REFERENCE FP80 | 25.70864 | 6.55578 | 11.56949 | 11.24369 | 7.709552 | 5.818771 | · · · | 28.76077 | 3.235093 | 4.217419 | 5.674382 | |
| REFERENCE FP81 | 24.95631 | 6.882163 | 12.10839 | 11.19636 | 7.243775 | 5.95506 | · · · | 27.85074 | 3.156492 | 2.711058 | 5.909272 | |
| REFERENCE FP82 | 25.13536 | 6.996024 | 12.17565 | 11.21373 | 7.208914 | 5.948516 | · · · | 28.11332 | 3.297449 | 3.585945 | 5.951647 | |

↑
REFERENCE FP NAME

[FIG.99]

139

| | P1 | P2 | P3 | P4 | P5 | P6 | • • • | P59 | P60 | P61 | P62 | ↓ REFERENCE GROUP FP PEAK NUMBER |
|---|---|---|---|---|---|---|---|---|---|---|---|---|
| | 3.74 | 4.19 | 4.97 | 5.15 | 5.3 | 5.79 | • • • | 54.63 | 55.65 | 58.56 | 59.13 | ↓ REFERENCE GROUP FP RETENTION TIME (MINUTE) |
| M100−364 | 25.17536 | 7.446205 | 13.59427 | 10.46504 | 7.924618 | 3.533023 | • • | 19.63221 | 3.449513 | 3.44023 | 5.512316 | ← PEAK DATA FEATURE VALUE OF TARGET FP |

↑
TARGET FP NAME

[FIG.100]

SUBROUTINE 2
(CALCULATION OF DEGREE OF MATCHING BETWEEN UV SPECTRA)

START OF CALCULATION DEGREE OF
MATCHING BETWEEN UV SPECTRA

S2001
$x \leftarrow U1$, $y \leftarrow U2$, $z \leftarrow 0$,
INTERVAL 1 $\leftarrow$ w1, AND INTERVAL 2 $\leftarrow$ w2

S2002
ACQUIRE NUMBER OF DATA PIECES OF "x" (a)

S2003
$i \leftarrow 1$

S2004
ACQUIRE xi-TH DATA (b)

S2005
ACQUIRE yi-TH DATA (c)

S2006
CALCULATE INTER-UV SPECTRUM DISTANCE (d)
AND SUM OF SQUARE OF INTER-UV
SPECTRUM DISTANCE (z)

$d = b-c$
$z = z+d2$

S2007
UPDATE "i" ($i \leftarrow i + 1$)

S2008
HAS PROCESS OF ALL DATA
OF "x" BEEN COMPLETED?          NO

YES

S2010
IS DNS ADDED?          YES

NO

S2011
CALCULATE MOVING AVERAGES OF "x" AND "y"
IN INTERVAL 1 (w1)

S2012
CALCULATE MOVING INCLINATIONS OF "x" AND "y"
IN INTERVAL 2 (w2)

CALCULATE NUMBERS OF MISMATCHES BETWEEN CODES OF
MOVING INCLINATIONS OF "x" AND "y" (DNS)

S2013

S2009A
CALCULATE DEGREE OF MATCHING BETWEEN
UV SPECTRA OF "x" AND "y" (UV_Sim)

CASE WHERE DNS IS NOT ADDED
$UV\_Sim = \sqrt{(z/a)}$

CASE WHERE DNS IS ADDED
$UV\_Sim = \sqrt{(z/a)} * 1.1 \char`\^ DNS$

CALCULATE DEGREE OF MATCHING
BETWEEN UV SPECTRA

117

[FIG. 101]

EP 2 717 049 B1

118

| UV DATA | a1 | a2 | a3 | a4 | a5 | a6 | a7 |
|---|---|---|---|---|---|---|---|
| MOVING AVERAGE (CASE OF INTERVAL 1 (w1 = 3)) | | | m1=(a1+a2+a3)/3 | m2=(a2+a3+a4)/3 | m3=(a3+a4+a5)/3 | m4=(a4+a5+a6)/3 | m5=(a5+a6+a7)/3 |
| MOVING INCLINATION (CASE OF INTERVAL 2 (w2 = 3)) | | | | | s1=(m3−m1)/3 | s1=(m4−m2)/3 | s1=(m5−m3)/3 |

**EP 2 717 049 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002214215 A
- WO 9739347 A1
- WO 9739347 A
- CN 1670529 A
- EP 1760464 A1

### Non-patent literature cited in the description

- *Pharmaceuticals monthly,* 1986, vol. 28 (3), 67-71
- Comprehensive two-dimensional gas chromatography for fingerprint pattern recognition in cachaca production. **CARDEAL et al.** TALANTA. ELSEVIER, 02 January 2008, vol. 74, 793-799
- Mathematics for Quality Engineering. Japanese Standards Association, 2000, 136-138
- Technical Developments in Chemistry, Pharmacy and Biology. Quality Engineering of Application Course. Japanese Standards Association, 1999, 454-456
- *Quality Engineering,* 2003, vol. 11 (5), 78-84
- *Introduction to MT System,* 2008